(19) Europäisches Patentamt
European Patent Office
Office européen des brevets

(11) Veröffentlichungsnummer: **0 154 190**
**A1**

(12) # EUROPÄISCHE PATENTANMELDUNG

(21) Anmeldenummer: 85101326.8

(22) Anmeldetag: 08.02.85

(51) Int. Cl.[4]: C 07 D 417/04
C 07 D 513/04, C 07 D 213/85
C 07 D 417/14, C 07 D 471/04
C 07 D 215/54, C 07 D 401/04
C 07 D 401/06, C 07 D 401/12
C 07 D 401/14, C 07 D 413/04

(30) Priorität: 22.02.84 DE 3406329

(43) Veröffentlichungstag der Anmeldung:
11.09.85 Patentblatt 85/37

(84) Benannte Vertragsstaaten:
AT BE CH DE FR GB IT LI NL SE

(71) Anmelder: Merck Patent Gesellschaft mit beschränkter Haftung
Frankfurter Strasse 250
D-6100 Darmstadt(DE)

(72) Erfinder: Juraszyk, Horst, Dr.
Kleiner Ring 14
D-6104 Seeheim 1(DE)

(72) Erfinder: Enenkel, Hans-Joachim, Dr.
Wingertsbergstrasse 5
D-6100 Darmstadt(DE)

(72) Erfinder: Minck, Klaus Otto, Dr.
Büchestrasse 8
D-6105 Ober-Ramstadt(DE)

(72) Erfinder: Schliep, Hans-Joachim, Dr.
Weingartenstrasse 16
D-6109 Mühltal(DE)

(72) Erfinder: Piulats, Jaime, Dr.
Rvds. Quintin Mallo pe 2
Barcelona 30(ES)

(54) **Pyridone.**

(57) Neue 2(1H)-Pyridone der allgemeinen Formel I

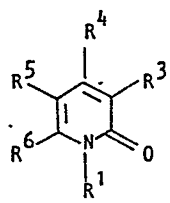

(I)

worin
$R^1$ sowie $R^3$ bis $R^6$ die in Patentanspruch 1 angegebene Bedeutung haben,
sowie die entsprechenden Lactimester und Salze zeigen positiv inotrope Wirkungen.

EP 0 154 190 A1

Croydon Printing Company Ltd.

Pyridone

Die Erfindung betrifft neue 2(1H)-Pyridone, vor- und nachstehend kurz als "2-Pyridone" bezeichnet, der allgemeinen Formel I gemäß Patentanspruch 1

worin

$R^1$ H, A oder Benzyl,

$R^3$ H, CN, $CONH_2$, COOH, COOA, $COOCH_2C_6H_5$ oder $NH_2$,

$R^4$ H, A, $A_2N-CH=CH-$, Ar-alkyl, OH, OA, SA, SO-A, $SO_2-A$ oder $NR^7R^8$,

$R^5$ H, $R^9-CHR^{10}-CO-$, $A_2N-CH=CR^{10}-CO-$, Ar, Py, unsubstituiertes oder ein- oder mehrfach durch $R^{10}$ und/oder $R^{11}$ substituiertes Thiazolyl, Imidazolyl, Pyrazolyl, Isoxazolyl oder Imidazopyridyl,

$R^6$ H, A, COOA oder zusammen mit $R^{10}$ eine $-CH_2CH_2-$ oder -CH=CH-Gruppe,

A Alkyl,

Ar Phenyl oder ein- bis dreifach durch A, OA, F, Cl, Br, J, $CF_3$, OH, $NO_2$, $NH_2$, ANH, $A_2N$, CN, $CONR^7R^8$, COOH, COOA, $CH_2CONR^7R^8$, $CH_2COOH$, $CH_2COOA$, $OCH_2COOH$, $OCH_2COOA$, SA, SO-A, $SO_2A$, Phosphonomethyl, Alkylphosphonomethyl, Dialkylphosphonomethyl, AO-alkyl, AS-alkyl, Ar', Ar'O, Ar'-thioalkyl, Hydroxyalkyl und/oder Imidazolyl substituiertes Phenyl,

Ar' Phenyl oder Fluorphenyl,

Py 2-, 3- oder 4-Pyridyl oder einfach durch A, OA, F, Cl, Br, J, $NO_2$, CN, $CONH_2$, COOH, N-Oxido-sauerstoff oder Pyridyl substituiertes 2-, 3- oder 4-Pyridyl,

$R^7$ H, A, Alkenyl, Hydroxyalkyl, Dihydroxyalkyl, AO-alkyl, Ar, Py, $R^{12}R^{13}N$-alkyl, Ar-alkyl, Ar-hydroxyalkyl, Indanyl, Py-alkyl, 1-$R^{14}$-piperidyl oder 2-(4-Methyl-5-imidazolylmethyl-thio)-ethyl,

| | |
|---|---|
| $R^8$ | H, A, Hydroxyalkyl, Ar, Ar-alkyl, H-CO-, A-CO-, Ar-CO-, Py-CO-, Ar-alkyl-CO-, COOA oder CONHA, |
| $R^7$ und $R^8$ | zusammen auch $A_2N$-CH= oder eine Alkylengruppe mit 3 bis 5 C-Atomen, die durch -O- oder $-NR^{15}-$ unterbrochen und/oder ein- oder mehrfach durch Carbonylsauerstoff, A, Ar-alkyl, COOH, COOA oder $ArCH_2OCO$- substituiert sein kann, |
| $R^9$ | H, H-CO-, AO-CO-, A-CO-, Cl, Br, OH, OA, A-COO-, $CF_3COO$-, $A-SO_2-O-$, $Ar-SO_2-O-$, SCN, 2-Imidazolylthio, 2-Benzimidazolylthio, Ar-CO-, Py-CO, Furoyl oder Thenoyl, |
| $R^{10}$ | H, A oder zusammen mit $R^6$ eine $-CH_2CH_2-$ oder -CH=CH-Gruppe, |
| $R^{11}$ | H, A, Alkenyl, AO-alkyl, $R^7R^8N$-CO-, $R^7R^8N-CO-CH_2-$, $AOOC-CH_2-$, CN, $CN-CH_2-$, Ar, Ar-alkyl, ArO-alkyl, ArS-alkyl, ArSO-alkyl, $ArSO_2$-alkyl, Ar-alkyl-O-alkyl, Ar-alkenyl, Py, Py-alkyl, Pyrimidyl, F, Cl, Br, J, OH, SH, OA, SA, SO-A, $SO_2$-A, $NR^7R^8$ oder 2-Oxo-1,2-dihydro-4-(A-CO)-5-A-pyridyl, |
| $R^{12}$ und $R^{13}$ | jeweils H, A, Ar-alkyl, H-CO-, A-CO-, Ar-CO- oder Ar-alkyl-CO- oder zusammen eine Alkylengruppe mit 4 oder 5 C-Atomen, die durch -O- oder $-NR^{15}-$ unterbrochen und/oder ein- oder mehrfach durch Carbonylsauerstoff, A, Ar-alkyl, COOH, COOA oder $Ar-CH_2O-CO$- substituiert sein kann, |
| $R^{14}$ | H, Formyl, COOA oder Benzyl, |
| $R^{15}$ | H, A, Hydroxyalkyl, Dihydroxyalkyl, $A_2N$-alkyl, A-CO-, Ar, Thienyl, Ar-alkyl, $R^{12}R^{13}N-CO-CH_2-$, Ar-CO-, Furoyl, Thenoyl, $H_2N$-CO-, ANH-CO-, ArNH-CO- oder A-COO-alkyl |

bedeuten,

worin die Alkyl- und Alkenylgruppen jeweils bis zu 6 C-Atome besitzen,

worin ferner

(a) nur zwei der Reste $R^3$ bis $R^6$ H bedeuten können,

(b) $R^5$ nur dann H sein kann, wenn gleichzeitig $R^4$ Ar-alkyl oder $NR^7R^8$ bedeutet,

(c) $R^5$ nur dann Ar, Py, unsubstituiertes Thiazolyl, unsubstituiertes Imidazolyl, unsubstituiertes Pyrazolyl oder unsubstituiertes Isoxazolyl sein kann, wenn gleichzeitig $R^4$ A, $A_2N-CH=CH-$, Ar-alkyl, OH, OA, SO-A, $SO_2-A$ oder $NR^7R^8$ und/oder $R^6$ COOA bedeuten,

(d) $R^5$ nur dann $CH_3-CO-$ oder $A-CH_2-CO-$ sein kann, wenn gleichzeitig $R^4$ $A_2N-CH=CH-$, Ar-alkyl, OH, OA, SO-A, $SO_2-A$ oder $NR^7R^8$ und/oder $R^6$ COOA bedeuten,

(e) $R^5$ und $R^6$ zusammen nur dann $-COCH_2CH_2CH_2-$ sein können, wenn gleichzeitig $R^3$ CN, COOH, COOA, $COOCH_2C_6H_5$ oder $NH_2$ und/oder $R^4$ A, $A_2N-CH=CH-$, Ar-alkyl, OH, OA, SO-A, $SO_2-A$ oder $NR^7R^8$ bedeuten,

sowie die entsprechenden Lactimester und die Salze der Verbindungen der Formel I.

Die Verbindungen der Formel I, in denen $R^1$ H bedeutet, können auch in tautomeren Formen, z.B. in Form der tautomeren Lactime (2-Hydroxypyridine) der Formel I' vorliegen. Die Erfindung erstreckt sich auch auf alle

tautomeren Formen der Verbindungen sowie auch auf die entsprechenden Lactimester (2-Hydroxypyridinester) der Formel IX

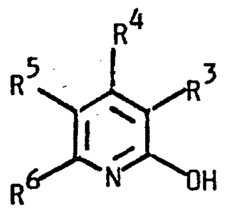

I'

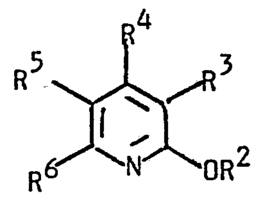

IX

worin $R^2$ eine Acylgruppe bedeutet, vorzugsweise H-CO-, A-CO-, Ar-CO-, Py-CO-, Ar-alkyl-CO-, COOA, CONHA, A-$SO_2$- oder Ar-$SO_2$.

Ähnliche Verbindungen sind bekannt aus:

EP-OS 0074091; EP-OS 0089022;
DE-OS 26 46 469; DE-OS 31 06 460;
US-PS 4264609; US-PS 4312875; US-PS 4313951;
Chem.Abstr. <u>80</u>, 133202r (1974); <u>83</u>, 193029m;
<u>83</u>, 206076b;
J.Chem.Soc.Perkin Trans. I 1978(6) 549-553, 554-558, 857-862.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I, ihrer Lactimester und ihrer Salze gelöst.

Es wurde gefunden, daß diese Verbindungen bei guter Verträglichkeit wertvolle pharmakologische Eigenschaften besitzen. Insbesondere zeigen sie eine Wirkung auf den Blutdruck, die Herzkraft (positiv inotrope Wirksamkeit) und eine Ulcus-Wirkung. Die Blutdruck- und Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen oder Katze ermittelt werden, z.B. nach Methoden, wie sie in der DE-OS 26 46 469 oder in Arzneimittelforschung, Band 31 (I) Nr. 1a (1981), Seiten 141 bis 170, beschrieben sind.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind Verbindungen der Formel I, ihre Lactimester und ihre Salze.

In den genannten Resten ist A eine vorzugsweise unverzweigte Alkylgruppe, die vorzugsweise 1 - 4, insbesondere 1, 2 oder 3 C-Atome hat und bevorzugt für Methyl, ferner bevorzugt für Ethyl, Propyl, Isopropyl, Butyl, Isobutyl, sek.-Butyl, tert.-Butyl, Pentyl, Isopentyl (3-Methylbutyl), Hexyl oder Isohexyl (4-Methylpentyl) steht, ferner auch z.B. für 1- oder 2-Methylbutyl, 1,1-, 1,2- oder 2,2-Dimethylpropyl (Neopentyl), 1-Ethylpropyl, 1-, 2- oder 3-Methylpentyl, 1- oder 2-Ethylbutyl, 1,1-, 1,2-, 1,3-, 2,2-, 2,3- oder 3,3-Dimethylbutyl, 1-Methyl-1-ethylpropyl, 1-Ethyl-2-methylpropyl, 1,1,2- oder 1,2,2-Trimethylpropyl.

Dementsprechend ist A-CO- vorzugsweise Acetyl oder Propionyl, weiterhin bevorzugt Butyryl, Isobutyryl, Valeryl, Capronyl oder Heptanoyl. COOA ist bevorzugt Methoxycarbonyl oder Ethoxycarbonyl. $A_2N-CH=CH-$ ist bevorzugt 2-Dimethylaminovinyl oder 2-Diethylaminovinyl. OA ist bevorzugt Methoxy oder Ethoxy. SA ist bevorzugt Methylthio oder Ethylthio. SO-A ist bevorzugt Methylsulfinyl oder Ethylsulfinyl. $SO_2-A$ ist bevorzugt Methylsulfonyl oder Ethylsulfonyl. $A_2N-C=CR^{10}-CO-$ ist bevorzugt $(CH_3)_2N-C=CR^{10}-CO-$ oder $(C_2H_5)_2N-C=CR^{10}-CO-$. ANH ist bevorzugt Methylamino oder Ethylamino. $A_2N$ ist bevorzugt Dimethylamino oder Diethylamino. AO-alkyl ist bevorzugt Methoxymethyl, Ethoxymethyl, 2-Methoxyethyl oder 2-Ethoxyethyl. AS-alkyl ist bevorzugt Methylthiomethyl, Ethylthiomethyl, 2-Methylthioethyl oder 2-Ethylthioethyl. Hydroxyalkyl ist bevorzugt 2-Hydroxyethyl, ferner auch Hydroxymethyl, 1-Hydroxyethyl, 1-, 2- oder 3-Hydroxypropyl. Dihydroxyalkyl ist bevorzugt

2,3-Dihydroxypropyl. CONHA ist bevorzugt N-Methyl- oder N-Ethylcarbamoyl. A-COO- ist bevorzugt Acetoxy oder Propionoxy. A-$SO_2$-O- ist bevorzugt Methan- oder Ethansulfonyloxy. $A_2$N-CH= ist bevorzugt $(CH_3)_2$N-CH=, ferner auch bevorzugt $(C_2H_5)_2$N-CH=. $A_2$N-alkyl ist bevorzugt 2-Dimethylaminoethyl, 2-Diethylaminoethyl, 2- oder 3-Dimethylaminopropyl, 2- oder 3-Diethylaminopropyl. ANH-CO- ist bevorzugt N-Methyl-carbamoyl oder N-Ethyl-carbamoyl.

Alkenyl ist vorzugsweise unverzweigt, hat insbesondere 2 - 4, bevorzugt 3 C-Atome und steht bevorzugt für Allyl, ferner für Vinyl, 1-Propen-1-yl, Butenyl wie 1-Buten-1-yl, 2-Buten-1-yl, 3-Buten-1-yl, Pentenyl wie 1-Penten-1-yl, 2-Penten-1-yl, 3-Penten-1-yl. Die Alkenylgruppen können aber auch verzweigt sein; Beispiele dafür sind 1-Methyl-2-propen-1-yl, 1-Methyl-2-buten-1-yl, 2-Methyl-3-buten-1-yl, 1,1-Dimethyl-2-propen-1-yl.

Ar ist bevorzugt Phenyl oder einfach substituiertes Phenyl, insbesondere o-, m- oder p-Tolyl, o-, m- oder p-Ethylphenyl, o-, m- oder p-Propylphenyl, o-, m- oder p-Isopropylphenyl, o-, m- oder p-Butylphenyl, o-, m- oder p-Isobutylphenyl, o-, m- oder p-Hexylphenyl, o-, m- oder p-Methoxyphenyl, o-, m- oder p-Ethoxyphenyl, o-, m- oder p-Propoxyphenyl, o-, m- oder p-Isopropoxyphenyl, o-, m- oder p-Butoxyphenyl, o-, m- oder p-Isobutoxyphenyl, o-, m- oder p-Hexoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m- oder p-Hydroxyphenyl, o-, m- oder p-Nitrophenyl,

o-, m- oder p-Aminophenyl, o-, m- oder p-Methylaminophenyl, o-, m- oder p-Ethylaminophenyl, o-, m- oder p-Dimethylaminophenyl, o-, m- oder p-Methylethylaminophenyl, o-, m- oder p-Diethylaminophenyl, o-, m- oder p-Cyanphenyl, o-, m- oder p-Carbamoylphenyl, o-, m- oder p-Carboxyphenyl, o-, m- oder p-Methoxycarbonylphenyl, o-, m- oder p-Ethoxycarbonylphenyl, o-, m- oder p-Carboxymethylphenyl, o-, m- oder p-Methoxycarbonylmethylphenyl, o-, m- oder p-Ethoxycarbonylmethylphenyl, o-, m- oder p-Methylthiophenyl, o-, m- oder p-Ethylthiophenyl, o-, m- oder p-Methylsulfinylphenyl, o-, m- oder p-Ethylsulfinylphenyl, o-, m- oder p-Methylsulfonylphenyl, o-, m- oder p-Ethylsulfonylphenyl, o-, m- oder p-Phosphonomethylphenyl, o-, m- oder p-Methylphosphonomethylphenyl, o-, m- oder p-Ethylphosphonomethylphenyl, o-, m- oder p-Dimethylphosphonomethylphenyl, o-, m- oder p-Diethylphosphonomethylphenyl, o-, m- oder p-Methoxymethylphenyl, o-, m- oder p-(2-Methoxyethyl)phenyl, o-, m- oder p-Methylthiomethylphenyl, o-, m- oder p-(2-Methylthioethyl)-phenyl, 2-, 3- oder 4-Biphenylyl, 2'-, 3'- oder 4'-Fluor-4-biphenylyl, o-, m- oder p-Phenoxyphenyl, o-, m- oder p-(p-Fluorphenoxy)-phenyl, o-, m- oder p-Phenylthiomethylphenyl, o-, m- oder p-Hydroxymethylphenyl, o-, m- oder p-(2-Hydroxyethyl)-phenyl.

Ar kann auch eine zweifach, ferner eine dreifach substituierte Phenylgruppe sein, worin die Substituenten gleich oder voneinander verschieden sein können, z. B. 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Dihydroxyphenyl, 3,4-Diaminophenyl, 3-Methoxy-4-hydroxyphenyl, 3-Hydroxy-4-methoxyphenyl, 3-Nitro-4-hydroxyphenyl, 3-Amino-4-hydroxyphenyl, 3,4,5-Trimethoxyphenyl.

Besonders bevorzugte Reste Ar sind Phenyl, o-, m- und p-Methoxyphenyl, 2,4- und 3,4-Dimethoxyphenyl. Dementsprechend ist Ar-alkyl bevorzugt Benzyl, o-, m- oder p-Methoxybenzyl, 2,4- oder 3,4-Dimethoxybenzyl, 2-Phenylethyl, 2-(o-, 2-(m- oder 2-(p-Methoxyphenyl)-ethyl, 2-(2,4- oder 2-(3,4-Dimethoxyphenyl)-ethyl, und Ar-CO-ist bevorzugt Benzoyl, o-, m- oder p-Methoxybenzoyl, 2,4- oder 3,4-Dimethoxybenzoyl. Ar-$SO_2$-O- ist bevorzugt Benzol- oder p-Toluolsulfonyloxy. Ar-hydroxyalkyl ist bevorzugt α-Hydroxybenzyl, 1- oder 2-Hydroxy-2-phenylethyl, α-Hydroxy-p-methoxybenzyl oder 1- oder 2-Hydroxy-2-p-methoxyphenylethyl. Ar-alkyl-CO ist bevorzugt Phenylacetyl, 3-Phenylpropionyl, p-Methoxyphenylacetyl oder 3-p-Methoxyphenylpropionyl. $ArCH_2OCO$- ist bevorzugt Benzyloxycarbonyl oder p-Methoxybenzyloxycarbonyl. Ar-NH-CO- ist bevorzugt N-Phenylcarbamoyl oder N-p-Methoxyphenyl-carbamoyl.

Py steht vorzugsweise für 4-Pyridyl, ferner bevorzugt für 2- oder 3-Pyridyl, weiterhin für 3-, 4-, 5- oder 6-Methyl-2-pyridyl, 2-, 4-, 5- oder 6-Methyl-3-pyridyl, 2- oder 3-Methyl-4-pyridyl, 2- oder 3-Ethyl-4-pyridyl, 3-, 4-, 5- oder 6-Methoxy-2-pyridyl, 2-, 4-, 5- oder 6-Methoxy-3-pyridyl, 2- oder 3-Methoxy-4-pyridyl, 2- oder 3-Ethoxy-4-pyridyl, 3-, 4-, 5- oder 6-Fluor-2-pyridyl, 2-, 4-, 5- oder 6-Fluor-3-pyridyl, 2- oder 3-Fluor-4-pyridyl, 3-, 4-, 5- oder 6-Chlor-2-pyridyl, 2-, 4-, 5- oder 6-Chlor-3-pyridyl, 2- oder 3-Chlor-4-pyridyl, 3-, 4-, 5- oder 6-Brom-2-pyridyl, 2-, 4-, 5- oder 6-Brom-3-pyridyl, 2- oder 3-Brom-4-pyridyl, 3-, 4-, 5- oder 6-Jod-2-pyridyl, 2-, 4-, 5- oder 6-Jod-3-pyridyl, 2- oder 3-Jod-4-pyridyl, 3-, 4-, 5- oder 6-Nitro-2-pyridyl, 2-, 4-, 5- oder 6-Nitro-3-pyridyl, 2- oder 3-Nitro-4-pyridyl, 3-, 4-, 5- oder 6-Cyan-2-pyridyl, 2-, 4-, 5- oder 6-Cyan-3-pyridyl,

2-oder 3-Cyan-4-pyridyl, 3-, 4-, 5- oder 6-Carbamoyl-2-pyridyl, 2-, 4-, 5- oder 6-Carbamoyl-3-pyridyl, 2- oder 3-Carbamoyl-4-pyridyl, 3-, 4-, 5- oder 6-Carboxy-2-pyridyl, 2-, 4-, 5- oder 6-Carboxy-3-pyridyl, 2- oder 3-Carboxy-4-pyridyl, N-Oxido-2-, -3- oder -4-pyridyl,3-, 4-, 5- oder 6-(2-Pyridyl)-2-pyridyl, 2-, 4-, 5- oder 6-(2-Pyridyl)-3-pyridyl, 2- oder 3-(2-Pyridyl)-4-pyridyl, 3-, 4-, 5- oder 6-(3-Pyridyl)-2-pyridyl, 2-, 4-, 5- oder 6-(3-Pyridyl)-3-pyridyl, 2- oder 3-(3-Pyridyl)-4-pyridyl, 3-, 4-, 5- oder 6-(4-Pyridyl)-2-pyridyl, 2-, 4-, 5- oder 6-(4-Pyridyl)-3-pyridyl, 2- oder 3-(4-Pyridyl)-4-pyridyl.

Dementsprechend ist Py-alkyl vorzugsweise 2-, 3- oder 4-Pyridylmethyl, 2-(2-, 2-(3- oder 2-(4-Pyridyl)-ethyl, und Py-CO- ist bevorzugt Picolinoyl, Nicotinoyl oder Isonicotinoyl.

Thiazolyl ist bevorzugt 4-Thiazolyl, insbesondere 2-$R^{11}$-4-thiazolyl oder 2-$R^{11}$-5-$R^{10}$-4-thiazolyl, aber auch 2-Thiazolyl oder 5-Thiazolyl, insbesondere 2-$R^{11}$-4-$R^{10}$-5-thiazolyl.

Imidazolyl ist bevorzugt 4(5)-Imidazolyl, insbesondere 2-$R^{11}$-4(5)-imidazolyl.

Pyrazolyl ist bevorzugt 3-Pyrazolyl, insbesondere 1-$R^{10}$-3-pyrazolyl, 1-$R^{11}$-5-$R^{10}$-3-pyrazolyl oder 2-$R^{10}$-5-$R^{11}$-3-pyrazolyl, oder 5-Pyrazolyl, insbesondere 1-$R^{10}$-3-$R^{11}$-5-pyrazolyl, 3-$R^{11}$-5-pyrazolyl, 1-$R^{11}$-3-$R^{10}$-5-pyrazolyl, 4-$R^{10}$-5-pyrazolyl, 1-$R^{11}$-4-$R^{10}$-pyrazolyl, 3-$R^{11}$-4-$R^{10}$-5-pyrazolyl oder 1,3-Di-$R^{11}$-4-$R^{10}$-5-pyrazolyl.

Imidazopyridyl ist bevorzugt Imidazo[1,2-a]pyridyl, insbesondere 2-Imidazo[1,2-a]pyridyl.

Indanyl ist bevorzugt 1- oder 2-Indanyl. 1-$R^{14}$-piperidyl ist bevorzugt 4-Piperidyl, 1-Formyl-4-piperidyl, 1-Methoxycarbonyl-4-piperidyl, 1-Ethoxycarbonyl-4-piperidyl oder 1-Benzyl-4-piperidyl. Furoyl ist bevorzugt 2-Furoyl, aber auch 3-Furoyl. Thenoyl ist bevorzugt 2-Thenoyl, aber auch 3-Thenoyl. Pyrimidyl ist bevorzugt 2-Pyrimidyl, aber auch 4- oder 5-Pyrimidyl. 2-Oxo-1,2-dihydro-4-(A-CO)-5-A-pyridyl ist bevorzugt 2-Oxo-1,2-dihydro-4-acetyl-5-methyl-pyridyl. Thienyl ist bevorzugt 2-Thienyl, aber auch 3-Thienyl.

Im einzelnen bedeutet $R^1$ vorzugsweise H, in zweiter Linie Methyl, Ethyl oder Benzyl.

$R^2$ ist vorzugsweise Ar-CO-.

$R^3$ ist vorzugsweise CN, ferner bevorzugt $CONH_2$ oder $NH_2$, weiterhin bevorzugt H oder COOH.

$R^4$ ist vorzugsweise H, ferner bevorzugt OA (insbesondere Methoxy), SA (insbesondere Methylthio), $NR^7R^8$, vor allem $NH_2$, $NA_2$ (insbesondere Dimethylamino), NH-alkyl-Ar [insbesondere 2-Phenylethylamino, 2-p-Methoxyphenylethylamino, 2-(3,4-Dimethoxyphenyl)-ethylamino] oder $(CH_3)_2N-CH=CH-$, weiterhin bevorzugt A (insbesondere Methyl), Ar-alkyl (insbesondere Benzyl oder p-Methoxybenzyl), OH, SO-A (insbesondere $SO-CH_3$), $SO_2-A$ (insbesondere $SO_2-CH_3$) oder 2-Hydroxyethylamino.

$R^5$ ist vorzugsweise $R^9-CHR^{10}-$, unsubstituiertes oder ein- oder mehrfach durch $R^{10}$ und/oder $R^{11}$ substituiertes Thiazolyl, unsubstituiertes oder ein- oder mehrfach durch $R^{10}$

und/oder $R^{11}$ substituiertes Imidazolyl, unsubstituiertes oder ein- oder mehrfach durch $R^{10}$ und/oder $R^{11}$ substituiertes Imidazopyridyl, ferner bevorzugt unsubstituiertes oder ein- oder mehrfach durch $R^{10}$ und/oder $R^{11}$ substituiertes Pyrazolyl oder unsubstituiertes oder ein- oder mehrfach durch $R^{10}$ und/oder $R^{11}$ substituiertes Isoxazolyl, weiterhin - unter den in Anspruch 1 angegebenen Bedingungen - auch bevorzugt H, Ar oder Py.

$R^6$ ist vorzugsweise A, insbesondere $CH_3$, ferner bevorzugt zusammen mit $R^{10}$ eine $-CH_2CH_2-$ oder -CH=CH-Gruppe, weiterhin bevorzugt H.

$R^7$ ist vorzugsweise H, A (insbesondere Methyl, Ethyl, Propyl, Isopropyl), AO-alkyl (insbesondere 2-Methoxyethyl), Ar (insbesondere Phenyl, o-, m- oder p-Methoxyphenyl, o-, m- oder p-Hydroxyphenyl, o-, m- oder p-Methylsulfinylphenyl), Py (insbesondere 3-, ferner 2- oder 4-Pyridyl), $R^{12}R^{13}N$-alkyl (insbesondere 2-Dimethylaminoethyl, 2-Diethylaminoethyl oder 2-Morpholinoethyl), Ar-alkyl [insbesondere Benzyl, o-, m- oder p-Methoxybenzyl, 2-Phenylethyl, 2-p-Methoxyphenylethyl, 2-(3,4-Dimethoxyphenyl)-ethyl], Indanyl, Py-alkyl [insbesondere 2-(2-Pyridyl)-ethyl, 2-(3-Pyridyl)-ethyl oder 2-(4-Pyridyl)-ethyl] oder 1-$R^{14}$-piperidyl (insbesondere 1-Benzyl-4-piperidyl), ferner bevorzugt Alkenyl (insbesondere Allyl), Hydroxyalkyl (insbesondere 2-Hydroxyethyl) oder Dihydroxyalkyl (insbesondere 2,3-Dihydroxypropyl).

$R^8$ ist vorzugsweise H, A (insbesondere Methyl, Ethyl, Propyl, Isopropyl oder Butyl), Ar (insbesondere Phenyl oder o-, m- oder p-Methoxyphenyl), Ar-alkyl (insbesondere Benzyl, o-, m- oder p-Methoxybenzyl oder 2-o-, 2-m-oder 2-p-Methoxyphenylethyl), H-CO-, A-CO- (insbesondere Acetyl, Propionyl oder Butyryl), Ar-CO- (insbesondere Benzoyl oder p-Methoxybenzoyl), Py-CO- (insbesondere Nicotinoyl oder Isonicotinoyl) oder Ar-alkyl-CO- (insbesondere Phenylacetyl oder p-Methoxyphenylacetyl), ferner bevorzugt CONHA (insbesondere N-Methylcarbamoyl).

$R^7$ und $R^8$ zusammen sind bevorzugt $(CH_3)_2N-CH=$ oder eine Alkylengruppe mit 3 bis 5, insbesondere 4 oder 5 C-Atomen, die bevorzugt wie angegeben unterbrochen und/oder substituiert sein kann und im einzelnen vorzugsweise $-(CH_2)_3-$, $-(CH_2)_4-$, $-(CH_2)_5-$, $-(CH_2)_2-CH(CH_3)-(CH_2)_2-$, $-(CH_2)_2-O-(CH_2)_2-$ oder $-(CH_2)_2-NR^{15}-(CH_2)_2-$ bedeutet.

$R^7$ und $R^8$ zusammen bilden also mit dem N-Atom, an das sie gebunden sind, vorzugsweise einen Pyrrolidin-, Piperidin-, Morpholin- oder einen in 4-Stellung durch $R^{15}$ substiuierten Piperazinring, der (sonst) bevorzugt unsubstituiert ist oder bevorzugt durch Carbonylsauerstoff substituiert sein kann.

Besonders bevorzugte Gruppen $NR^7R^8$ sind NA-CO-H und NA-COA (N-Alkyl-alkanoylamido, z.B. N-Methyl-formamido, N-Ethyl-formamido, N-Ethyl-acetamido, N-Isopropyl-propionamido) und N-(Ar-alkyl)-CO-H und N-(Ar-alkyl)-COA (N-Aralkyl-alkanoylamido, z.B. N-Benzyl-formamido, N-Benzyl-acetamido, N-p-Methoxybenzyl-formamido, N-p-Methoxybenzyl-acetamido) sowie $R^{12}R^{13}$N-alkyl-N-CO-H und $R^{12}R^{13}$N-alkyl-N-COA (N-Dialkylaminoalkyl-alkanoylamido, z.B. N-2-Diethylaminoethyl-acetamido).

$R^{10}$ ist vorzugsweise H, ferner vorzugsweise zusammen mit $R^6$ eine $-CH_2CH_2-$ oder -CH=CH-Gruppe.

$R^{11}$ ist vorzugsweise H, A (insbesondere Methyl), $H_2N$-CO-, Ar (insbesondere Phenyl, o-, m- oder p-Methoxyphenyl, o-, m- oder p-Hydroxyphenyl, o-, m- oder p-Nitrophenyl, o-, m- oder p-Diethylphosphonomethylphenyl, 2,4-Dimethoxyphenyl), Py (insbesondere 2-, 3- oder 4-Pyridyl, 4-Methyl-3-pyridyl), Cl oder $NR^7R^8$ (insbesondere die oben angegebenen Gruppen, z.B. $NH_2$; NHA wie Methylamino, Isopropylamino; $(CH_3)_2N$-CH=N-; NH-Ar wie p-Methoxyanilino; $NA_2$ wie Dimethylamino, Diisopropylamino; NA-CO-H wie N-Methylformamido, N-Ethyl-formamido; NA-COA wie N-Ethylpropionamido; N(Ar-alkyl)-COA wie N-p-Methoxybenzylacetamido).

Falls in einer Verbindung der Formel I mehrere Substituenten $R^{11}$ enthalten sind, so können sie gleich oder voneinander verschieden sein.

$R^{12}$ ist vorzugsweise H, A (insbesondere Methyl, Ethyl oder Propyl), H-CO-, A-CO- (insbesondere Acetyl, Propionyl oder Butyryl), Ar-CO- (insbesondere Benzoyl oder p-Methoxybenzoyl) oder Ar-alkyl-CO- (insbesondere Phenylacetyl, p-Methoxyphenylacetyl oder 3,4-Dimethoxyphenylacetyl).

$R^{13}$ ist vorzugsweise H oder A (insbesondere Methyl, Ethyl oder Propyl).

$R^{12}$ und $R^{13}$ zusammen sind vorzugsweise $-(CH_2)_4-$, $-(CH_2)_5-$, $-CH(CH_3)-(CH_2)_4-$, $-CO-(CH_2)_3-$, $-CO-(CH_2)_2-CO-$, $-CO-(CH_2)_4-$, $-CO-(CH_2)_3-CO-$, $-(CH_2)_2-CH(CH_2C_6H_5)-(CH_2)_2-$, $-CH(COOH)-(CH_2)_3-$, $-CH(COOCH_3)-(CH_2)_3-$, $-CH(COOC_2H_5)-(CH_2)_3-$, $-CH(COOCH_2C_6H_5)-(CH_2)_3-$, $-(CH_2)_2-O-(CH_2)_2-$, $-(CH_2)_2-NH-(CH_2)_2-$,

$-(CH_2)_2-N(CH_3)-(CH_2)_2-$, $-(CH_2)_2-N(C_2H_5)-(CH_2)_2-$, $-COCH_2NHCH_2CO-$, $-COCH_2N(CH_3)CH_2CO-$, $-COCH_2N(C_2H_5)CH_2CO-$, $-(CO)_2-NH-(CH_2)_2-$, $-(CO)_2-N(CH_3)-(CH_2)_2$, $-(CO)_2-N(C_2H_5)-(CH_2)_2-$, $-CH_2CO-N[CH_2CH_2N(CH_3)_2]-COCH_2-$, $-CO-NH-(CH_2)_2-$, $-CONHCH_2CO-$, $-CON(CH_3)CH_2CO-$, $-CON(C_2H_5)CH_2CO-$.

Besonders bevorzugte Gruppen $R^{12}R^{13}N$-alkyl sind dementsprechend z.B. 2-Aminoethyl, 3-Aminopropyl, 2-Methylaminoethyl, 3-Methylaminopropyl, 2-Ethylaminoethyl, 3-Ethylaminopropyl, 2-Dimethylaminoethyl, 3-Dimethylaminopropyl, 2-Diethylaminoethyl, 3-Diethylaminopropyl, 2-Formamidoethyl, 3-Formamidopropyl, 2-Acetamidoethyl, 3-Acetamidopropyl, 2-Propionamidoethyl, 3-Propionamidopropyl, 2-Butyramidoethyl, 3-Butyramidopropyl, 2-Benzamidoethyl, 3-Benzamidopropyl, 2-p-Methoxybenzamidoethyl, 3-p-Methoxybenzamidopropyl, 2-Phenylacetamidoethyl, 3-Phenylacetamidopropyl, 2-p-Methoxyphenylacetamidoethyl, 3-p-Methoxyphenylacetamidopropyl, 2-(3-Phenylpropionamido)-ethyl, 3-(3-Phenylpropionamido)-propyl, 2-(3-p-Methoxyphenylpropionamido)-ethyl, 3-(3-p-Methoxyphenylpropionamido)-propyl, 2-[3-(3,4-Dimethoxyphenyl)-propionamido]-ethyl, 3-[3-(3,4-Dimethoxyphenyl)-propionamido]-propyl, 2-Pyrrolidinoethyl, 3-Pyrrolidinopropyl, 4-Pyrrolidinobutyl, 2-Piperidinoethyl, 3-Piperidinopropyl, 4-Piperidinobutyl, 2-(2-Methylpiperidino)-ethyl, 3-(2-Methylpiperidino)-propyl, 2-(2-Oxopyrrolidino)-ethyl, 3-(2-Oxopyrrolidino)-propyl, 2-(2,5-Dioxopyrrolidino)-ethyl, 3-(2,5-Dioxopyrrolidino)propyl, 2-(2-Oxopiperidino)-ethyl, 3-(2-Oxopiperidino)-propyl, 2-(2,6-Dioxopiperidino)-ethyl, 3-(2,6-Dioxopiperidino)-propyl, 2-(4-Benzylpiperidino)-ethyl, 3-(4-Benzylpiperidino)-propyl, 2-(2-Carboxypyrrolidino)-ethyl, 3-(2-Carboxypyrrolidino)-propyl, 2-(2-Ethoxycarbonylpyrrolidino)-ethyl, 3-(2-Ethoxycarbonylpyrrolidino)-propyl, 2-(2-Benzyloxycarbonylpyrrolidino)-ethyl, 3-(2-Benzyloxycarbonylpyrrolidino)-propyl, 2-Morpholinoethyl, 3-Morpholinopropyl, 4-Morpholinobutyl, 2-Piperazinoethyl, 3-Piperazinopropyl, 2-(2,6-Dioxopiperazino)-ethyl, 3-(2,6-Dioxopiperazino)-propyl, 2-(4-Methyl-2,6-dioxopiperazino)-ethyl, 3-(4-Methyl-2,6-dioxopiperazino)-propyl, 2-(4-Ethyl-2,6-dioxopiperazino)-ethyl, 3-(4-Ethyl-2,6-dioxopiperazino)-propyl, 2-(2,3-Dioxopiperazino)-ethyl, 3-(2,3-Dioxopiperazino)-propyl, 2-(4-Methyl-2,3-dioxopiperazino)-ethyl, 3-(4-Methyl-2,3-dioxopiperazino)-propyl, 2-(4-Ethyl-2,3-dioxopiperazino)-ethyl, 3-(4-Ethyl-2,3-dioxopiperazino)-propyl, 2-[4-(2-Dimethylaminoethyl)-3,5-dioxopiperazino]-ethyl, 2-(2-Oxo-1-imidazolidinyl)-ethyl, 3-(2-oxo-1-imidazolidinyl)-propyl, 2-(2,5-Dioxo-1-imidazolidinyl)-ethyl, 3-(2,5-Dioxo-1-imidazolidinyl)-propyl, 2-(3-Methyl-2,5-dioxo-1-imidazolidinyl)-ethyl, 3-(3-Methyl-2,5-dioxo-1-imidazolidinyl)-propyl, 2-(3-Ethyl-2,5-dioxo-1-imidazolidinyl)-ethyl, 3-(3-Ethyl-2,5-dioxo-1-imidazolidinyl)-propyl.

$R^{14}$ ist vorzugsweise H, Formyl, Methoxycarbonyl, Ethoxycarbonyl oder Benzyl.

$R^{15}$ ist vorzugsweise H, A (insbesondere Methyl oder Ethyl), 2-Hydroxyethyl, 3-Hydroxypropyl, 2,3-Dihydroxypropyl, $A_2N$-alkyl, z.B. 2-Dimethylaminoethyl, 2-Diethylaminoethyl, 3-Dimethylaminopropyl, Acetyl, Phenyl, o-, m- und p-Methoxyphenyl, 2-Thienyl, Benzyl, p-Methoxybenzyl, $ANH-CO-CH_2-$, z.B. N-Methyl-, N-Ethyl- oder N-Isopropylcarbamoylmethyl, $A_2N-CO-CH_2-$, z.B. N,N-Dimethyl-oder N,N-Diethylcarbamoylmethyl, Benzoyl, o-, m- oder p-Methoxybenzoyl, 2-Furoyl, 2-Thenoyl, Carbamoyl, ANH-CO-, z.B. N-Methyl- oder N-Ethylcarbamoyl, N-Phenylcarbamoyl, N-o-, -m- oder -p-Methoxyphenylcarbamoyl, A-COOmethyl wie Acetoxymethyl.

Besonders bevorzugte Gruppen $R^7R^8N$ (bzw. $R^{12}R^{13}N$), worin $R^7$ und $R^8$ (bzw. $R^{12}$ und $R^{13}$) eine durch $-NR^{15}-$ unterbrochene und wie angegeben substituierte Alkylgruppe bedeuten, sind demnach z.B. Piperazino, 4-Methylpiperazino, 4-Ethylpiperazino, 4-(2,3-Dihydroxypropyl)-piperazino, 2,6-Dioxopiperazino,

4-Methyl-2,6-dioxopiperazino, 4-Ethyl-2,6-dioxopiperazino, 2,3-Dioxopiperazino, 4-Methyl-2,3-dioxopiperazino, 4-Ethyl-2,3-dioxopiperazino, 3,5-Dioxopiperazino, 4-Methyl-3,5-dioxopiperazino, 4-Ethyl-3,5-dioxopiperazino, 4-(2-Dimethylaminoethyl)-3,5-dioxopiperazino, 2-Oxo-1-imidazolidinyl, 2,5-Dioxo-1-imidazolidinyl, 3-Methyl-2,5-dioxo-1-imidazolidinyl, 3-Ethyl-2,5-dioxo-1-imidazolidinyl.

Gegenstand der Erfindung sind insbesondere diejenigen Verbindungen, der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Iz und Iaa bis Iaz ausgedrückt werden, die der Formel I entsprechen und worin die nicht näher bezeichneten Reste die bei der Formel I angegebene Bedeutung haben, worin jedoch

| | | |
|---|---|---|
| in Ia | $R^5$ | $R^9-CHR^{10}CO-$ bedeutet; |
| in Ib | $R^5$ | $Cl-CHR^{10}-CO-$ oder $Br-CHR^{10}CO-$ bedeutet; |
| in Ic | $R^5$ | $R^{17}-CO-CHR^{10}-CO-$ bedeutet; |

in Id $R^5$ $R^{18}$-$CHR^{10}$-CO- bedeutet;
in Ie $R^5$ $R^{19}$-$CHR^{10}$-CO- bedeutet;
in If $R^5$ $A_2N$-CH=$CR^{10}$-CO- bedeutet;
in Ig $R^5$ Ar bedeutet;
in Ih $R^5$ Py bedeutet;
in Ii $R^5$ unsubstituiertes oder ein- oder mehrfach durch $R^{10}$ und/oder $R^{11}$ substituiertes Thiazolyl bedeutet;
in Ij $R^5$ 2-$R^{11}$-5-$R^{10}$-4-thiazolyl bedeutet;
in Ik $R^5$ 2-$R^{11}$-4-thiazolyl bedeutet;
in Il $R^5$ 2-$R^{11}$-5-$R^{10}$-4-thiazolyl und $R^6$ und $R^{10}$ zusammen $-CH_2CH_2-$ bedeuten;
in Im $R^5$ 2-$R^{11}$-5-$R^{10}$-4-thiazolyl und $R^6$ und $R^{10}$ zusammen -CH=CH- bedeuten;
in In $R^5$ 2-$R^{11}$-4-$R^{10}$-5-thiazolyl bedeutet;
in Io $R^5$ unsubstituiertes oder ein- oder mehrfach durch $R^{10}$ und/oder $R^{11}$ substituiertes Imidazolyl bedeutet;
in Ip $R^5$ 2-$R^{11}$-4(5)-imidazolyl bedeutet;
in Iq $R^5$ unsubstituiertes oder ein- oder mehrfach durch $R^{10}$ und/oder $R^{11}$ substituiertes Pyrazolyl bedeutet;
in Ir $R^5$ 1-$R^{10}$-3-pyrazolyl, 1-$R^{11}$-5-$R^{10}$-3-pyrazolyl, 1-$R^{10}$-5-$R^{11}$-3-pyrazolyl, 3-$R^{11}$-5-pyrazolyl, 1-$R^{10}$-3-$R^{11}$-5-pyrazolyl, 1-$R^{11}$-3-$R^{10}$-5-pyrazolyl oder 1,3-Di-$R^{11}$-4-$R^{10}$-5-pyrazolyl bedeutet;
in Is $R^5$ 1,3-Di-$R^{11}$-4-$R^{10}$-5-pyrazolyl und $R^6$ und $R^{10}$ zusammen $-CH_2CH_2-$ bedeuten;
in It $R^5$ 1,3-Di-$R^{11}$-4-$R^{10}$-5-pyrazolyl und $R^6$ und $R^{10}$ zusammen -CH=CH- bedeuten;

in Iu $R^5$ unsubstituiertes oder ein- oder mehrfach durch $R^{10}$ und/oder $R^{11}$ substituiertes Isoxazolyl bedeutet;

in Iv $R^5$ 3-$R^{11}$-4-$R^{10}$-5-isoxazolyl und $R^6$ und $R^{10}$ zusammen $-CH_2CH_2-$ oder -CH=CH- bedeuten;

in Iw $R^5$ unsubstituiertes oder ein- oder mehrfach durch $R^{10}$ und/oder $R^{11}$ substituiertes Imidazopyridyl bedeutet;

in Ix $R^5$ unsubstituiertes oder einfach durch $R^{11}$ substituiertes 2-Imidazo[1,2-a]pyridyl bedeutet;

in Iy $R^5$ unsubstituiertes oder einfach durch $CH_3$, $H_2N$-CO-, CN, Cl, OH oder $NH_2$ substituiertes 2-Imidazo[1,2-a]pyridyl bedeutet;

in Iz $R^4$ Ar-alkyl oder $NR^7R^8$ und $R^5$ H bedeuten.

Bei den Verbindungen der Formel Il handelt es sich um 4,5,6,7-Tetrahydro-thiazolo/4,5 -f/chinolin-7-one (The Ring-Index, Second Edition, American Chemical Society 1960, Nr. 2702), bei denjenigen der Formel Im um 6,7-Dihydro-thiazolo/4,5-f/chinolin-7-one.

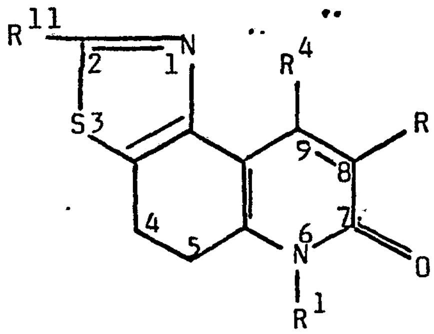

Il

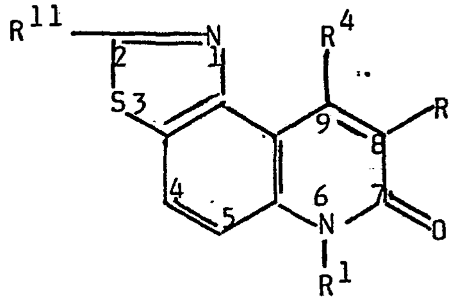

Im

Verbindungen der Formeln Is und It sind dementsprechend 4,5,6,7-Tetrahydro- bzw. 6,7-Dihydro-1H-pyrazolo/3,4-f/-chinolin-7-one (Ring-Index, l.c., 2762):

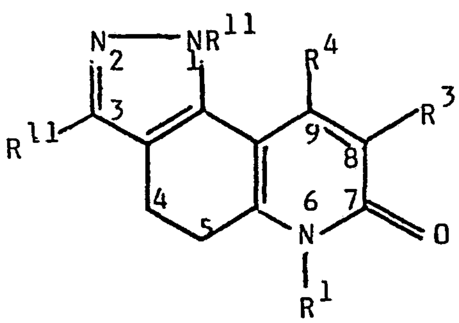

Is

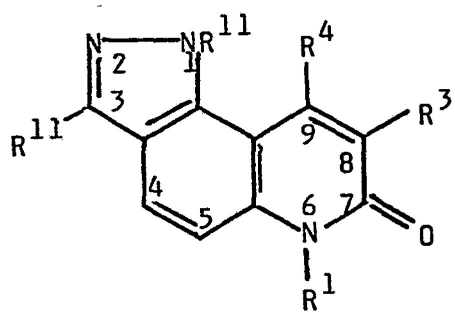

It

Verbindungen der Formel Iv umschließen die nachstehenden 4,5,6,7-Tetrahydro- bzw. 6,7-Dihydro-isoxazolo/4,5-f/-chinolin-7-one (nicht in Ring-Index, l.c., beschrieben):

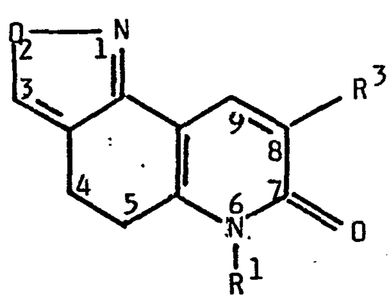

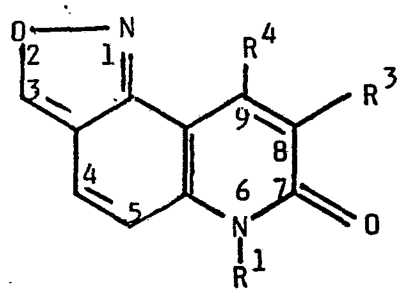

Von den Verbindungen der Formeln Ia bis Iz sind besonders bevorzugt diejenigen der Formel Ii und von diesen wiederum diejenigen der Formeln Ij und vor allem Ik; weiterhin sind Verbindungen der Formeln Ib und Ic, die insbesondere als Zwischenprodukte zur Herstellung anderer Verbindungen der Formel I von Bedeutung sind, sowie der Formeln Il, Im, In, Iq und Iw, darunter insbesondere die der Formel Iy, bevorzugt.

Weitere bevorzugte Verbindungen sind die der Teilformeln Iaa bis Iaz, worin

| | | |
|---|---|---|
| in Iaa | $R^5$ | Br-$CHR^{10}$-CO- bedeutet; |
| in Iab | $R^5$ | Cl-$CHR^{10}$-CO- bedeutet; |
| in Iac | $R^5$ | $CH_3$-CO-$CHR^{10}$-CO- bedeutet; |
| in Iad | $R^5$ | 2-Ar-4-thiazolyl bedeutet; |
| in Iae | $R^5$ | 2-Py-4-thiazolyl bedeutet; |
| in Iaf | $R^5$ | 2-$NR^7R^8$-4-thiazolyl bedeutet; |
| in Iag | $R^5$ | 2-$NR^7R^8$-4-thiazolyl und |
| | $R^7$ und $R^8$ | jeweils H oder A bedeuten; |
| in Iah | $R^5$ | 2-NHA-4-thiazolyl bedeutet; |
| in Iai | $R^5$ | 2-$(CH_3)_2$N-CH=N-4-thiazolyl bedeutet; |
| in Iaj | $R^5$ | 2-NHAr-4-thiazolyl bedeutet; |
| in Iak | $R^5$ | 2-$NA_2$-4-thiazolyl bedeutet; |
| in Ial | $R^5$ | 2-$NR^7R^8$-4-thiazolyl, |
| | $R^7$ | H, A, AO-alkyl, Ar, $R^{12}R^{13}$N-alkyl oder Ar-alkyl und |
| | $R^8$ | H-CO-, A-CO-, Ar-CO- oder Ar-alkyl-CO bedeuten; |
| in Iam | $R^5$ | 2-$NAR^8$-4-thiazolyl und |
| | $R^8$ | H-CO-, A-CO-, Ar-CO- oder Ar-alkyl-CO bedeuten; |
| in Ian | $R^5$ | 2-$NAR^8$-4-thiazolyl und |
| | $R^8$ | H-CO- oder A-CO- bedeuten; |

in Iao $R^5$ 2-$NR^7R^8$-4-thiazolyl,
$R^7$ H, A, AO-alkyl, Ar, $R^{12}R^{13}$N-alkyl oder Ar-alkyl und
$R^8$ H-CO- oder A-CO- bedeuten;

in Iap $R^5$ 2-$NR^7R^8$-4-thiazolyl,
$R^7$ A und
$R^8$ H-CO- oder A-CO- bedeuten;

in Iaq $R^5$ 2-$NR^7R^8$-4-thiazolyl,
$R^7$ A, AO-alkyl, $R^{12}R^{13}$N-alkyl oder Ar-alkyl und
$R^8$ A, Ar oder Ar-alkyl bedeuten;

in Iar $R^5$ 2-$NR^7R^8$-4-thiazolyl,
$R^7$ $R^{12}R^{13}$N-alkyl und
$R^8$ Ar oder Ar-alkyl bedeuten;

in Ias $R^5$ 2-$NR^7R^8$-4-A-5-thiazolyl bedeutet;

in Iat $R^5$ 2-$NR^7R^8$-4-A-5-thiazolyl,
$R^7$ H oder A und
$R^8$ A-CO- bedeuten;

in Iau $R^5$ 2-$NR^7R^8$-5-$R^{10}$-4-thiazolyl und
$R^6$ und $R^{10}$ zusammen $-CH_2CH_2-$ bedeuten;

in Iav $R^5$ 2-$NR^7R^8$-5-$R^{10}$-4-thiazolyl und
$R^6$ und $R^{10}$ zusammen -CH=CH- bedeuten;

in Iaw $R^5$ 2-$NR^7R^8$-5-$R^{10}$-4-thiazolyl,
$R^6$ und $R^{10}$ zusammen -CH=CH-,
$R^7$ H oder A und
$R^8$ H, A, H-CO- oder A-CO- bedeuten;

in Iax $R^5$ 1-$R^{10}$-3-$R^{11}$-5-pyrazolyl,
$R^{10}$ H oder A und
$R^{11}$ H, A oder Py bedeuten;

in Iay $R^5$ 1,3-Di-$R^{11}$-4-$R^{10}$-5-pyrazolyl,
$R^6$ und $R^{10}$ zusammen $-CH_2CH_2-$ und
$R^{11}$ jeweils H, A oder Py bedeuten;

in Iaz $R^5$ 1,3-Di-$R^{11}$-4-$R^{10}$-5-pyrazolyl,
$R^6$ und $R^{10}$ zusammen -CH=CH- und
$R^{11}$ jeweils H, A oder Py bedeuten.

Von diesen Verbindungen sind jeweils diejenigen bevorzugt, in denen A, Ar und Py die oben angegebenen bevorzugten Bedeutungen haben.

Weiterhin sind bevorzugt Verbindungen der Formeln I, Ia bis Iz und Iaa bis Iaz, worin jeweils zusätzlich

(a) $R^1$ H bedeutet;
(b) $R^3$ CN, $CONH_2$ oder $NH_2$ bedeutet;
(c) $R^3$ CN bedeutet;
(d) $R^3$ $CONH_2$ bedeutet;
(e) $R^3$ $NH_2$ bedeutet;
(f) $R^4$ H, OA, SA, $NR^7R^8$ oder $(CH_3)_2N\text{-}CH\text{=}CH\text{-}$ bedeutet;
(g) $R^4$ H bedeutet;
(h) $R^4$ $NR^7R^8$ bedeutet;
(i) $R^6$ A bedeutet;
(j) $R^6$ $CH_3$ bedeutet;
(k) $R^1$ H und
$R^3$ CN, $CONH_2$ oder $NH_2$ bedeuten;
(l) $R^1$ H und
$R^3$ CN bedeuten;
(m) $R^1$ H und
$R^4$ H, OA, SA, $NR^7R^8$ oder $(CH_3)_2N\text{-}CH\text{=}CH\text{-}$ bedeuten;
(n) $R^1$ H und
$R^6$ A bedeuten;
(o) $R^1$ H und
$R^6$ $CH_3$ bedeuten;
(p) $R^1$ H,
$R^3$ CN, $CONH_2$ oder $NH_2$,
$R^4$ H, OA, SA, $NR^7R^8$ oder $(CH_3)_2N\text{-}CH\text{=}CH\text{-}$ und
$R^6$ A bedeuten;
(q) $R^1$ H,
$R^3$ CN,
$R^4$ H, OA, SA, $NR^7R^8$ oder $(CH_3)_2N\text{-}CH\text{=}CH\text{-}$ und
$R^6$ A bedeuten;

(r) $R^1$ H,
$R^3$ CN,
$R^4$ H oder $NR^7R^8$ und
$R^6$ A bedeuten;

(s) $R^1$ H,
$R^3$ CN,
$R^4$ H und
$R^6$ $CH_3$ bedeuten;

(t) $R^1$ H,
$R^3$ CN,
$R^4$ $NR^7R^8$ und
$R^6$ $CH_3$ bedeuten;

(u) $R^1$ H,
$R^3$ $CONH_2$,
$R^4$ H und
$R^6$ $CH_3$ bedeuten;

(v) $R^1$ H,
$R^3$ $CONH_2$,
$R^4$ $NR^7R^8$ und
$R^6$ $CH_3$ bedeuten;

(w) $R^1$ H,
$R^3$ $NH_2$,
$R^4$ H und
$R^6$ $CH_3$ bedeuten.

Bei der Definition dieser Formeln müssen natürlich die oben genannten Einschränkungen berücksichtigt werden.

Im übrigen haben vor- und nachstehend $R^1$ bis $R^{21}$, A, Ar, Ar' und Py die in Patentanspruch 1 oder 3 angegebenen Bedeutungen, sofern nicht ausdrücklich etwas anderes angegeben ist.

Gegenstand der Erfindung ist ferner ein Verfahren zur Herstellung der Verbindungen der Formel I sowie ihrer Lactimester und ihrer Salze, dadurch gekennzeichnet, daß man

3.1 eine Dicarbonylverbindung der Formel II

$$R^6CO-CHR^5-CO-R^4 \qquad II$$

worin

$R^4$, $R^5$ und $R^6$ die in Anspruch 1 angegebenen Bedeutungen haben

oder eines ihrer reaktionsfähigen Derivate mit einer Verbindung der Formel III

$$R^{16}-CH_2-R^3 \qquad III$$

worin

$R^{16}$ $R^1NHCO-$ oder CN bedeutet und die in Anspruch 1 angegebenen Bedeutungen haben

$R^1$ und $R^3$

umsetzt, oder daß man

3.2 ein Amid der Formel IV

$$R^5-CH_2-CR^4=CR^3-CONH_2 \qquad IV$$

worin

$R^3$, $R^4$ und $R^5$ die in Anspruch 1 angegebenen Bedeutungen haben

oder eines seiner reaktionsfähigen Derivate mit einer Verbindung der Formel V

$$X\text{-}CR^6(OA)_2 \qquad V$$

worin

X AO oder $A_2N$ bedeuten und

$R^6$ und A die in Anspruch 1 angegebenen Bedeutungen haben

umsetzt, oder daß man

3.3 ein Keton der Formel VI

$$R^6\text{-}CO\text{-}CH_2\text{-}R^5 \qquad VI$$

worin

$R^5$ und $R^6$ die in Anspruch 1 angegebenen Bedeutungen haben

oder eines seiner reaktionsfähigen Derivate mit einem Nitril der Formel VII

$$Z{=}C{=}C(CN)\text{-}R^{16} \qquad VII$$

worin

Z $(AS)_2$ oder (H,Y) und

Y HO, AO, A-CO-O-, Ar-$SO_2$-O-, $A_2N$ oder $H_2N$-CO-NH-

bedeuten und

A, Ar und $R^{16}$ die in Anspruch 1 bzw. oben angegebenen Bedeutungen haben

umsetzt, und/oder daß man

3.4 ein Keton der Formel I ($R^5 = R^{10}-CH_2-CO-$)

3.4.1 zu einem Haloketon der Formel I ($R^5 = Cl-CHR^{10}-CO-$ oder $Br-CHR^{10}-CO-$) halogeniert oder

3.4.2 mit einem Ester der Formel $R^{17}-COOA$ (worin $R^{17}$, H, AO, A, Ar, Py, Furyl, oder Thienyl bedeutet) zu einer Dicarbonylverbindung der Formel I ($R^5 = R^{17}-CO-CHR^{10}-CO-$) kondensiert, und/oder daß man

3.5 ein Haloketon der Formel I ($R^5 = Cl-CHR^{10}-CO-$ oder $Br-CHR^{10}-CO-$)

3.5.1 mit einer Verbindung der Formel $R^{18}-H$ (worin $R^{18}$ OH, OA, A-COO- oder SCN bedeutet) oder einem ihrer reaktionsfähigen Derivate zu einer Verbindung der Formel I ($R^5 = R^{18}-CHR^{10}-CO-$) umsetzt oder

3.5.2 mit einem Thioamid der Formel $R^{11}-CS-NH_2$ oder einem seiner reaktionsfähigen Derivate zu einem Thiazolderivat der Formel I ($R^5$ = unsubstituiertes oder ein- oder mehrfach durch $R^{10}$ und/oder $R^{11}$ substituiertes Thiazolyl) umsetzt oder

3.5.3 mit einer Verbindung der Formel $R^{19}$ -SM (worin $R^{19}$ 2-Imidazolinylthio oder 2-Benzimidazolylthio bedeutet) oder einem ihrer reaktionsfähigen Derivate zu einer Verbindung der Formel I ($R^5 = R^{19}$-$CHR^{10}$-CO-) umsetzt oder

3.5.4 mit einem Guanidin der Formel $H_2N$-C(=NH)-$NR^7R^8$ ( worin $R^7$ und $R^8$ die in Anspruch 1 angegebenen Bedeutungen haben) oder einem seiner reaktionsfähigen Derivate zu einer Verbindung der Formel I ($R^5$ = 2-$NR^7R^8$-4-imidazolyl) umsetzt oder

3.5.5 mit einem 2-Aminopyridinderivat der Formel VIII

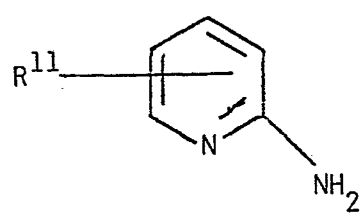

VIII

worin
$R^{11}$ die in Anspruch 1 angegebene Bedeutung hat zu einem Imidazopyridinderivat der Formel I ($R^5$ = unsubstituiertes oder ein- oder mehrfach durch $R^{10}$ und/oder $R^{11}$ substituiertes Imidazo[1,2-a]pyridin-2-yl) umsetzt, oder daß man

3.6 eine Verbindung der Formel I ($R^5$ = HO-$CHR^{10}$-CO-) mit einer Verbindung der Formel $R^{20}$-H (worin $R^{20}$ A-COO-, $CF_3$-COO-, A-$SO_2$-O- oder Ar-$SO_2$-O-

bedeutet) oder einem ihrer reaktionsfähigen Derivate zu einer Verbindung der Formel I ($R^5$ = $R^{20}$-$CHR^{10}$-CO-) umsetzt, oder daß man

3.7 eine 1,3-Dicarbonylverbindung der Formel I ($R^5$ = $R^{17}$-CO-$CHR^{10}$-CO-)

3.7.1 mit einem Hydrazinderivat der Formel $R^{11}$-NH-$NH_2$ zu einem Pyrazolderivat der Formel I ($R^5$ = unsubstituiertes oder ein- oder mehrfach durch $R^{10}$ und/oder $R^{11}$ substituiertes Pyrazolyl) oder

3.7.2 mit Hydroxylamin zu einem Isoxazolderivat der Formel I ($R^5$ = unsubstituiertes oder ein- oder mehrfach durch $R^{10}$ und/oder $R^{11}$ substituiertes Isoxazolyl) umsetzt, oder daß man

3.8 eine Carbonylverbindung der Formel I ($R^5$ = $R^{21}$-$CHR^{10}$-CO-, $R^{21}$ = Cl, Br, A-COO-, $CF_3COO$-, A-$SO_2$-O-oder Ar-$SO_2$-O-) mit einem Amidinderivat der Formel $R^{11}$-C(=NH)-$NH_2$ zu einem Imidazolderivat der Formel I ($R^5$ = unsubstituiertes oder ein- oder mehrfach durch $R^{10}$ und/oder $R^{11}$ substituiertes Imidazolyl)

umsetzt,

und/oder daß man in einer Verbindung der Formel I einen oder mehrere der Reste $R^1$,$R^3$,$R^4$,$R^5$ und/oder $R^6$ in andere Reste $R^1$,$R^3$,$R^4$,$R^5$ und/oder $R^6$ umwandelt, indem man

3.9 eine primäre oder sekundäre Aminogruppe alkyliert, benzyliert oder acyliert und/oder

3.10 eine CN-Gruppe zu einer $CONH_2$- oder einer COOH-Gruppe oder eine Carbamoyl- oder COOA-Gruppe zu einer COOH-Gruppe oder einen Lactimester zum freien Pyridon und/oder eine Dialkylphosphono-Gruppe zu einer Alkylphosphono- oder zu einer Phosphono-Gruppe oder eine Alkylphosphono-Gruppe zu einer Phosphono-Gruppe und/oder eine Thioethergruppe zu einer OH-Gruppe und/oder eine N-Acyl-Gruppe zu einer NH-Gruppe hydrolysiert und/oder

3.11 eine Thioethergruppe mit Ammoniak oder einem Amin der Formel $HNR^7R^8$ in eine $NR^7R^8$-Gruppe umwandelt und/oder

3.12 eine Thioethergruppe mit einem Alkohol oder einem seiner reaktionsfähigen Derivate in eine AO-Gruppe umwandelt und/oder

3.13 eine COOH-Gruppe decarboxyliert und/oder

3.14 eine -CH-CH-Gruppe zu einer -C=C-Gruppe dehydriert und/oder

3.15 eine Thioethergruppe zu einer Sulfoxid- oder zu einer Sulfongruppe oxydiert und/oder

3.16 eine COOH- oder Phosphonsäure-Gruppe verestert und/oder

3.17 eine N-Oxid-Gruppe zur entsprechenden tertiären Aminogruppe reduziert und/oder

3.18 zwei H-Atome durch Kondensation mit einem Amidacetal der Formel $A_2N-CH(OA)$ durch die Gruppe $A_2N-CH=$ ersetzt und/oder

3.19 eine Verbindung der Formel I ($R^5$ = 2-$R^{11}$-5-H-4-thiazolyl, $R^6$ = $CH_3$) mit einem Ameisensäurederivat zu einer Verbindung der Formel I ($R^5$ = 2-$R^{11}$-5-$R^{10}$-4-thiazolyl, $R^6$ und $R^{10}$ zusammen = -CH=CH-) umsetzt und/oder

3.20 zwei H-Atome durch Kondensation mit einem Aldehyd durch die Gruppe Ar-CH= ersetzt und/oder

3.21 eine Carbamoylgruppe durch Behandeln mit Halogen und einer starken Base in eine Aminogruppe umwandelt und/oder

daß man eine Base der Formel I durch Behandeln mit einer Säure in eines ihrer Säureadditionssalze überführt.

Die Verbindungen der Formel I werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart; ferner in den oben angegebenen Literaturstellen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die einzelnen Verfahrensvarianten werden im folgenden näher erläutert.

3.1 Bevorzugte Verbindungen der Formel II sind solche der Formel $CH_3$-CO-$CHR^5$-CHO bzw. $CH_3$-CO-$CR^5$=CHOH. Als reaktionsfähige Derivate eignen sich besonders die Mono-oxo-monoenamine der Formel $R^6$-CO-$CR^5$=$CR^4$-N$(R^{22})_2$, insbesondere $CH_3$-CO-$CR^5$=CH-N$(R^{22})_2$, worin $R^{22}$ A, insbesondere $CH_3$, die beiden Reste $R^{22}$ zusammen auch $-(CH_2)_4-$, $-(CH_2)_5-$ oder $-(CH_2)_2-O-(CH_2)_2-$ bedeuten. Enamine der Formel $R^6$-CO-$CR^5$=CH-$NA_2$ können z.B. aus Carbonylverbindungen der Formel $R^6$-CO-$CH_2$-$R^5$ durch Reaktion mit einem Amidacetal der Formel $A_2N$-CH$(OA)_2$ (worin die Gruppen A gleich oder verschieden sein können) erhalten werden. Weitere gut geeignete reaktionsfähige Derivate sind Enolether, z.B. solche der Formel $R^6$-CO-$CR^5$=$CR^4$-OA, insbesondere $R^6$-CO-$CR^5$=CHOA, sowie zur Herstellung von Verbindungen der Formel I ($R^4$ = SA) die entsprechenden Ketenmercaptale der Formel $R^6$-CO-$CR^5$=C$(SA)_2$.

Bevorzugte Verbindungen der Formel III sind Cyanacetamid und Malodinitril.

Die Verbindungen der Formel II bzw. ihre reaktionsfähigen Derivate werden mit den Verbindungen der Formel III vorzugsweise in Gegenwart eines inerten Lösungsmittels, beispielsweise eines Alkohols wie Methanol oder Ethanol, eines Ethers wie Tetrahydrofuran (THF) oder eines Amids wie Dimethylformamid (DMF) sowie in Gegenwart eines basischen Katalysators, z.B. eines Alkoholats wie Natrium- oder Kaliummethylat, -ethylat oder -propylat oder eines Hydrids wie NaH umgesetzt. Mit Cyanacetamid und den genannten Enaminen erhält man unter diesen Bedingungen Verbindungen der Formel I mit $R^3$ = CN. Arbeitet man dagegen in Essigsäure, so entstehen überwiegend Verbindungen der Formel I mit $R^3 = CONH_2$. Die Reaktionstemperaturen liegen zweckmäßig zwischen etwa 20 und etwa 150 °, vorzugsweise zwischen 60 und 120 °.

3.2 Die Reaktion eines Amids der Formel IV (erhältlich z.B. durch Kondensation einer Carbonylverbindung der Formel $R^5-CH_2-CO-R^4$ mit einem Amid der Formel $R^3-CH_2-CONH_2$, vorzugsweise Cyanacetamid) mit einer Verbindung der Formel V [vorzugsweise einem Amidacetal der Formel $A_2N-CR^6(OA)_2$, aber auch einem Orthoester der Formel $R^6C(OA)_3$] erfolgt zweckmäßig in Gegenwart eines inerten Lösungsmittels wie DMF bei Temperaturen zwischen etwa 100 und etwa 160 °.

3.3 Von den Ketonen der Formel VI sind diejenigen mit $R^6$ = A bevorzugt. Diese sind beispielsweise erhältlich durch Reaktion von Estern der Formel $R^5-CH_2COOA$ mit Säureanhydriden zu Diketonen der Formel $R^5-CH(CO-A)_2$ und nachfolgende Säurespaltung durch Behandeln mit Basen. Als reaktionsfähige Derivate von VI eignen sich z.B. die entsprechenden Enolether, Enolester und Enamine.

Bevorzugte Nitrile der Formel VII sind Enamine des Typs $A_2N-CH=C(CN)-R^{16}$, insbesondere $(CH_3)_2N-CH=C(CN)_2$ und $(CH_3)_2N-CH=C(CN)-CONH_2$, die aus Malodinitril bzw. aus Cyanacetamid mit Amidacetalen der Formel $A_2N-CH(OA)_2$ erhältlich sind. Weiterhin sind Ketenmercaptale der Formel $(AS)_2C=C(CN)-R^{16}$ bevorzugt. Die Reaktion von VI mit VII gelingt zweckmäßig in Gegenwart eines inerten Lösungsmittels, z.B. eines Amids wie DMF oder eines Ethers wie THF, sowie in Gegenwart eines der angegebenen basischen Katalysatoren, z.B. NaH, bei Temperaturen zwischen etwa 20 und etwa 150 °, vorzugsweise zwischen 50 und 100 °.

3.4.1 Chlorierung oder Bromierung von Ketonen der Formel I ($R^5 = R^{10}-CH_2-CO-$) führt zu den entsprechenden Chlor- oder Bromketonen der Formel Ib. Die Ausgangsstoffe sind entweder bekannt, oder sie können nach einem der vorstehend beschriebenen Verfahren hergestellt werden. Die Halogenierung erfolgt zweckmäßig mit elementarem Chlor oder Brom in einem inerten Lösungsmittel, z.B. einem chlorierten Kohlenwasserstoff wie $CH_2Cl_2$, $CHCl_3$, $CCl_4$ oder Trichlorethylen, bei Temperaturen zwischen etwa -10 und etwa 50 °, vorzugsweise bei 10 - 30 °. Ein Zusatz von $CuCl_2$ bzw. $CuBr_2$ ist zweckmäßig. Man kann auch mit einem N-Chlor- oder N-Bromamid-derivat, z.B. N-Bromsuccinimid oder N-Chlorhydantoin, halogenieren, vorteilhaft in einem der genannten chlorierten Kohlenwasserstoffe oder in einem Alkohol bei Temperaturen zwischen etwa -10 und etwa 80 ° unter Bestrahlung und/oder Zusatz eines Radikalbildners, z.B. eines Peroxids wie Benzoylperoxid.

3.4.2 Kondensation von Ketonen der Formel I ($R^5 = R^{10}-CH_2-CO-$) mit Estern der Formel $R^{17}-COOA$ führt

zu entsprechenden Dicarbonylverbindungen der Formel Ic, zweckmäßig in einem inerten Lösungsmittel, z.B. einem Amid wie DMF, oder auch in einem Überschuß des eingesetzten Esters bei Temperaturen zwischen etwa -10 und etwa 100 °, vorzugsweise bei 10 - 50 °. Als Kondensationsmittel eignen sich z.B. die oben (3.1) angegebenen Basen.

3.5.1 Umsetzung der Haloketone der Formel Ib mit Verbindungen der Formel $R^{18}$-H ($H_2O$, Alkoholen der Formel A-OH, Fettsäuren der Formel A-COOH oder HSCN) oder ihren reaktionsfähigen Derivaten, z.B. Alkalien bzw. den entsprechenden Alkoholaten oder Salzen, führt zu den entsprechenden Hydroxy-, Alkoxy-, Alkanoyloxy- oder Thiocyanatoketonen der Formel Id, zweckmäßig in Gegenwart eines inerten Lösungsmittels, z.B. eines Nitrils wie Acetonitril, oder in Gegenwart eines Überschusses der Verbindung der Formel $R^{18}$-H, z.B. eines Alkohols wie Methanol, Ethanol, Isopropanol oder Propanol oder einer Carbonsäure wie Essigsäure, bei Temperaturen zwischen etwa 0 und etwa 150 °, vorzugsweise zwischen 15 und 120 °. So erhält man mit NaOH in Ethanol die entsprechenden Hydroxyketone, mit Na-ethylat in Ethanol dagegen die entsprechenden Ethoxyketone. Zur Herstellung der entsprechenden Alkanoyloxyketone eignet sich zweckmäßig die Umsetzung der Haloketone mit Formamidinium-alkanoaten, wobei die entsprechende Carbonsäure als Lösungsmittel dient, bei Temperaturen um etwa 100 °. Thiocyanate werden z.B. durch Reaktion der Haloketone mit NaSCN oder KSCN in Acetonitril hergestellt.

3.5.2 Besonders wichtig ist die Umsetzung der Haloketone mit Thioamiden oder Thioharnstoffen der Formel $R^{11}$-CS-$NH_2$ zu den bevorzugten Thiazolderivaten der For-

mel Ii, insbesondere Ij oder Ik. Diese Reaktion erfolgt zweckmäßig in einem inerten Lösungsmittel, z.B. einem Alkohol wie Methanol, Ethanol, Propanol, Isopropanol, Butanol, einem Ether wie THF oder Dioxan, einem Amid wie DMF, einem Nitril wie Acetonitril, einem Keton wie Aceton oder in Gemischen dieser Lösungsmittel untereinander und/oder mit Wasser, bei Temperaturen zwischen etwa 10 und etwa 150 °, vorzugsweise zwischen 60 und 120 °.

Verwendet man als Haloketon ein 6-Halo-1,2,5,6,7,8-hexahydrochinolin-2,5-dion der Formel Ib, worin $R^6$ und $R^{10}$ zusammen $-CH_2CH_2-$ bedeuten, so erhält man 4,5,6,7-Tetrahydro-thiazolo[4,5-f]chinolin-7-one der Formel Il. Arbeitet man dabei in DMF als Lösungsmittel, so kann, insbesondere bei höheren Temperaturen (etwa 100 - 130 °), gleichzeitig eine Dehydrierung zu 6,7-Dihydro-thiazolo[4,5-f]chinolin-7-onen der Formel Im erfolgen.

Verwendet man als Thioamid ein Dithiocarbamat wie Ammoniumdithiocarbamat, so erhält man 2-Mercaptothiazolderivate (Ii, $R^{11}$ = SH); mit S-Alkyl-dithiocarbamaten entstehen 2-Alkylthiothiazolderivate (Ii, $R^{11}$ = SA).

3.5.3 Ethylenthioharnstoff und 2-Mercaptobenzimidazol reagieren mit den Haloketonen nicht zu den entsprechenden Thiazolderivaten; es entstehen unter Abspaltung von HCl bzw. HBr die entsprechenden Verbindungen der Formel Ie.

3.5.4 Die Haloketone Ib können mit Guanidinen der Formel $H_2N-C(=NH)-NR^7R^8$ oder deren reaktionsfähigen Derivaten, insbesondere deren Salzen wie Guanidiniumsulfat, zu entsprechenden Imidazolderivaten (I, $R^5$ = $2-NR^7R^8$-4-imida-

zolyl) umgesetzt werden, zweckmäßig in einem inerten Lösungsmittel, z.B. einem Amid wie DMF, in Gegenwart einer der oben (vgl. 3.1) angegebenen Basen wie NaH bei Temperaturen zwischen etwa 0 und etwa 150 °, insbesondere zwischen 10 und 50 °.

3.5.5 Reaktion der Haloketone Ib mit 2-Aminopyridinen der Formel VIII führt zu den entsprechenden Imidazo[1,2-a]pyridinen der Formel Iw, zweckmäßig unter den oben (3.5.2) für die Herstellung der Thiazolderivate der Formel Ii angegebenen Bedingungen.

3.6 Veresterung von Hydroxyketonen der Formel I ($R^5$ = HO-$CHR^{10}$-CO-) mit Carbon- oder Sulfonsäuren der Formel $R^{20}$-H oder ihren reaktionsfähigen Derivaten, z.B. ihren Anhydriden wie Acetanhydrid, Trifluoressigsäureanhydrid, oder ihren Chloriden oder Bromiden wie Acetylchlorid, Propionylbromid, Methan- oder Benzolsulfochlorid führt zu den entsprechenden Ketoestern der Formel I ($R^5$ = $R^{20}$-$CHR^{10}$-CO-). Die Umsetzung erfolgt zweckmäßig in Gegenwart eines inerten Lösungsmittels, z.B. eines Kohlenwasserstoffs wie Toluol oder eines chlorierten Kohlenwasserstoffs wie $CH_2Cl_2$, $CHCl_3$ oder Trichlorethylen und/oder in Gegenwart einer Base wie Triethylamin oder Pyridin, die im Überschuß auch als Lösungsmittel dienen kann, bei Temperaturen zwischen etwa -10 und etwa 150 °, vorzugsweise zwischen 10 und 60 °.

3.7.1 Reaktion von 1,3-Dicarbonylverbindungen der Formel Ic, die z.B. nach 3.4.2 erhältlich sind, mit Hydrazinderivaten der Formel $R^{11}$-NH-$NH_2$ liefert Pyrazolderivate bzw. deren Salze der Formel Iq, zweckmäßig in Gegenwart eines der oben (vgl. 3.5.2) genannten inerten Lösungsmittel, vorzugsweise eines der genannten Alkohole, bei

Temperaturen zwischen etwa -10 ° und etwa 150 °, vorzugsweise zwischen 10 und 100 °, vorteilhaft unter Säurekatalyse (z.B. unter Zusatz von Halogenwasserstoffsäuren, p-Toluolsulfonsäure oder Essigsäure). Mit 6-$R^{17}$-CO-1,2,5,6,7,8-hexahydrochinolin-2,5-dionen (Formel Ic, $R^6$ und $R^{10}$ zusammen = $-CH_2CH_2-$) entstehen 3-$R^{17}$-4,5,6,7-tetrahydro-1H-pyrazolo[3,4-f]-chinolin-7-one der Formel Is (3-$R^{11}$ = $R^{17}$). Aus ß-Ketoestern der Formel I ($R^5$ = AOOC-$CHR^{10}$-CO-) entstehen die entsprechenden 3-Hydroxypyrazolderivate (I, $R^5$ = 1-$R^{11}$-3-hydroxy-4-$R^{10}$-5-pyrazolyl).

3.7.2 Aus den 1,3-Dicarbonylverbindungen der Formel IC entstehen mit Hydroxylamin, das auch in Form seiner Salze eingesetzt werden kann, die entsprechenden Isoxazolderivate der Formeln Iu bzw. Iv, zweckmäßig unter den vorstehend (vgl. 3.7.1) genannten Bedingungen.

3.8 Imidazolderivate der Formel Io sind durch Reaktion von Carbonylverbindungen der Formel I ($R^5$=$R^{21}$-$CHR^{10}$-CO-) mit Amidinderivaten der Formel $R^{11}$-C(=NH)-$NH_2$ erhältlich. Die Amidinderivate entstehen zweckmäßig in situ aus entsprechenden Imidsäureestern der Formel $R^{11}$-C(=NH)-OA unter der Einwirkung von Ammoniak, insbesondere mit flüssigem Ammoniak bei Temperaturen zwischen etwa -20 und etwa 100 °, vorzugsweise zwischen 50 und 90 °.

3.9 Eine primäre oder sekundäre Aminogruppe kann durch Behandlung mit alkylierenden oder benzylierenden Mitteln in die entsprechende sekundäre oder tertiäre Aminogruppe umgewandelt werden. Als alkylierende Mittel eignen sich z.B. Verbindungen der Formel A-Hal (worin Hal Cl, Br oder J bedeutet) oder entsprechende Sulfonsäureester wie Methylchlorid, Methylbromid, Methyljodid, Dimethylsulfat, p-Toluolsulfonsäure-methylester, Ethylchlorid, Ethylbromid, Ethyljodid, Diethylsulfat, n-Propylchlorid, -bromid oder -jodid, Benzylchlorid, -bromid oder -jodid. Man kann ferner mit Aldehyden oder Ketonen unter Bildung von Aldehyd-Ammoniak-Verbindungen oder Schiffschen Basen kondensieren und diese anschließend entweder hydrieren oder mit einem Alkylierungsmittel behandeln und das erhaltene quartäre Salz anschließend hydrolysieren. Beispielsweise kann man ein primäres Amin durch Kondensation mit Benzaldehyd in die N-Benzylidenverbindung überführen und diese mit einem Alkylhalogenid in eines ihrer quartären Salze umwandeln, das nachfolgend z.B. durch Behandeln mit wässerigem Alkohol unter Abspaltung von Benzaldehyd in das sekundäre Amin übergeführt werden kann. Man kann ferner mit Aldehyden oder Ketonen unter reduzierenden Bedingungen alkylieren, z.B. in Gegenwart von Wasserstoff und einem Hydrierungskatalysator, oder in Gegenwart von $NaBH_4$ oder $NaCNBH_3$, wobei als Zwischenprodukte die entsprechenden Aldehyd-Ammoniake entstehen. Beispielsweise kann man eine oder zwei Methylgruppen mit Formaldehyd in Gegenwart von Ameisensäure einführen. Weiterhin kann man mit einem Alkohol, der 1 - 6 C-Atome besitzt, in Gegenwart von Raney-Nickel alkylieren. Die Alkylierung wird zweckmäßig in Gegenwart oder Abwesenheit eines der genannten inerten Lösungsmittel, z.B. DMF, bei Temperaturen zwischen etwa 0 und etwa 120 °, vorzugsweise zwischen 40 und 100 °, vorgenommen, wobei auch ein Katalysator zugegen sein kann, vorzugsweise eine Base wie Kalium-tert.-butylat oder NaH.

Als acylierende Mittel zur Acylierung von primären oder sekundären Aminen der Formel I eignen sich zweckmäßig die Halogenide (z.B. Chloride oder Bromide) oder Anhydride

von Carbonsäuren der Formeln A-COOH, Ar-COOH, Py-COOH, Aralkyl-COOH, z.B. Acetanhydrid, Propionylchlorid, Isobutyrylbromid, Ameisensäure-essigsäureanhydrid, Benzoylchlorid, p-Methoxybenzoylchlorid, Nicotinoylchlorid, Isonicotinoylchlorid. Der Zusatz einer Base wie Pyridin oder Triethylamin bei der Acylierung ist möglich, aber nicht notwendig. Die Acylierung erfolgt zweckmäßig in Gegenwart oder Abwesenheit eines inerten Lösungsmittels, z.B. eines Kohlenwasserstoffs wie Benzol oder Toluol, eines Nitrils wie Acetonitril, eines Amids wie DMF oder eines Überschusses einer tertiären Base wie Pyridin oder Triethylamin bei Temperaturen zwischen etwa 0 und etwa 160 °, vorzugsweise zwischen 20 und 120 °. Behandeln der Amine mit Isocyanaten führt analog zu den entsprechenden Harnstoffen ($R^{11}$ = A-NH-CO-NH).

Wenn man mit überschüssigem Acylierungsmittel in saurem, neutralem oder nur schwach basischem Medium arbeitet, entstehen bei der Acylierung zunächst in der Regel die entsprechenden Lactimester der Formel II, die - besonders im Falle von aromatischen Acylierungsmitteln - isoliert werden können. In alkalischem Medium können sie wieder zu den zugrundeliegenden Pyridonen der Formel I hydrolysiert werden.

3.10 In einer Verbindung der Formel I können, falls erwünscht, funktionelle Gruppen hydrolysiert werden, zweckmäßig durch Behandeln mit Säuren, z.B. $H_2SO_4$, HCl, HBr, $H_3PO_4$, oder mit Basen, z.B. NaOH, KOH, $Na_2CO_3$ oder $K_2CO_3$, in wässeriger oder teilweise wässeriger Lösung, wobei ein zusätzliches inertes Lösungsmittel anwesend sein kann, z.B. ein Alkohol wie Methanol, Ethanol oder Isopropanol. Die Reaktionstemperaturen liegen zwischen etwa 0 und etwa 150 °, vorzugsweise zwischen 15 und 110 °.

Im einzelnen können z.B. Nitrilgruppen mit 50-80 %iger Schwefelsäure zu $CONH_2$-Gruppen oder unter kräftigeren Bedingungen, z.B. bei höheren Temperaturen, zu COOH-Gruppen hydrolysiert werden. Auch die Hydrolyse von $CONH_2$- zu COOH-Gruppen gelingt vorteilhaft mit 50 - 80 %iger Schwefelsäure. Estergruppen können mit verdünnter wässerig-alkoholischer NaOH oder KOH verseift werden. Behandeln der Lactimester mit alkoholischer HCl bei etwa 20 - 80 ° führt zu den zugrundeliegenden 2-Pyridonen; unter diesen Bedingungen bleiben Säureamid- und/oder CN-Gruppen im gleichen Molekül in der Regel unverändert. Behandeln von Phosphonsäuredialkylestern mit alkoholischem HCl oder HBr liefert bei milden Bedingungen (z.B. bei ca. 20 °) die entsprechenden Phosphonsäuremonoalkylester, bei kräftigeren Bedingungen (z.B. bei etwa 60 - 70 °) die Phosphonsäuren. Thioethergruppen, insbesondere solche in 4-Stellung des Pyridonrings, können in basischem Medium in Gegenwart von Spuren Wasser zu OH-Gruppen hydrolysiert werden, z.B. auch mit Na-alkoholaten in nicht ganz wasserfreien Alkoholen. N-Acylgruppen können unter Bildung der freien Amine durch Behandeln mit wässerig-alkoholischen Basen, z.B. wässerig-methanolischer NaOH bei 60 - 80 °, abgespalten werden, wobei gleichzeitig vorhandene 3-CN-Gruppen in der Regel nicht angegriffen werden.

3.11 Behandeln einer Thioethergruppe, besonders einer solchen in 4-Stellung (I, $R^4$ = SA), mit Ammoniak (z.B. flüssigem oder konzentriert-wässerigem $NH_3$) oder einem Amin der Formel $HNR^7R^8$ führt zur entsprechenden $NR^7R^8$-Gruppe, wobei eines der genannten inerten Lösungsmittel anwesend sein kann. Die Reaktionstemperaturen liegen

zwischen etwa 60 und etwa 180 °, vorzugsweise zwischen 110 und 150 °, wobei man erforderlichenfalls unter Druck arbeitet.

3.12 Analog kann eine Thioethergruppe, insbesondere eine solche in 4-Stellung, durch Behandeln mit einem Alkohol oder einem seiner reaktionsfähigen Derivate, z.B. einem entsprechenden Alkalimetallalkoholat, in die entsprechende AO-Gruppe umgewandelt werden. Der auch als Lösungsmittel benutzte Alkohol muß möglichst wasserfrei sein, da sonst eine Hydrolyse zur OH-Gruppe erfolgen kann. Man arbeitet zweckmäßig bei Temperaturen zwischen etwa 0 und etwa 150 °, vorzugsweise zwischen 60 und 120 °.

3.13 Eine COOH-Gruppe, insbesondere eine solche in 3-Stellung kann durch Decarboxylierung entfernt werden, z.B. durch Erhitzen in einer hochsiedenden Base wie Chinolin auf etwa 180 bis etwa 220 °. Ein Zusatz eines Katalysators wie Cu kann vorteilhaft sein. Besonders leicht können 4-Aminopyridon-3-carbonsäuren (I, $R^3$ = COOH, $R^4 = NR^7R^8$) als vinyloge Carbaminsäuren decarboxyliert werden. Zweckmäßig werden sie nicht isoliert; wenn man z.B. die entsprechenden 3-Cyanverbindungen mit Alkali, z.B. verdünnter wässeriger NaOH, behandelt, entstehen direkt die entsprechenden Verbindungen der Formel I ($R^3$ = H, $R^4 = NR^7R^8$).

3.14 Eine -CH-CH-Gruppe in Verbindungen der Formel I ($R^5 = R^9\text{-CHR}^{10}\text{-CO-}$, $R^6$ und $R^{10}$ zusammen = $-CH_2CH_2-$) kann durch einfaches Erhitzen in DMF auf etwa 90 bis 120 ° zu einer -C=C-Gruppe dehydriert werden. Andere geeignete Dehydrierungsmittel sind beispielsweise Schwefel, Selen,

Pt-, Pd-, Ni-, Co-, Ag- oder Cu-Katalysatoren, einzeln oder als Mischkatalysatoren (als Metalle oder auf Trägern wie z.B. Kohle, $CaCO_3$ oder $SrCO_3$), $SeO_2$, Dialkyldisulfide wie Diisoamyldisulfid, Chloranil, konzentrierte Schwefelsäure, $FeCl_3$, $MnO_2$, verdünnte $HNO_3$ oder Nitrobenzol. Man dehydriert zweckmäßig bei Temperaturen zwischen etwa 70 und etwa 300 °. Als zusätzliche Lösungsmittel eignen sich z.B. Mesitylen, p-Cymol, Chinolin oder Acetanilid.

3.15 Eine in einer Verbindung der Formel I vorhandene Thioethergruppe, insbesondere SA-Gruppe, kann zu einer Sulfoxid- oder zu einer Sulfongruppe oxydiert werden, zweckmäßig mit Wasserstoffperoxid, Persäuren wie Perbenzoesäure oder m-Chlorperbenzoesäure oder Cr(VI)-Verbindungen wie Chromsäure in Gegenwart eines inerten Lösungsmittels, z.B. Wasser, einem Alkohol wie Methanol, Ethanol oder Propanol, einem halogenierten Kohlenwasserstoff wie Chloroform, einer Säure wie Essigsäure oder einem Keton wie Aceton, bei Temperaturen zwischen etwa -20 und etwa 100 °, vorzugsweise 40 und 70 °. Wenn man die berechnete Menge des Oxydationsmittels unter relativ milden Bedingungen verwendet, erhält man überwiegend die Sulfoxide. Mit überschüssigem Oxydationsmittel unter kräftigeren Bedingungen erhält man dagegen überwiegend die Sulfone.

3.16 In einer Verbindung der Formel I enthaltene COOH- oder Phosphonsäuregruppen können gewünschtenfalls verestert werden. Zur Veresterung eignen sich Alkohole der Formel A-OH sowie Benzylalkohol und deren reaktionsfähige Derivate. Als reaktionsfähige Derivate kommen insbesondere die entsprechenden Metallalkoholate in Betracht, vorzugsweise die Alkalimetallalkoholate, z.B. Natrium- oder Kaliumalkoholate.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 ° und +250 °, vorzugsweise zwischen -20 ° und +80 °. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen

insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind.

3.17 Ferner gelingt eine Reduktion einer N-Oxidgruppe zur entsprechenden tertiären Aminogruppe, beispielsweise mit einem Schwermetall wie Eisen, zweckmäßig in Pulverform, wobei man in wässeriger Essigsäure bei Temperaturen zwischen etwa 20 und etwa 120 °, vorzugsweise bei Siedetemperatur, arbeiten kann.

3.18 Man kann weiterhin in einer Verbindung der Formel I zwei H-Atome durch Kondensation mit einem Amidacetal der Formel $A_2N\text{-}CH(OA)_2$ durch die Gruppe $A_2N\text{-}CH\text{=}$ ersetzen. Aus primären Aminen entstehen so Verbindungen, die die Gruppierung $A_2N\text{-}CH\text{=}N\text{-}$ enthalten. In dem Amidacetal können die Alkylgruppen gleich oder voneinander verschieden sein; bevorzugt verwendet man Dimethylformamid-dimethylacetal oder -diethylacetal in einem inerten Lösungsmittel wie DMF bei Temperaturen zwischen etwa 50 und etwa 160 °, vorzugsweise zwischen 120 und 150 °.

3.19 Reaktion eines 5-(2-$R^{11}$-5-H-4-thiazolyl)-6-methylpyridons der Formel I mit einem Ameisensäurederivat, bevorzugt einem Amidacetal der Formel $A_2N\text{-}CH(OA)_2$ oder einem Orthoameisensäureester der Formel $HC(OA)_3$, führt

zu einem 2-$R^{11}$-6,7-dihydrothiazolo[4,5-f]chinolin-7-on (Formel I, $R^5$ = 2-$R^{11}$-5-$R^{10}$-4-thiazolyl, $R^6$ und $R^{10}$ zusammen = -CH=CH-):

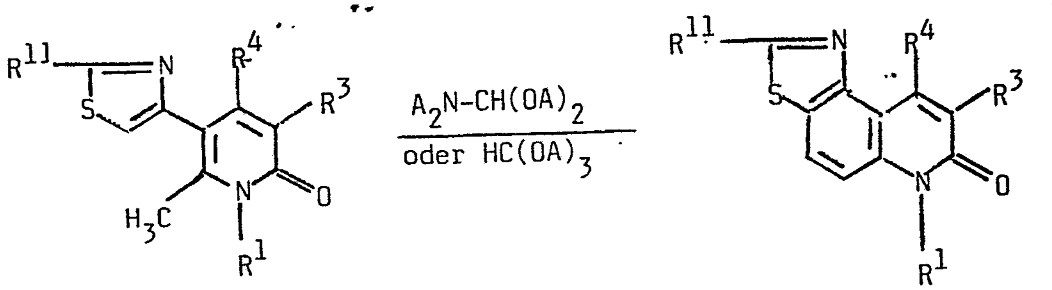


Man kann z.B. in Essigsäure/Acetanhydrid bei Temperaturen zwischen etwa 20 und etwa 140 °, vorzugsweise zwischen 100 und 120 ° arbeiten. Das tricyclische Produkt kann auch nach 3.18 als Nebenprodukt erhalten werden, wenn man eine geeignete Ausgangsverbindung verwendet.

3.20 Verbindungen der Formel I, die eine aktive $CH_2$-Gruppe enthalten (z.B. solche, die einen Rest $R^{11}$= $AOOC-CH_2$- enthalten), können mit Aldehyden der Formel Ar-CHO kondensiert werden, wobei die entsprechenden Benzylidenverbindungen erhalten werden. Die Kondensation kann in Gegenwart saurer oder basischer Katalysatoren vorgenommen werden, z.B. mit Acetanhydrid/Essigsäure oder mit Pyridin, wobei ein Überschuß der Säure oder Base auch als Lösungsmittel dienen kann, bei Temperaturen zwischen etwa 15 und etwa 150 °, vorzugsweise zwischen 80 und 130 °.

3.21 Eine Carbamoylverbindung der Formel I, vorzugsweise eine der Formel I, worin $R^3$ eine Carbamoylgruppe bedeutet, kann durch Behandeln mit Halogen, vorzugsweise Brom oder Chlor, und einer starken Base, vorzugsweise einem Alkalimetallhydroxid wie NaOH oder KOH, in die entsprechende Aminoverbindung umgewandelt werden, vorzugsweise in eine solche der Formel I, worin $R^3$ $NH_2$ bedeutet. Man arbeitet zweckmäßig unter den Bedingungen eines Hofmann-Abbaus, bevorzugt in wässeriger Lösung bei Temperaturen zwischen etwa 40 und etwa 100 °, insbesondere bei etwa 100 °.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch- aktiven Camphersulfonsäuren wie ß-Camphersulfonsäure.

Natürlich ist es auch möglich, optisch-aktive Verbindungen der Formel I nach den oben beschriebenen Methoden zu erhalten, indem man Ausgangsstoffe verwendet, die bereits optisch-aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von Krankheiten, insbesondere der Herzinsuffizienz und der arteriellen Hypertonie sowie ihre Verwendung bei der therapeutischen Behandlung des menschlichen oder tierischen Körpers.

Dabei werden die erfindungsgemäßen Substanzen in der Regel in Analogie zu bekannten positiv inotrop wirksamen Substanzen, wie Sulmazol oder Amrinon, verabreicht, vorzugsweise in Dosierungen zwischen etwa 5 und 500 mg, insbesondere zwischen 20 und 100 mg pro Dosierungseinheit. Die tägliche Dosierung liegt vorzugsweise zwischen etwa 0,1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

Vor- und nachstehend sind alle Temperaturen in °C angegeben.

Beispiel 1

Man erhitzt ein Gemisch von 56 g Cyclohexan-1,3-dion und 60 g Dimethylformamid-dimethylacetal 2,5 Std. auf dem Dampfbad und kühlt ab. Der erhaltenen Lösung von 2-Dimethylaminomethylencyclohexan-1,3-dion werden 300 ml Methanol, 27 g $CH_3ONa$ und 47 g Cyanacetamid zugesetzt. Das Gemisch wird 4 Std. auf 100° erhitzt, in Wasser gegossen und mit Essigsäure angesäuert. Man kühlt, filtriert ab, trocknet und erhält so 3-Cyan-1,2,5,6,7,8-hexahydrochinolin-2,5-dion; Zers. ab 290°.

Analog erhält man mit den entsprechenden N-$R^1$-cyanacetamiden:

1-Methyl-3-cyan-1,2,5,6,7,8-hexahydrochinolin-2,5-dion
1-Ethyl-3-cyan-1,2,5,6,7,8-hexahydrochinolin-2,5-dion
1-Propyl-3-cyan-1,2,5,6,7,8-hexahydrochinolin-2,5-dion
1-Butyl-3-cyan-1,2,5,6,7,8-hexahydrochinolin-2,5-dion
1-Isobutyl-3-cyan-1,2,5,6,7,8-hexahydrochinolin-2,5-dion
1-Hexyl-3-cyan-1,2,5,6,7,8-hexahydrochinolin-2,5-dion
1-Benzyl-3-cyan-1,2,5,6,7,8-hexahydrochinolin-2,5-dion

Beispiel 2

Man löst 0,23 g Na in 40 ml Propanol, gibt 3,09 g 2-(N-Isopropyl-acetamido)-4-methyl-5-(1-dimethylamino-3-oxo-1-buten-2-yl)-thiazol [erhältlich durch Reaktion von 3-Brom-4-oxopentansäureethylester mit N-Isopropylthioharnstoff zu 2-Isopropylamino-4-methyl-thiazol-5-essigsäureethylester, Verseifung zur freien Säure (F. 195-197°), Reaktion mit Acetanhydrid/Pyridin, nachfolgende Säurespaltung zu 2-(N-Isopropylacetamido)-4-methyl-5-(2-oxopropyl)-thiazol und Umsetzung mit Dimethylformamid-diethylacetal] und 0,84 g Cyanacetamid unter Rühren

hinzu, kocht das Gemisch 6 Std., dampft ein, versetzt mit Wasser, dann mit Salzsäure bis pH 2 und erhält 3-Cyan-5-(2-N-isopropyl-acetamido-4-methyl-5-thiazolyl)-6-methyl-2-pyridon, F. 248-250°. Daneben wird 3-Carbamoyl-5-(2-N-isopropyl-acetamido-4-methyl-5-thiazolyl)-6-methyl-2-pyridon isoliert, F. 269-270°.

Analog erhält man:

mit 2-Acetamido-4-methyl-5-(1-dimethylamino-3-oxo-1-buten-2-yl)-thiazol das 3-Cyan-5-(2-acetamido-4-methyl-5-thiazolyl)-6-methyl-2-pyridon, F. > 355°;

mit 2-(N-p-Methoxyphenyl-acetamido)-4-methyl-5-(1-dimethylamino-3-oxo-1-buten-2-yl)-thiazol das 3-Cyan-5-(2-N-methoxyphenyl-acetamido-4-methyl-5-thiazolyl)-6-methyl-2-pyridon;

mit 2-N-Ethyl-propionamido-4-(1-dimethylamino-3-oxo-1-buten-2-yl)-thiazol das 3-Cyan-5-(2-N-ethyl-propionamido-4-thiazolyl)-6-methyl-2-pyridon, F. 263-265°;

mit 2-N-Methyl-acetamido-4-(1-dimethylamino-3-oxo-1-buten-2-yl)-thiazol das 3-Cyan-5-(2-N-methylacetamido-4-thiazolyl)-6-methyl-2-pyridon, F. 320-322°;

mit 2-N-Benzyl-acetamido-4-(1-dimethylamino-3-oxo-1-buten-2-yl)-thiazol das 3-Cyan-5-(2-N-benzylacetamido-4-thiazolyl)-2-pyridon, F. 250-253°.

Beispiel 3

Ein Gemisch von 3,09 g 2-(N-Isopropyl-acetamido)-4-methyl-5-(1-dimethylamino-3-oxo-1-buten-2-yl)-thiazol und 0,95 g Cyanacetamid in 5 ml Essigsäure wird 1 Std. auf 120° erhitzt. Man kühlt ab und versetzt mit Wasser. 3-Carbamoyl-5-(2-N-isopropyl-acetamido-4-methyl-5-thiazolyl)-6-methyl-2-pyridon, F. 269-270°, fällt aus.

Analog erhält man 3-Carbamoyl-5-(2,4-dimethoxyphenyl)-2-pyridon-6-carbonsäuremethylester.

Beispiel 4

Man löst 2,3 g Na in 50 ml Methanol, tropft eine Lösung von 8,4 g Cyanacetamid in 100 ml Methanol hinzu, rührt 5 Min. und gibt eine Lösung von 16,8 g 2-Ethoxymethylen-1,3-cyclohexandion in 60 ml Methanol hinzu. Nach 1std. Kochen dampft man ein und löst das zurückbleibende Na-Salz in wenig Wasser. Nach dem Ansäuern wird das erhaltene 2-(2-Cyan-2-carbamoylethyliden)-cyclohexandion abfiltriert, mit Wasser gewaschen, in 200 ml Ethanol gelöst und 2 Std. gekocht. Nach dem Eindampfen erhält man 3-Cyan-1,2,5,6,7,8-hexahydrochinolin-2,5-dion; Zers. ab 290°.

Beispiel 5

Man löst 2,3 g Na in 350 ml Isopropanol, versetzt unter Rühren mit 8,4 g Cyanacetamid und nach 15 Min. mit 35,1 g 1,1-Dimethylthio-2-(2-p-methoxyphenyl-4-thiazolyl)-1-buten-3-on [erhältlich durch Reaktion von p-Methoxy-thiobenzamid mit 3-Oxo-4-chlorbutansäureethylester zu 2-p-Methoxyphenyl-thiazol-4-essigsäureethylester, Verseifung, Reaktion mit Acetanhydrid/Pyridin zu 2-p-Methoxyphenyl-4-(2,4-dioxo-3-pentyl)-thiazol, Säurespaltung zu 2-p-Methoxyphenyl-4-(2-oxopropyl)-thiazol und Umsetzung mit $CS_2/CH_3J$]. Nach 4std. Kochen dampft man ein, löst das erhaltene Salz in

Wasser und fällt mit 8 %iger Salzsäure das 3-Cyan-4-methylthio-5-(2-p-methoxyphenyl-4-thiazolyl)-6-methyl-2-pyridon.

Beispiel 6

Ein Gemisch von 2,93 g 2-Oxo-3-dimethylaminomethylen-3-(2,4-dimethoxyphenyl)-propionsäuremethylester [erhältlich durch Kondensation von 2,4-Dimethoxybenzaldehyd mit Dimethylaminoessigsäuremethylester zu 2-Dimethylamino-3-(2,4-dimethoxyphenyl)-acrylsäuremethylester, Hydrolyse mit 1 n Salzsäure bei 20° zu 2-Oxo-3-(2,4-dimethoxyphenyl)-propionsäuremethylester und Reaktion mit Dimethylformamid-diethylacetal], 8 g Cyanacetamid, 0,48 g NaH und 100 ml DMF wird bei 110° 4 Std. gerührt. Man gießt auf Eis, säuert mit Salzsäure an und erhält 3-Cyan-5-(2,4-dimethoxyphenyl)-2-pyridon-6-carbonsäuremethylester, F. 207-209°.

Beispiel 7

Man rührt ein Gemisch von 23 g 2-Cyan-3-methyl-4-p-methoxyphenyl-2-butensäureamid (cis-trans-Gemisch; erhältlich durch Kondensation von p-Methoxyphenylaceton mit Cyanacetamid), 16,2 g Dimethylformamid-diethylacetal und 45 ml DMF 6 Std. bei 145° und dampft ein. Nach chromatographischer Trennung (Kieselgel; Elution mit Ethylacetat) erhält man 3-Cyan-4-(2-dimethylaminovinyl)-5-p-methoxyphenyl-2-pyridon (F. 260-261°; aus Ethylacetat), 3-Cyan-4-p-methoxybenzyl-2-pyridon (F. 199-200°; aus wässeriger Essigsäure) und 3-Cyan-4-methyl-5-p-methoxyphenyl-2-pyridon (F. 261-265°; aus wässeriger Essigsäure).

Analog erhält man:

3-Cyan-4-methyl-5-phenyl-2-pyridon
3-Cyan-4-methyl-5-o-tolyl-2-pyridon
3-Cyan-4-methyl-5-m-tolyl-2-pyridon
3-Cyan-4-methyl-5-o-methoxyphenyl-2-pyridon
3-Cyan-4-methyl-5-m-methoxyphenyl-2-pyridon
3-Cyan-4-methyl-5-p-chlorphenyl-2-pyridon
3-Cyan-4-methyl-5-(3,4-dimethoxyphenyl)-2-pyridon
3-Cyan-4-methyl-5-(2-pyridyl)-2-pyridon
3-Cyan-4-methyl-5-(3-pyridyl)-2-pyridon
3-Cyan-4-methyl-5-(4-pyridyl)-2-pyridon
3-Cyan-4-(2-dimethylaminovinyl)-5-phenyl-2-pyridon
3-Cyan-4-(2-dimethylaminovinyl)-5-o-tolyl-2-pyridon
3-Cyan-4-(2-dimethylaminovinyl)-5-m-tolyl-2-pyridon
3-Cyan-4-(2-dimethylaminovinyl)-5-o-methoxyphenyl-2-pyridon
3-Cyan-4-(2-dimethylaminovinyl)-5-m-methoxyphenyl-2-pyridon
3-Cyan-4-(2-dimethylaminovinyl)-5-p-methoxyphenyl-2-pyridon
3-Cyan-4-(2-dimethylaminovinyl)-5-p-chlorphenyl-2-pyridon
3-Cyan-4-(2-dimethylaminovinyl)-5-(3,4-dimethoxyphenyl)-2-pyridon
3-Cyan-4-(2-dimethylaminovinyl)-5-(2-pyridyl)-2-pyridon
3-Cyan-4-(2-dimethylaminovinyl)-5-(3-pyridyl)-2-pyridon
3-Cyan-4-(2-dimethylaminovinyl)-5-(4-pyridyl)-2-pyridon
3-Cyan-4-benzyl-2-pyridon
3-Cyan-4-o-methylbenzyl-2-pyridon
3-Cyan-4-m-methylbenzyl-2-pyridon
3-Cyan-4-o-methoxybenzyl-2-pyridon
3-Cyan-4-m-methoxybenzyl-2-pyridon
3-Cyan-4-p-methoxybenzyl-2-pyridon
3-Cyan-4-p-chlorbenzyl-2-pyridon
3-Cyan-4-(3,4-dimethoxybenzyl)-2-pyridon
3-Cyan-4-(2-pyridylmethyl)-2-pyridon
3-Cyan-4-(3-pyridylmethyl)-2-pyridon
3-Cyan-4-(4-pyridylmethyl)-2-pyridon.

Beispiel 8

Ein Gemisch von 11,2 g Cyclohexan-1,3-dion, 12,1 g Dimethylaminomethylen-malonsäuredinitril, 4,8 g NaH und 250 ml THF wird 6 Std. unter Rühren gekocht. Man konzentriert, gibt Wasser zu, säuert mit Salzsäure an und erhält 3-Cyan-1,2,5,6,7,8-hexahydrochinolin-2,5-dion; Zers. ab 290°.

Analog erhält man aus 2-(2-Oxopropyl)-8-carbamoyl-imidazo[1,2-a]pyridin (erhältlich durch Reaktion von 2-Amino-3-carbamoylpyridin mit 4-Chlor-3-oxo-butansäureethylester zu 8-Carbamoylimidazo[1,2-a]pyridin-2-essigsäureethylester, Reaktion mit Acetanhydrid/Pyridin zu 2-(2,4-Dioxo-3-pentyl)-8-carbamoyl-imidazo[1,2-a]pyridin und Säurespaltung) das 3-Cyan-5-(8-carbamoyl-imidazo[1,2-a]pyridin-2-yl)-6-methyl-2-pyridon, Zers. ab 350°.

Beispiel 9

Man suspendiert 17,6 g 3-Cyan-5-acetyl-6-methyl-2-pyridon (vgl. EP-OS 0089022) in 250 ml Dichlormethan, fügt 0,5 g $CuBr_2$ hinzu und versetzt tropfenweise mit 17,6 g Brom unter Rühren. Nach 12 Std. Rühren bei 20° wird das erhaltene 3-Cyan-5-bromacetyl-6-methyl-2-pyridon abfiltriert und mit wässerigem Methanol gewaschen. F. 238°.

Analog erhält man durch Bromierung der entsprechenden Ketone:

3-Cyan-5-bromacetyl-2-pyridon
3-Cyan-4-methoxy-5-bromacetyl-6-methyl-2-pyridon
3-Cyan-5-(2-brompropionyl)-6-methyl-2-pyridon, F. 229° (Zers.)

3-Cyan-5-(2-brombutyryl)-6-methyl-2-pyridon
3-Cyan-5-bromacetyl-6-ethyl-2-pyridon
3-Cyan-5-(2-brompropionyl)-6-ethyl-2-pyridon
3-Cyan-5-(2-brombutyryl)-6-ethyl-2-pyridon
3-Cyan-5-bromacetyl-6-propyl-2-pyridon
3-Cyan-5-(2-brompropionyl)-6-propyl-2-pyridon
3-Cyan-5-(2-brombutyryl)-6-propyl-2-pyridon
3-Cyan-5-bromacetyl-6-butyl-2-pyridon
3-Cyan-5-(2-brompropionyl)-6-butyl-2-pyridon
3-Cyan-5-(2-brombutyryl)-6-butyl-2-pyridon
3-Cyan-6-brom-1,2,5,6,7,8-hexahydro-chinolin-2,5-dion, F. 225-227°.
3-Carbamoyl-5-bromacetyl-6-methyl-2-pyridon.

Durch Chlorierung sind analog ($CuCl_2$; tropfenweises Versetzen mit einer Lösung der berechneten Menge Chlor in $CH_2Cl_2$) erhältlich:

3-Cyan-5-chloracetyl-6-methyl-2-pyridon
3-Cyan-4-methoxy-5-chloracetyl-2-pyridon
3-Cyan-5-(2-chlorpropionyl)-2-pyridon
3-Cyan-5-(2-chlorbutyryl)-2-pyridon
3-Cyan-5-chloracetyl-6-ethyl-2-pyridon
3-Cyan-5-(2-chlorpropionyl)-6-ethyl-2-pyridon
3-Cyan-5-(2-chlorbutyryl)-6-ethyl-2-pyridon
3-Cyan-5-chloracetyl-6-propyl-2-pyridon
3-Cyan-5-(2-chlorpropionyl)-6-propyl-2-pyridon
3-Cyan-5-(2-chlorbutyryl)-6-propyl-2-pyridon
3-Cyan-5-chloracetyl-6-butyl-2-pyridon
3-Cyan-5-(2-chlorpropionyl)-6-butyl-2-pyridon
3-Cyan-5-(2-chlorbutyryl)-6-butyl-2-pyridon
3-Cyan-6-chlor-1,2,5,6,7,8-hexahydro-chinolin-2,5-dion
3-Carbamoyl-5-chloracetyl-6-methyl-2-pyridon.

Beispiel 10

Eine Lösung von 1,76 g 3-Cyan-5-acetyl-6-methyl-2-pyridon in 40 ml DMF wird unter Rühren mit 1,2 g NaH, dann nach 30 Min. mit 2 ml Diethylcarbonat versetzt. Anschließend werden weitere 5,3 ml Diethylcarbonat bei 35 - 40 ° zugetropft. Man rührt noch 3,5 Std. bei 20 - 25 °, gießt auf Eis, säuert bis pH 2 an und filtriert das erhaltene 3-Cyan-5-ethoxycarbonylacetyl-6-methyl-2-pyridon ab; F. 174 - 175°.

Analog erhält man:

3-Cyan-5-ethoxycarbonylacetyl-2-pyridon
3-Cyan-5-methoxycarbonylacetyl-6-methyl-2-pyridon
3-Cyan-4-methoxy-5-ethoxycarbonylacetyl-6-methyl-2-pyridon
3-Cyan-5-(2-ethoxycarbonyl-propionyl)-6-methyl-2-pyridon
3-Cyan-5-ethoxycarbonylacetyl-6-ethyl-2-pyridon
3-Cyan-6-ethoxycarbonyl-1,2,5,6,7,8-hexahydro-chinolin-2,5-dion
3-Carbamoyl-5-ethoxycarbonylacetyl-6-methyl-2-pyridon.

Beispiel 11

Man versetzt eine Lösung von 1,76 g 3-Cyan-5-acetyl-6-methyl-2-pyridon in 30 ml DMF mit 1,2 g NaH (80 %ig) und anschließend mit 4,4 g Ethylacetat. Nach 3std. Rühren bei 20° gießt man auf Eis, gibt 2 n HCl bis pH 5,5 hinzu und filtriert das erhaltene 3-Cyan-5-(1,3-dioxobutyl)-6-methyl-2-pyridon ab; F. 222-225°.

Analog erhält man durch Esterkondensation:

3-Cyan-5-formylacetyl-6-methyl-2-pyridon
3-Cyan-5-(1,3-dioxobutyl)-2-pyridon

3-Cyan-5-(1,3-dioxopentyl)-6-methyl-2-pyridon
3-Cyan-5-(1,3-dioxo-3-phenylpropyl)-6-methyl-2-pyridon
3-Cyan-5-(1,3-dioxo-3-p-methoxyphenyl-propyl)-6-methyl-2-pyridon
3-Cyan-5-[1,3-dioxo-3-(2,4-dimethoxyphenyl)-propyl]-6-methyl-2-pyridon
3-Cyan-5-[1,3-dioxo-3-(4-pyridyl)-propyl]-6-methyl-2-pyridon
3-Cyan-5-[1,3-dioxo-3-(2-furyl)-propyl]-6-methyl-2-pyridon
3-Cyan-5-[1,3-dioxo-3-(2-thienyl)-propyl]-6-methyl-2-pyridon
3-Cyan-5-(1,3-dioxobutyl)-6-ethyl-2-pyridon
3-Cyan-5-(1,3-dioxopentyl)-6-ethyl-2-pyridon
3-Cyan-5-(1,3-dioxo-3-phenylpropyl)-6-ethyl-2-pyridon
3-Cyan-5-(1,3-dioxo-3-p-methoxyphenyl-propyl)-6-ethyl-2-pyridon
3-Cyan-5-[1,3-dioxo-3-(2,4-dimethoxyphenyl)-propyl]-6-ethyl-2-pyridon
3-Cyan-5-[1,3-dioxo-3-(4-pyridyl)-propyl]-6-ethyl-2-pyridon
3-Cyan-5-[1,3-dioxo-3-(2-furyl)-propyl]-6-ethyl-2-pyridon
3-Cyan-5-[1,3-dioxo-3-(2-thienyl)-propyl]-6-ethyl-2-pyridon
3-Cyan-6-hydroxymethylen-1,2,5,6,7,8-hexahydro-chinolin-2,5-dion
3-Carbamoyl-6-acetyl-1,2,5,6,7,8-hexahydro-chinolin-2,5-dion
3-Carbamoyl-6-(2,4-dimethoxybenzoyl)-1,2,5,6,7,8-hexahydro-chinolin-2,5-dion
3-Carbamoyl-6-(4-pyridylcarbonyl)-1,2,5,6,7,8-hexahydro-chinolin-2,5-dion
3-Cyan-6-acetyl-1,2,5,6,7,8-hexahydro-chinolin-2,5-dion, F. 265-268° (Zers.)
3-Cyan-6-propionyl-1,2,5,6,7,8-hexahydro-chinolin-2,5-dion
3-Cyan-6-benzoyl-1,2,5,6,7,8-hexahydro-chinolin-2,5-dion

3-Cyan-6-p-methoxybenzoyl-1,2,5,6,7,8-hexahydro-chinolin-2,5-dion

3-Cyan-6-(2,4-dimethoxybenzoyl)-1,2,5,6,7,8-hexahydro-chinolin-2,5-dion, ab 230° Zers.

3-Cyan-6-(4-pyridylcarbonyl)-1,2,5,6,7,8-hexahydro-chinolin-2,5-dion, F. 298-300° (Zers.)

3-Cyan-6-(2-furoyl)-1,2,5,6,7,8-hexahydro-chinolin-2,5-dion

3-Cyan-6-(2-thenoyl)-1,2,5,6,7,8-hexahydro-chinolin-2,5-dion

3-Carbamoyl-5-(1,3-dioxobutyl)-6-methyl-2-pyridon.

Beispiel 12

Eine Suspension von 2,55 g 3-Cyan-5-bromacetyl-6-methyl-2-pyridon und 0,5 g NaOH in 50 ml Ethanol wird 16 Std. bei 20° gerührt. Nach Eindampfen und Waschen des Rückstandes mit Wasser erhält man 3-Cyan-5-hydroxyacetyl-6-methyl-2-pyridon, F. 246-248°.

Analog erhält man durch Hydrolyse der entsprechenden Brom- oder Chlorverbindungen:

3-Cyan-5-hydroxyacetyl-2-pyridon

3-Cyan-4-methoxy-5-hydroxyacetyl-6-methyl-2-pyridon

3-Cyan-5-(2-hydroxypropionyl)-6-methyl-2-pyridon

3-Cyan-5-(2-hydroxybutyryl)-6-methyl-2-pyridon

3-Cyan-5-hydroxyacetyl-6-ethyl-2-pyridon

3-Cyan-5-(2-hydroxypropionyl)-6-ethyl-2-pyridon

3-Cyan-5-(2-hydroxybutyryl)-6-ethyl-2-pyridon

3-Cyan-5-hydroxyacetyl-6-propyl-2-pyridon

3-Cyan-5-(2-hydroxypropionyl)-6-propyl-2-pyridon

3-Cyan-5-(2-hydroxybutyryl)-6-propyl-2-pyridon

3-Cyan-5-hydroxyacetyl-6-butyl-2-pyridon

3-Cyan-5-(2-hydroxypropionyl)-6-butyl-2-pyridon
3-Cyan-5-(2-hydroxybutyryl)-6-butyl-2-pyridon
3-Cyan-6-hydroxy-1,2,5,6,7,8-hexahydro-chinolin-2,5-dion
3-Carbamoyl-5-hydroxyacetyl-6-methyl-2-pyridon.

Beispiel 13

Man löst 0,26 g Na in 50 ml absolutem Ethanol, fügt 2,55 g 3-Cyan-5-bromacetyl-6-methyl-2-pyridon hinzu und rührt 16 Std. bei 20°. Nach Eindampfen und Waschen des Rückstandes mit Wasser erhält man 3-Cyan-5-ethoxyacetyl-6-methyl-2-pyridon, F. 167-169°.

Analog erhält man mit den entsprechenden Alkoholen:

3-Cyan-5-ethoxyacetyl-2-pyridon
3-Cyan-5-methoxyacetyl-6-methyl-2-pyridon
3-Cyan-5-propoxyacetyl-6-methyl-2-pyridon
3-Cyan-5-isopropoxyacetyl-6-methyl-2-pyridon
3-Cyan-5-butoxyacetyl-6-methyl-2-pyridon
3-Cyan-5-pentoxyacetyl-6-methyl-2-pyridon
3-Cyan-5-hexoxyacetyl-6-methyl-2-pyridon
3-Cyan-4-methoxy-5-ethoxyacetyl-6-methyl-2-pyridon
3-Cyan-5-(2-ethoxypropionyl)-6-methyl-2-pyridon
3-Cyan-5-ethoxyacetyl-6-ethyl-2-pyridon
3-Cyan-6-ethoxy-1,2,5,6,7,8-hexahydro-chinolin-2,5-dion
3-Carbamoyl-5-ethoxyacetyl-6-methyl-2-pyridon.

Beispiel 14

Ein Gemisch von 2,55 g 3-Cyan-5-bromacetyl-6-methyl-2-pyridon und 1,04 g Formamidiniumacetat in 25 ml Essigsäure wird bei 100 ° 4 Std. gerührt. Man konzentriert die erhaltene Lösung, wobei 3-Cyan-5-acetoxyacetyl-6-methyl-2-pyridon auskristallisiert. F. 243-244 °.

Analog erhält man:

3-Cyan-5-acetoxyacetyl-2-pyridon
3-Cyan-5-acetoxyacetyl-6-ethyl-2-pyridon
3-Cyan-5-acetoxyacetyl-6-propyl-2-pyridon
3-Cyan-5-acetoxyacetyl-6-butyl-2-pyridon
3-Cyan-4-methoxy-5-acetoxyacetyl-6-methyl-2-pyridon
3-Cyan-5-(2-acetoxypropionyl)-6-methyl-2-pyridon
3-Cyan-5-(2-acetoxypropionyl)-6-ethyl-2-pyridon
3-Cyan-5-(2-acetoxypropionyl)-6-propyl-2-pyridon
3-Cyan-6-acetoxy-1,2,5,6,7,8-hexahydro-chinolin-2,5-dion
3-Carbamoyl-5-acetoxyacetyl-6-methyl-2-pyridon
3-Cyan-5-propionyloxyacetyl-6-methyl-2-pyridon.

Beispiel 15

Ein Gemisch von 2,55 g 3-Cyan-5-bromacetyl-6-methyl-2-pyridon, 1,46 g KSCN und 50 ml Acetonitril wird 2 Std. bei 20 ° gerührt. Das erhaltene 3-Cyan-5-thiocyanato-acetyl-6-methyl-2-pyridon wird abfiltriert und mit Wasser gewaschen. F. 218 ° (Zers.).

Analog erhält man

3-Cyan-5-thiocyanatoacetyl-2-pyridon
3-Cyan-5-thiocyanatoacetyl-6-ethyl-2-pyridon
3-Cyan-5-thiocyanatoacetyl-6-propyl-2-pyridon
3-Cyan-5-thiocyanatoacetyl-6-butyl-2-pyridon
3-Cyan-4-methoxy-5-thiocyanatoacetyl-2-pyridon
3-Cyan-5-(2-thiocyanatopropionyl)-6-methyl-2-pyridon
3-Cyan-5-(2-thiocyanatopropionyl)-6-ethyl-2-pyridon
3-Cyan-5-(2-thiocyanatopropionyl)-6-propyl-2-pyridon
3-Cyan-6-thiocyanato-1,2,5,6,7,8-hexahydro-chinolin-2,5-dion
3-Carbamoyl-5-thiocyanatoacetyl-6-ethyl-2-pyridon.

Beispiel 16

Man kocht 2,55 g 3-Cyan-5-bromacetyl-6-methyl-2-pyridon (oder 2,1 g 3-Cyan-5-chloracetyl-6-methyl-2-pyridon) und 0,75 g Thioacetamid in 50 ml Ethanol 5 Std., dampft ein, chromatographiert an Kieselgel ($CH_2Cl_2/CH_3OH$ 9:1) und erhält 3-Cyan-5-(2-methyl-4-thiazolyl)-6-methyl-2-pyridon, F. 288-293 ° (Zers.).

Analog erhält man mit den entsprechenden Thioamiden:

3-Cyan-5-(2-ethyl-4-thiazolyl)-6-methyl-2-pyridon
3-Cyan-5-(2-hexyl-4-thiazolyl)-6-methyl-2-pyridon
3-Cyan-5-(2-allyl-4-thiazolyl)-6-methyl-2-pyridon
3-Cyan-5-(2-ethoxycarbonylmethyl-4-thiazolyl)-6-methyl-2-pyridon, F. 192-194 °
3-Cyan-5-(2-carbamoylmethyl-4-thiazolyl)-6-methyl-2-pyridon, Zers. ab 178 °
3-Cyan-5-(2-cyanmethyl-4-thiazolyl)-6-methyl-2-pyridon, F. 281-283 °
3-Cyan-5-(2-benzyl-4-thiazolyl)-6-methyl-2-pyridon, F. 238-239°
3-Carbamoyl-5-(2-ethoxycarbonylmethyl-4-thiazolyl)-6-methyl-2-pyridon
3-Carbamoyl-5-(2-cyanmethyl-4-thiazolyl)-6-methyl-2-pyridon.

Beispiel 17

Man rührt 2,55 g 3-Cyan-5-bromacetyl-6-methyl-2-pyridon und 1,67 g p-Methoxythiobenzamid 3 Std. bei 100 ° in 50 ml DMF, kühlt ab, filtriert und erhält 3-Cyan-5-(2-p-methoxyphenyl-4-thiazolyl)-6-methyl-2-pyridon, F. > 300 °.

Analog erhält man mit den entsprechenden Thioamiden (wobei man auch Triethylamin zur Bindung des entstehenden HBr zusetzen kann):

3-Cyan-5-(2-phenyl-4-thiazolyl)-6-methyl-2-pyridon, F. 299-308°

3-Cyan-5-(2-o-tolyl-4-thiazolyl)-6-methyl-2-pyridon, F. 246-248°

3-Cyan-5-(2-p-tolyl-4-thiazolyl)-6-methyl-2-pyridon, F. 310-312°

3-Cyan-5-(2-p-ethylphenyl-4-thiazolyl)-6-methyl-2-pyridon

3-Cyan-5-(2-p-ethoxyphenyl-4-thiazolyl)-6-methyl-2-pyridon

3-Cyan-5-(2-p-fluorphenyl-4-thiazolyl)-6-methyl-2-pyridon

3-Cyan-5-(2-p-chlorphenyl-4-thiazolyl)-6-methyl-2-pyridon, F. > 300 °

3-Cyan-5-(2-p-bromphenyl-4-thiazolyl)-6-methyl-2-pyridon

3-Cyan-5-(2-p-jodphenyl-4-thiazolyl)-6-methyl-2-pyridon

3-Cyan-5-(2-m-trifluormethylphenyl-4-thiazolyl)-6-methyl-2-pyridon

3-Cyan-5-(2-o-hydroxyphenyl-4-thiazolyl)-6-methyl-2-pyridon, F. > 300 °

3-Cyan-5-(2-m-hydroxyphenyl-4-thiazolyl)-6-methyl-2-pyridon

3-Cyan-5-(2-p-hydroxyphenyl-4-thiazolyl)-6-methyl-2-pyridon, F. > 300 °

3-Cyan-5-(2-o-nitrophenyl-4-thiazolyl)-6-methyl-2-pyridon, F. 268-277 ° (Zers.)

3-Cyan-5-(2-m-nitrophenyl-4-thiazolyl)-6-methyl-2-pyridon, F. 301-304 °

3-Cyan-5-(2-p-nitrophenyl-4-thiazolyl)-6-methyl-2-pyridon, F. 317-320 °

3-Cyan-5-(2-o-aminophenyl-4-thiazolyl)-6-methyl-2-pyridon, F. 247 °

3-Cyan-5-(2-m-aminophenyl-4-thiazolyl)-6-methyl-2-pyridon, F. > 300 °

3-Cyan-5-(2-p-aminophenyl-4-thiazolyl)-6-methyl-2-pyridon, F. > 300 °

3-Cyan-5-(2-o-methylaminophenyl-4-thiazolyl)-6-methyl-2-pyridon

3-Cyan-5-(2-m-methylaminophenyl-4-thiazolyl)-6-methyl-2-pyridon

3-Cyan-5-(2-p-methylaminophenyl-4-thiazolyl)-6-methyl-2-pyridon

3-Cyan-5-(2-o-dimethylaminophenyl-4-thiazolyl)-6-methyl-2-pyridon

3-Cyan-5-(2-m-dimethylaminophenyl-4-thiazolyl)-6-methyl-2-pyridon

3-Cyan-5-(2-p-dimethylaminophenyl-4-thiazolyl)-6-methyl-2-pyridon, F. > 300 °

3-Cyan-5-(2-p-diethylaminophenyl-4-thiazolyl)-6-methyl-2-pyridon

3-Cyan-5-(2-p-dihexylaminophenyl-4-thiazolyl)-6-methyl-2-pyridon

3-Cyan-5-(2-o-cyanphenyl-4-thiazolyl)-6-methyl-2-pyridon

3-Cyan-5-(2-m-cyanphenyl-4-thiazolyl)-6-methyl-2-pyridon

3-Cyan-5-(2-p-cyanphenyl-4-thiazolyl)-6-methyl-2-pyridon

3-Cyan-5-(2-o-carbamoylphenyl-4-thiazolyl)-6-methyl-2-pyridon

3-Cyan-5-(2-m-carbamoylphenyl-4-thiazolyl)-6-methyl-2-pyridon

3-Cyan-5-(2-p-carbamoylphenyl-4-thiazolyl)-6-methyl-2-pyridon, F. > 300°

3-Cyan-5-(2-p-methylthiophenyl-4-thiazolyl)-6-methyl-2-pyridon

3-Cyan-5-(2-p-hexylthiophenyl-4-thiazolyl)-6-methyl-2-pyridon

3-Cyan-5-(2-p-methylsulfinylphenyl-4-thiazolyl)-6-methyl-2-pyridon

3-Cyan-5-(2-p-ethylsulfonylphenyl-4-thiazolyl)-6-methyl-2-pyridon

3-Cyan-5-(2-o-dimethylphosphonomethylphenyl-4-thiazolyl)-6-methyl-2-pyridon

3-Cyan-5-(2-m-dimethylphosphonomethylphenyl-4-thiazolyl)-6-methyl-2-pyridon

3-Cyan-5-(2-p-dimethylphosphonomethylphenyl-4-thiazolyl)-6-methyl-2-pyridon

3-Cyan-5-(2-o-diethylphosphonomethylphenyl-4-thiazolyl)-6-methyl-2-pyridon, F. 225-229°

3-Cyan-5-(2-m-diethylphosphonomethylphenyl-4-thiazolyl)-6-methyl-2-pyridon, F. 181-183°

3-Cyan-5-(2-p-diethylphosphonomethylphenyl-4-thiazolyl)-6-methyl-2-pyridon, F. 160-162°

3-Cyan-5-(2-o-methoxycarbonylphenyl-4-thiazolyl)-6-methyl-2-pyridon

3-Cyan-5-(2-m-methoxycarbonylphenyl-4-thiazolyl)-6-methyl-2-pyridon

3-Cyan-5-(2-p-methoxycarbonylphenyl-4-thiazolyl)-6-methyl-2-pyridon, F. > 300°

3-Cyan-5-(2-o-ethoxycarbonylphenyl-4-thiazolyl)-6-methyl-2-pyridon

3-Cyan-5-(2-m-ethoxycarbonylphenyl-4-thiazolyl)-6-methyl-2-pyridon

3-Cyan-5-(2-p-ethoxycarbonylphenyl-4-thiazolyl)-6-methyl-2-pyridon, F. > 300°

3-Cyan-5-(2-o-methoxycarbonylmethylphenyl-4-thiazolyl)-6-methyl-2-pyridon

3-Cyan-5-(2-m-methoxycarbonylmethylphenyl-4-thiazolyl)-6-methyl-2-pyridon

3-Cyan-5-(2-p-methoxycarbonylmethylphenyl-4-thiazolyl)-6-methyl-2-pyridon, F. 272°

3-Cyan-5-(2-o-ethoxycarbonylmethylphenyl-4-thiazolyl)-6-methyl-2-pyridon

3-Cyan-5-(2-m-ethoxycarbonylmethylphenyl-4-thiazolyl)-6-methyl-2-pyridon

3-Cyan-5-(2-p-ethoxycarbonylmethylphenyl-4-thiazolyl)-6-methyl-2-pyridon

3-Cyan-5-(2-p-methoxymethylphenyl-4-thiazolyl)-6-methyl-2-pyridon

3-Cyan-5-(2-p-ethoxymethylphenyl-4-thiazolyl)-6-methyl-2-pyridon

3-Cyan-5-[2-p-(2-methoxyethyl)-phenyl-4-thiazolyl]-6-methyl-2-pyridon

3-Cyan-5-(2-p-methylthiomethylphenyl-4-thiazolyl)-6-methyl-2-pyridon

3-Cyan-5-(2-p-ethylthiomethylphenyl-4-thiazolyl)-6-methyl-2-pyridon

3-Cyan-5-[2-p-(2-methylthioethyl)-phenyl-4-thiazolyl]-6-methyl-2-pyridon

3-Cyan-5-[2-(4-biphenylyl)-4-thiazolyl]-6-methyl-2-pyridon

3-Cyan-5-[2-(4'-fluor-4-biphenylyl)-4-thiazolyl]-6-methyl-2-pyridon

3-Cyan-5-(2-p-phenoxyphenyl-4-thiazolyl)-6-methyl-2-pyridon

3-Cyan-5-[2-(p-4-fluorphenoxy-phenyl)-4-thiazolyl]-6-methyl-2-pyridon

3-Cyan-5-(2-p-phenylthiomethyl-phenyl-4-thiazolyl)-6-methyl-2-pyridon

3-Cyan-5-[2-(p-4-fluorphenylthiomethyl-phenyl)-4-thiazolyl]-6-methyl-2-pyridon

3-Cyan-5-[2-(p-2-hydroxyethylphenyl)-4-thiazolyl]-6-methyl-2-pyridon

3-Cyan-5-[2-(2,4-dimethoxyphenyl)-4-thiazolyl]-6-methyl-2-pyridon, F. > 300°

3-Cyan-5-[2-(3,4,5-trimethoxyphenyl)-4-thiazolyl]-6-methyl-2-pyridon

3-Cyan-5-[2-(2-pyridyl)-4-thiazolyl]-6-methyl-2-pyridon, F. > 300°

3-Cyan-5-[2-(3-pyridyl)-4-thiazolyl]-6-methyl-2-pyridon, F. 267-272° (Zers.)

3-Cyan-5-[2-(4-pyridyl)-4-thiazolyl]-6-methyl-2-pyridon, F. > 300°

3-Cyan-5-[2-(4-methyl-3-pyridyl)-4-thiazolyl]-6-methyl-2-pyridon, F. 298-299°

3-Cyan-5-[2-(5-fluor-3-pyridyl)-4-thiazolyl]-6-methyl-2-pyridon

3-Cyan-5-[2-(5-chlor-3-pyridyl)-4-thiazolyl]-6-methyl-2-pyridon

3-Cyan-5-[2-(5-brom-3-pyridyl)-4-thiazolyl]-6-methyl-2-pyridon

3-Cyan-5-[2-(5-jod-3-pyridyl)-4-thiazolyl]-6-methyl-2-pyridon

3-Cyan-5-[2-(5-nitro-3-pyridyl)-4-thiazolyl]-6-methyl-2-pyridon

3-Cyan-5-[2-(5-cyan-3-pyridyl)-4-thiazolyl]-6-methyl-2-pyridon

3-Cyan-5-[2-(5-carbamoyl-3-pyridyl)-4-thiazolyl]-6-methyl-2-pyridon

3-Cyan-5-[2-(5-methoxycarbonyl-3-pyridyl)-4-thiazolyl]-6-methyl-2-pyridon

3-Cyan-5-[2-(2-pyrimidyl)-4-thiazolyl]-6-methyl-2-pyridon

3-Cyan-5-[2-(5-acetyl-6-methyl-2-pyridon-3-yl)-4-thiazolyl]-6-methyl-2-pyridon, F. 264-265°

3-Carbamoyl-5-(2-p-methoxyphenyl-4-thiazolyl)-6-methyl-2-pyridon

3-Carbamoyl-5-(2-p-chlorphenyl-4-thiazolyl)-6-methyl-2-pyridon

3-Carbamoyl-5-(2-o-hydroxyphenyl-4-thiazolyl)-6-methyl-2-pyridon

3-Carbamoyl-5-(2-p-hydroxyphenyl-4-thiazolyl)-6-methyl-2-pyridon

3-Carbamoyl-5-(2-o-nitrophenyl-4-thiazolyl)-6-methyl-2-pyridon

3-Carbamoyl-5-(2-m-nitrophenyl-4-thiazolyl)-6-methyl-2-pyridon

3-Carbamoyl-5-(2-p-nitrophenyl-4-thiazolyl)-6-methyl-2-pyridon

3-Carbamoyl-5-(2-o-aminophenyl-4-thiazolyl)-6-methyl-2-pyridon

3-Carbamoyl-5-(2-m-aminophenyl-4-thiazolyl)-6-methyl-2-pyridon

3-Carbamoyl-5-(2-p-aminophenyl-4-thiazolyl)-6-methyl-2-pyridon

3-Carbamoyl-5-(2-p-dimethylaminophenyl-4-thiazolyl)-6-methyl-2-pyridon

3-Carbamoyl-5-(2-o-diethylphosphonomethylphenyl-4-thiazolyl)-6-methyl-2-pyridon

3-Carbamoyl-5-(2-m-diethylphosphonomethylphenyl-4-thiazolyl)-6-methyl-2-pyridon

3-Carbamoyl-5-(2-p-diethylphosphonomethylphenyl-4-thiazolyl)-6-methyl-2-pyridon

3-Carbamoyl-5-[2-(2,4-dimethoxyphenyl)-4-thiazolyl]-6-methyl-2-pyridon

3-Carbamoyl-5-[2-(2-pyridyl)-4-thiazolyl]-6-methyl-2-pyridon

3-Carbamoyl-5-[2-(3-pyridyl)-4-thiazolyl]-6-methyl-2-pyridon

3-Carbamoyl-5-[2-(4-pyridyl)-4-thiazolyl]-6-methyl-2-pyridon

3-Carbamoyl-5-[2-(4-methyl-2-pyridyl)-4-thiazolyl]-6-methyl-2-pyridon.

Beispiel 18

Analog Beispiel 16 erhält man aus 3-Cyan-6-brom-1,2,5,6,7,8-hexahydro-chinolin-2,5-dion mit Thioformamid das

8-Cyan-4,5,6,7-tetrahydro-thiazolo[4,5-f]chinolin-7-on, F. > 300°.

Analog erhält man mit den entsprechenden Thioamiden die folgenden 8-Cyan-4,5,6,7-tetrahydro-thiazolo[4,5-f]chinolin-7-one:

2-Methyl-, F. > 300°
2-Ethoxycarbonylmethyl-
2-Carbamoylmethyl-
2-Cyanmethyl-,
2-Phenyl-
2-p-Tolyl-, F. > 300°
2-p-Ethylphenyl-
2-p-Methoxyphenyl-, F. > 300°
2-p-Ethoxyphenyl-
2-p-Fluorphenyl-
2-p-Chlorphenyl-
2-p-Bromphenyl-
2-p-Jodphenyl-
2-m-Trifluormethylphenyl-
2-o-Hydroxyphenyl-
2-m-Hydroxyphenyl-
2-p-Hydroxyphenyl-
2-o-Nitrophenyl-
2-m-Nitrophenyl-
2-p-Nitrophenyl-
2-o-Aminophenyl-
2-m-Aminophenyl-
2-p-Aminophenyl-
2-p-Dimethylaminophenyl-
2-o-Dimethylphosphonomethylphenyl-
2-m-Dimethylphosphonomethylphenyl-
2-p-Dimethylphosphonomethylphenyl-

2-o-Diethylphosphonomethylphenyl-, F. 222-225°
2-m-Diethylphosphonomethylphenyl-, F. 228°
2-p-Diethylphosphonomethylphenyl-
2-(2,4-Dimethoxyphenyl)-
2-(2-Pyridyl)-
2-(3-Pyridyl)-
2-(4-Pyridyl)-, F. > 300°
2-(4-Methyl-3-pyridyl)-

sowie die folgenden 8-Carbamoyl-4,5,6,7-tetrahydro-thiazolo[4,5-f]chinolin-7-one (mit 3-Carbamoyl-6-brom-1,2,5,6,7,8-hexahydro-chinolin-2,5-dion):

2-Methyl-
2-p-Tolyl-
2-p-Methoxyphenyl-
2-o-Diethylphosphonomethylphenyl-
2-m-Diethylphosphonomethylphenyl-
2-p-Diethylphosphonomethylphenyl-
2-(4-Pyridyl)-.

Beispiel 19

Analog Beispiel 16 erhält man aus 3-Cyan-5-bromacetyl-6-methyl-2-pyridon mit Ammonium-dithiocarbamat das 3-Cyan-5-(2-mercapto-4-thiazolyl)-6-methyl-2-pyridon, Zers. bei 295°.

Analog erhält man mit S-Methyl-dithiocarbamat das 3-Cyan-5-(2-methylthio-4-thiazolyl)-6-methyl-2-pyridon, F. 258-260°,

sowie aus 3-Carbamoyl-5-bromacetyl-6-methyl-2-pyridon:

3-Carbamoyl-5-(2-mercapto-4-thiazolyl)-6-methyl-2-pyridon
3-Carbamoyl-5-(2-methylthio-4-thiazolyl)-6-methyl-2-pyridon.

Beispiel 20

Analog Beispiel 17 erhält man aus 3-Cyan- bzw. 3-Carbamoyl-4-methoxy-5-bromacetyl-6-methyl-2-pyridon mit den entsprechenden Thioamiden:

3-Cyan-4-methoxy-5-(2-p-methoxyphenyl-4-thiazolyl)-6-methyl-2-pyridon, F. 251-253°
3-Cyan-4-methoxy-5-(2-p-hydroxyphenyl-4-thiazolyl)-6-methyl-2-pyridon
3-Cyan-4-methoxy-5-(2-p-diethylphosphonomethylphenyl-4-thiazolyl)-6-methyl-2-pyridon
3-Cyan-4-methoxy-5-[2-(2-pyridyl)-4-thiazolyl]-6-methyl-2-pyridon
3-Cyan-4-methoxy-5-[2-(3-pyridyl)-4-thiazolyl]-6-methyl-2-pyridon
3-Cyan-4-methoxy-5-[2-(4-pyridyl)-4-thiazolyl]-6-methyl-2-pyridon, F. > 300°.
3-Carbamoyl-4-methoxy-5-(2-p-methoxyphenyl-4-thiazolyl)-6-methyl-2-pyridon
3-Carbamoyl-4-methoxy-5-[2-(4-pyridyl)-4-thiazolyl]-6-methyl-2-pyridon.

Beispiel 21

Man kocht 2,55 g 3-Cyan-5-bromacetyl-6-methyl-2-pyridon und 0,76 g Thioharnstoff 3 Std. in 50 ml Ethanol. Das nach dem Abkühlen ausgefallene Hydrobromid wird abfiltriert und in wenig Wasser gelöst. Durch Zugabe von verdünnter Natronlauge fällt man 3-Cyan-5-(2-amino-4-thiazolyl)-6-methyl-2-pyridon, F. > 290°.

Analog erhält man mit den entsprechenden Thioharnstoffen die folgenden 6-Methyl-2-pyridone:

3-Cyan-5-(2-methylamino-4-thiazolyl)-, F. 275-278°
3-Cyan-5-(2-ethylamino-4-thiazolyl)-, F. 230-235°
3-Cyan-5-(2-propylamino-4-thiazolyl)-
3-Cyan-5-(2-isopropylamino-4-thiazolyl)-, F. 222-224°
3-Cyan-5-(2-butylamino-4-thiazolyl)-
3-Cyan-5-(2-isobutylamino-4-thiazolyl)-
3-Cyan-5-(2-sek.-butylamino-4-thiazolyl)-
3-Cyan-5-(2-tert.-butylamino-4-thiazolyl)-
3-Cyan-5-(2-pentylamino-4-thiazolyl)-
3-Cyan-5-(2-hexylamino-4-thiazolyl)-, F. 159-162°
3-Cyan-5-(2-allylamino-4-thiazolyl)-, F. 220-222°
3-Cyan-5-[2-(N-2-hydroxyethyl-amino)-4-thiazolyl]-, F. 248-251°
3-Cyan-5-[2-(N-3-hydroxypropyl-amino)-4-thiazolyl]-
3-Cyan-5-[2-(N-2,3-dihydroxypropyl-amino)-4-thiazolyl]-
3-Cyan-5-[2-(N-2-dimethylaminoethyl-amino)-4-thiazolyl]-, F. 199-202°
3-Cyan-5-[2-(N-3-dimethylaminopropyl-amino)-4-thiazolyl]-, Hydrochlorid, F. 278-280°
3-Cyan-5-[2-(N-2-diethylaminoethyl-amino)-4-thiazolyl]-, F. 250-254°
3-Cyan-5-[2-(N-2-formamidoethyl-amino)-4-thiazolyl]-
3-Cyan-5-[2-(N-2-acetamidoethyl-amino)-4-thiazolyl]-
3-Cyan-5-[2-(N-2-propionamidoethyl-amino)-4-thiazolyl]-
3-Cyan-5-[2-(N-2-butyramidoethyl-amino)-4-thiazolyl]-
3-Cyan-5-[2-(N-2-benzamidoethyl-amino)-4-thiazolyl]-
3-Cyan-5-[2-(N-2-p-methoxybenzamidoethyl-amino)-4-thiazolyl]-
3-Cyan-5-[2-(N-2-p-methoxyphenylacetamidoethyl-amino)-4-thiazolyl]-
3-Cyan-5-[2-(N-2-(3-p-methoxyphenylpropionamido)-ethyl-amino)-4-thiazolyl]-

3-Cyan-5-[2-(N-2-(3-(3,4-dimethoxyphenyl)-propionamido)-ethyl-amino)-4-thiazolyl]-

3-Cyan-5-[2-(N-2-pyrrolidinoethyl-amino)-4-thiazolyl]-, F. 199°

3-Cyan-5-[2-(N-2-piperidinoethyl-amino)-4-thiazolyl]-, F. 208-210°

3-Cyan-5-[2-(N-3-(2-methylpiperidino)-propyl-amino)-4-thiazolyl]-, Zers. ab 90°

3-Cyan-5-[2-(N-2-(2-oxopyrrolidino)-ethyl-amino)-4-thiazolyl]-

3-Cyan-5-[2-(N-3-(2-oxopyrrolidino)-propyl-amino)-4-thiazolyl]-, F. 178-181°

3-Cyan-5-[2-(N-2-(2,5-dioxopyrrolidino)-ethyl-amino)-4-thiazolyl]-

3-Cyan-5-[2-(N-2-(2-oxopiperidino)-ethyl-amino)-4-thiazolyl]-

3-Cyan-5-[2-(N-2-(2,6-dioxopiperidino)-ethyl-amino)-4-thiazolyl]-

3-Cyan-5-[2-(N-2-(4-benzylpiperidino)-ethyl-amino)-4-thiazolyl]-

3-Cyan-5-[2-(N-2-(2-carboxypyrrolidino)-ethyl-amino)-4-thiazolyl]-

3-Cyan-5-[2-(N-2-(2-ethoxycarbonylpyrrolidino)-ethyl-amino)-4-thiazolyl]-

3-Cyan-5-[2-(N-2-(2-benzyloxycarbonylpyrrolidino)-ethyl-amino)-4-thiazolyl]-

3-Cyan-5-[2-(N-2-morpholinoethyl-amino)-4-thiazolyl]-, F. 245-246°

3-Cyan-5-[2-(N-3-morpholinopropyl-amino)-4-thiazolyl]-, F. 183-185°

3-Cyan-5-[2-(N-2-piperazinoethyl-amino)-4-thiazolyl]-, F. 287-293°

3-Cyan-5-[2-(N-2-(2,6-dioxopiperazino)-ethyl-amino)-4-thiazolyl]-

3-Cyan-5-[2-(N-2-(4-methyl-2,6-dioxopiperazino)-ethyl-amino)-4-thiazolyl]-
3-Cyan-5-[2-(N-2-(4-ethyl-2,6-dioxopiperazino)-ethyl-amino)-4-thiazolyl]-
3-Cyan-5-[2-(N-2-(2,3-dioxopiperazino)-ethyl-amino)-4-thiazolyl]-
3-Cyan-5-[2-(N-2-(4-methyl-2,3-dioxopiperazino)-ethyl-amino)-4-thiazolyl]-
3-Cyan-5-[2-(N-2-(4-ethyl-2,3-dioxopiperazino)-ethyl-amino)-4-thiazolyl]-
3-Cyan-5-[2-(N-2-(3,5-dioxopiperazino)-ethyl-amino)-4-thiazolyl]-
3-Cyan-5-[2-(N-2-(4-methyl-3,5-dioxopiperazino)-ethyl-amino)-4-thiazolyl]-
3-Cyan-5-[2-(N-2-(4-ethyl-3,5-dioxopiperazino)-ethyl-amino)-4-thiazolyl]-
3-Cyan-5-[2-(N-2-(4-(2-dimethylaminoethyl)-3,5-dioxopiperazino)-ethyl-amino)-4-thiazolyl]-
3-Cyan-5-[2-(N-2-(2,5-dioxo-1-imidazolidinyl)-ethyl-amino)-4-thiazolyl]-
3-Cyan-5-[2-(N-2-(3-methyl-2,5-dioxo-1-imidazolidinyl)-ethyl-amino)-4-thiazolyl]-
3-Cyan-5-[2-(N-2-(3-ethyl-2,5-dioxo-1-imidazolidinyl)-ethyl-amino)-4-thiazolyl]-
3-Cyan-5-[2-(N-2-methoxyethyl-amino)-4-thiazolyl]-, F. 192-195°
3-Cyan-5-(2-benzylamino-4-thiazolyl)-, F. 275°
3-Cyan-5-(2-p-methoxybenzyl-amino-4-thiazolyl)-, F. 222-224°; Hydrobromid, F. 196-199°
3-Cyan-5-[2-(2-phenylethyl-amino)-4-thiazolyl]-
3-Cyan-5-[2-(2-p-methoxyphenylethyl-amino)-4-thiazolyl]-, F. 158-162°
3-Cyan-5-[2-(2-(3,4-dimethoxyphenyl)-ethyl-amino)-4-thiazolyl]-, F. 175-178°

BAD ORIGINAL

3-Cyan-5-[2-(N-2-hydroxy-2-phenyl-ethyl-amino)-4-thiazolyl]-
3-Cyan-5-[2-(N-1-indanylamino)-4-thiazolyl]-, F. 230-233°
3-Cyan-5-[2-(N-2-(2-pyridyl)-ethylamino)-4-thiazolyl]-, F. 245-249°
3-Cyan-5-[2-(N-2-(3-pyridyl)-ethylamino)-4-thiazolyl]-
3-Cyan-5-[2-(N-2-(4-pyridyl)-ethylamino)-4-thiazolyl]-, 238-240°
3-Cyan-5-[2-(N-4-piperidylamino)-4-thiazolyl]-
3-Cyan-5-[2-(N-(1-benzyl-4-piperidyl)-amino)-4-thiazolyl]-, F. 187-189°
3-Cyan-5-[2-(N-(1-formyl-4-piperidyl)-amino)-4-thiazolyl]-
3-Cyan-5-[2-(N-(1-ethoxycarbonyl-4-piperidyl)-amino)-4-thiazolyl]-, F. 261-264°
3-Cyan-5-[2-(N-2-(4-methyl-5-imidazolylmethylthio)-ethylamino)-4-thiazolyl]-, F. 143-144°
3-Cyan-5-(2-dimethylamino-4-thiazolyl)-, F. 290-294°
3-Cyan-5-(2-diethylamino-4-thiazolyl)-,
3-Cyan-5-(2-dipropylamino-4-thiazolyl)-
3-Cyan-5-(2-diisopropylamino-4-thiazolyl)-, F. 262-263°
3-Cyan-5-[2-(bis-2-hydroxyethyl-amino)-4-thiazolyl]-, Hydrobromid, F. 244-245°
3-Cyan-5-(2-N-methyl-N-benzyl-amino)-4-thiazolyl-, F. 258-261°
3-Cyan-5-(2-N-ethyl-N-benzyl-amino)-4-thiazolyl-, F. 225-228°
3-Cyan-5-[2-(N-methyl-N-2-(3,4-dimethoxyphenyl)-ethylamino)-4-thiazolyl]-, F. 232-235°
3-Cyan-5-[2-(N-methyl-N-2-dimethylaminoethyl-amino)-4-thiazolyl]-, Hydrobromid, F. 255-257°
3-Cyan-5-[2-(N-ethyl-N-2-dimethylaminoethyl-amino)-4-thiazolyl]-, F. 190-193°
3-Cyan-5-[2-(N-isopropyl-N-2-dimethylaminoethyl-amino)-4-thiazolyl]-, F. 233-236°
3-Cyan-5-[2-(N-benzyl-N-2-dimethylaminoethyl-amino)-4-thiazolyl]-, F. 166-169°

3-Cyan-5-[2-(N-p-methoxybenzyl-N-2-dimethylaminoethyl-amino)-4-thiazolyl]-, F. 156-158°

3-Cyan-5-[2-(N-(3,4-dimethoxybenzyl)-N-2-dimethylamino-ethyl-amino)-4-thiazolyl]-

3-Cyan-5-[2-(N-2-p-methoxyphenylethyl-N-2-dimethylamino-ethyl-amino)-4-thiazolyl]-, F. 145-147°

3-Cyan-5-[2-(N-2-(3,4-dimethoxyphenyl)-ethyl-N-2-dimethylaminoethyl-amino)-4-thiazolyl]-, F. 252-253°

3-Cyan-5-[2-(N-phenyl-N-2-dimethylaminoethyl-amino)-4-thiazolyl]-, F. 215-217°

3-Cyan-5-[2-(N-p-methoxyphenyl-N-2-dimethylaminoethyl-amino)-4-thiazolyl]-

3-Cyan-5-[2-(N-methyl-N-3-dimethylaminopropyl-amino)-4-thiazolyl]-

3-Cyan-5-[2-(N-ethyl-N-3-dimethylaminopropyl-amino)-4-thiazolyl]-

3-Cyan-5-[2-(N-isopropyl-N-3-dimethylaminopropyl-amino)-4-thiazolyl]-

3-Cyan-5-[2-(N-benzyl-N-3-dimethylaminopropyl-amino)-4-thiazolyl]-

3-Cyan-5-[2-(N-p-methoxybenzyl-N-3-dimethylaminopropyl-amino)-4-thiazolyl]-

3-Cyan-5-[2-(N-(3,4-dimethoxybenzyl)-N-3-dimethylaminopropyl-amino)-4-thiazolyl]-

3-Cyan-5-[2-(N-2-p-methoxyphenylethyl-N-3-dimethylaminopropyl-amino)-4-thiazolyl]-

3-Cyan-5-[2-(N-2-(3,4-dimethoxyphenyl)-ethyl-N-3-dimethylaminopropyl-amino)-4-thiazolyl]-

3-Cyan-5-[2-(N-phenyl-N-3-dimethylaminopropyl-amino)-4-thiazolyl]-

3-Cyan-5-[2-(N-p-methoxyphenyl-N-3-dimethylaminopropyl-amino)-4-thiazolyl]-

3-Cyan-5-[2-(N-methyl-N-2-diethylaminoethyl-amino)-4-thiazolyl]-
3-Cyan-5-[2-(N-ethyl-N-2-diethylaminoethyl-amino)-4-thiazolyl]-, F. 155-158°
3-Cyan-5-[2-(N-isopropyl-N-2-diethylaminoethyl-amino)-4-thiazolyl]-
3-Cyan-5-[2-(N-benzyl-N-2-diethylaminoethyl-amino)-4-thiazolyl]-, F. 171-173°
3-Cyan-5-[2-(N-p-methoxybenzyl-N-2-diethylaminoethyl-amino)-4-thiazolyl]-, F. 162-165°
3-Cyan-5-[2-(N-(3,4-dimethoxybenzyl)-N-2-diethylaminoethyl-amino)-4-thiazolyl]-
3-Cyan-5-[2-(N-2-p-methoxyphenylethyl-N-2-diethylaminoethyl-amino)-4-thiazolyl]-, F. 150-153°
3-Cyan-5-[2-(N-2-(3,4-dimethoxyphenyl)-ethyl-N-2-diethylaminoethyl-amino)-4-thiazolyl]-, F. 144-146°
3-Cyan-5-[2-(N-phenyl-N-2-diethylaminoethyl-amino)-4-thiazolyl]-, F. 225-227°
3-Cyan-5-[2-(N-p-methoxyphenyl-N-2-diethylaminoethyl-amino)-4-thiazolyl]-

3-Cyan-5-[2-(N-methyl-N-2-formamidoethyl-amino)-4-thiazolyl]-
3-Cyan-5-[2-(N-ethyl-N-2-formamidoethyl-amino)-4-thiazolyl]-
3-Cyan-5-[2-(N-isopropyl-N-2-formamidoethyl-amino)-4-thiazolyl]-
3-Cyan-5-[2-(N-benzyl-N-2-formamidoethyl-amino)-4-thiazolyl]-
3-Cyan-5-[2-(N-p-methoxybenzyl-N-2-formamidoethyl-amino)-4-thiazolyl]-
3-Cyan-5-[2-(N-(3,4-dimethoxybenzyl)-N-2-formamidoethyl-amino)-4-thiazolyl]-
3-Cyan-5-[2-(N-2-p-methoxyphenylethyl-N-2-formamidoethyl-amino)-4-thiazolyl]-

3-Cyan-5-[2-(N-2-(3,4-dimethoxyphenyl)-ethyl-N-2-formamidoethyl-amino)-4-thiazolyl]-
3-Cyan-5-[2-(N-phenyl-N-2-formamidoethyl-amino)-4-thiazolyl]-
3-Cyan-5-[2-(N-p-methoxyphenyl-N-2-formamidoethyl-amino)-4-thiazolyl]-

3-Cyan-5-[2-(N-methyl-N-2-acetamidoethyl-amino)-4-thiazolyl]-
3-Cyan-5-[2-(N-ethyl-N-2-acetamidoethyl-amino)-4-thiazolyl]-
3-Cyan-5-[2-(N-isopropyl-N-2-acetamidoethyl-amino)-4-thiazolyl]-
3-Cyan-5-[2-(N-benzyl-N-2-acetamidoethyl-amino)-4-thiazolyl]-
3-Cyan-5-[2-(N-p-methoxybenzyl-N-2-acetamidoethyl-amino)-4-thiazolyl]-
3-Cyan-5-[2-(N-(3,4-dimethoxybenzyl)-N-2-acetamidoethyl-amino)-4-thiazolyl]-
3-Cyan-5-[2-(N-2-p-methoxyphenylethyl-N-2-acetamidoethyl-amino)-4-thiazolyl]-
3-Cyan-5-[2-(N-2-(3,4-dimethoxyphenyl)-ethyl-N-2-acetamidoethyl-amino)-4-thiazolyl]-
3-Cyan-5-[2-(N-phenyl-N-2-acetamidoethyl-amino)-4-thiazolyl]-
3-Cyan-5-[2-(N-p-methoxyphenyl-N-2-acetamidoethyl-amino)-4-thiazolyl]-

3-Cyan-5-[2-(N-methyl-N-2-propionamidoethyl-amino)-4-thiazolyl]-
3-Cyan-5-[2-(N-ethyl-N-2-propionamidoethyl-amino)-4-thiazolyl]-
3-Cyan-5-[2-(N-isopropyl-N-2-propionamidoethyl-amino)-4-thiazolyl]-

3-Cyan-5-[2-(N-benzyl-N-2-propionamidoethyl-amino)-4-thiazolyl]-
3-Cyan-5-[2-(N-p-methoxybenzyl-N-2-propionamidoethyl-amino)-4-thiazolyl]-
3-Cyan-5-[2-(N-(3,4-dimethoxybenzyl)-N-2-propionamidoethyl-amino)-4-thiazolyl]-
3-Cyan-5-[2-(N-2-p-methoxyphenylethyl-N-2-propionamidoethyl-amino)-4-thiazolyl]-
3-Cyan-5-[2-(N-2-(3,4-dimethoxyphenyl)-ethyl-N-2-propionamidoethyl-amino)-4-thiazolyl]-
3-Cyan-5-[2-(N-phenyl-N-2-propionamidoethyl-amino)-4-thiazolyl]-
3-Cyan-5-[2-(N-p-methoxyphenyl-N-2-propionamidoethyl-amino)-4-thiazolyl]-

3-Cyan-5-[2-(N-methyl-N-2-butyramidoethyl-amino)-4-thiazolyl]-
3-Cyan-5-[2-(N-ethyl-N-2-butyramidoethyl-amino)-4-thiazolyl]-
3-Cyan-5-[2-(N-isopropyl-N-2-butyramidoethyl-amino)-4-thiazolyl]-
3-Cyan-5-[2-(N-benzyl-N-2-butyramidoethyl-amino)-4-thiazolyl]-
3-Cyan-5-[2-(N-p-methoxybenzyl-N-2-butyramidoethyl-amino)-4-thiazolyl]-
3-Cyan-5-[2-(N-(3,4-dimethoxybenzyl)-N-2-butyramidoethyl-amino)-4-thiazolyl]-
3-Cyan-5-[2-(N-2-p-methoxyphenylethyl-N-2-butyramidoethyl-amino)-4-thiazolyl]-
3-Cyan-5-[2-(N-2-(3,4-dimethoxyphenyl)-ethyl-N-2-butyramidoethyl-amino)-4-thiazolyl]-
3-Cyan-5-[2-(N-phenyl-N-2-butyramidoethyl-amino)-4-thiazolyl]-
3-Cyan-5-[2-(N-p-methoxyphenyl-N-2-butyramidoethyl-amino)-4-thiazolyl]-

3-Cyan-5-[2-(N-methyl-N-2-benzamidoethyl-amino)-4-thiazolyl]-
3-Cyan-5-[2-(N-ethyl-N-2-benzamidoethyl-amino)-4-thiazolyl]-
3-Cyan-5-[2-(N-isopropyl-N-2-benzamidoethyl-amino)-4-thiazolyl]-
3-Cyan-5-[2-(N-benzyl-N-2-benzamidoethyl-amino)-4-thiazolyl]-
3-Cyan-5-[2-(N-p-methoxybenzyl-N-2-benzamidoethyl-amino)-4-thiazolyl]-
3-Cyan-5-[2-(N-(3,4-dimethoxybenzyl)-N-2-benzamidoethyl-amino)-4-thiazolyl]-
3-Cyan-5-[2-(N-2-p-methoxyphenylethyl-N-2-benzamidoethyl-amino)-4-thiazolyl]-
3-Cyan-5-[2-(N-2-(3,4-dimethoxyphenyl)-ethyl-N-2-benzamidoethyl-amino)-4-thiazolyl]-
3-Cyan-5-[2-(N-phenyl-N-2-benzamidoethyl-amino)-4-thiazolyl]-
3-Cyan-5-[2-(N-p-methoxyphenyl-N-2-benzamidoethyl-amino)-4-thiazolyl]-

3-Cyan-5-[2-(N-methyl-N-2-p-methoxybenzamidoethyl-amino)-4-thiazolyl]-
3-Cyan-5-[2-(N-ethyl-N-2-p-methoxybenzamidoethyl-amino)-4-thiazolyl]-
3-Cyan-5-[2-(N-isopropyl-N-2-p-methoxybenzamidoethyl-amino)-4-thiazolyl]-
3-Cyan-5-[2-(N-benzyl-N-2-p-methoxybenzamidoethyl-amino)-4-thiazolyl]-
3-Cyan-5-[2-(N-p-methoxybenzyl-N-2-p-methoxybenzamidoethyl-amino)-4-thiazolyl]-
3-Cyan-5-[2-(N-(3,4-dimethoxybenzyl)-N-2-p-methoxybenzamidoethyl-amino)-4-thiazolyl]-
3-Cyan-5-[2-(N-2-p-methoxyphenylethyl-N-2-p-methoxybenzamidoethyl-amino)-4-thiazolyl]-

3-Cyan-5-[2-(N-2-(3,4-dimethoxyphenyl)-ethyl-N-2-p-methoxybenzamidoethyl-amino)-4-thiazolyl]-
3-Cyan-5-[2-(N-phenyl-N-2-p-methoxybenzamidoethyl-amino)-4-thiazolyl]-
3-Cyan-5-[2-(N-p-methoxyphenyl-N-2-p-methoxybenzamidoethyl-amino)-4-thiazolyl]-

3-Cyan-5-[2-(N-methyl-N-2-p-methoxyphenylacetamidoethyl-amino)-4-thiazolyl]-
3-Cyan-5-[2-(N-ethyl-N-2-p-methoxyphenylacetamidoethyl-amino)-4-thiazolyl]-
3-Cyan-5-[2-(N-isopropyl-N-2-p-methoxyphenylacetamidoethyl-amino)-4-thiazolyl]-
3-Cyan-5-[2-(N-benzyl-N-2-p-methoxyphenylacetamidoethyl-amino)-4-thiazolyl]-
3-Cyan-5-[2-(N-p-methoxybenzyl-N-2-p-methoxyphenylacetamidoethyl-amino)-4-thiazolyl]-
3-Cyan-5-[2-(N-(3,4-dimethoxybenzyl)-N-2-p-methoxyphenylacetamidoethyl-amino)-4-thiazolyl]-
3-Cyan-5-[2-(N-2-p-methoxyphenylethyl-N-2-p-methoxyphenylacetamidoethyl-amino)-4-thiazolyl]-
3-Cyan-5-[2-(N-2-(3,4-dimethoxyphenyl)-ethyl-N-2-p-methoxyphenylacetamidoethyl-amino)-4-thiazolyl]-
3-Cyan-5-[2-(N-phenyl-N-2-p-methoxyphenylacetamidoethyl-amino)-4-thiazolyl]-
3-Cyan-5-[2-(N-p-methoxyphenyl-N-2-p-methoxyphenylacetamidoethyl-amino)-4-thiazolyl]-

3-Cyan-5-[2-(N-methyl-N-2-(3-p-methoxyphenylpropionamido)-ethyl-amino)-4-thiazolyl]-
3-Cyan-5-[2-(N-ethyl-N-2-(3-p-methoxyphenylpropionamido)-ethyl-amino)-4-thiazolyl]-
3-Cyan-5-[2-(N-isopropyl-N-2-(3-p-methoxyphenylpropion-amido)-ethyl-amino)-4-thiazolyl]-

3-Cyan-5-[2-(N-benzyl-N-2-(3-p-methoxyphenylpropionamido)-ethyl-amino)-4-thiazolyl]-
3-Cyan-5-[2-(N-p-methoxybenzyl-N-2-(3-p-methoxyphenyl-propionamido)-ethyl-amino)-4-thiazolyl]-
3-Cyan-5-[2-(N-(3,4-dimethoxybenzyl)-N-2-(3-p-methoxy-phenylpropionamido)-ethyl-amino)-4-thiazolyl]-
3-Cyan-5-[2-(N-2-p-methoxyphenylethyl-N-2-(3-p-methoxy-phenylpropionamido)-ethyl-amino)-4-thiazolyl]-
3-Cyan-5-[2-(N-2-(3,4-dimethoxyphenyl)-ethyl-N-2-(3-p-methoxyphenylpropionamido)-ethyl-amino)-4-thiazolyl]-
3-Cyan-5-[2-(N-phenyl-N-2-(3-p-methoxyphenylpropion-amido)-ethyl-amino)-4-thiazolyl]-
3-Cyan-5-[2-(N-p-methoxyphenyl-N-2-(3-p-methoxyphenyl-propionamido)-ethyl-amino)-4-thiazolyl]-

3-Cyan-5-[2-(N-methyl-N-2-(3-(3,4-dimethoxyphenyl)-propionamido)-ethyl-amino)-4-thiazolyl]-
3-Cyan-5-[2-(N-ethyl-N-2-(3-(3,4-dimethoxyphenyl)-propionamido)-ethyl-amino)-4-thiazolyl]-
3-Cyan-5-[2-(N-isopropyl-N-2-(3-(3,4-dimethoxyphenyl)-propionamido)-ethyl-amino)-4-thiazolyl]-
3-Cyan-5-[2-(N-benzyl-N-2-(3-(3,4-dimethoxyphenyl)-propionamido)-ethyl-amino)-4-thiazolyl]-
3-Cyan-5-[2-(N-p-methoxybenzyl-N-2-(3-(3,4-dimethoxyphenyl)-propionamido)-ethyl-amino)-4-thiazolyl]-
3-Cyan-5-[2-(N-(3,4-dimethoxybenzyl)-N-2-(3-(3,4-dimethoxyphenyl)-propionamido)-ethyl-amino)-4-thiazolyl]-
3-Cyan-5-[2-(N-2-p-methoxyphenylethyl-N-2-(3-(3,4-dimethoxyphenyl)-propionamido)-ethyl-amino)-4-thiazolyl]-
3-Cyan-5-[2-(N-2-(3,4-dimethoxyphenyl)-ethyl-N-2-(3-(3,4-dimethoxyphenyl)-propionamido)-ethyl-amino)-4-thiazolyl]-
3-Cyan-5-[2-(N-phenyl-N-2-(3-(3,4-dimethoxyphenyl)-propionamido)-ethyl-amino)-4-thiazolyl]-
3-Cyan-5-[2-(N-p-methoxyphenyl-N-2-(3-(3,4-dimethoxyphenyl)-propionamido)-ethyl-amino)-4-thiazolyl]-

3-Cyan-5-[2-(N-methyl-N-2-pyrrolidinoethyl-amino)-4-thiazolyl]-
3-Cyan-5-[2-(N-ethyl-N-2-pyrrolidinoethyl-amino)-4-thiazolyl]-
3-Cyan-5-[2-(N-isopropyl-N-2-pyrrolidinoethyl-amino)-4-thiazolyl]-
3-Cyan-5-[2-(N-benzyl-N-2-pyrrolidinoethyl-amino)-4-thiazolyl]-
3-Cyan-5-[2-(N-p-methoxybenzyl-N-2-pyrrolidinoethyl-amino)-4-thiazolyl]-
3-Cyan-5-[2-(N-(3,4-dimethoxybenzyl)-N-2-pyrrolidinoethyl-amino)-4-thiazolyl]-
3-Cyan-5-[2-(N-2-p-methoxyphenylethyl-N-2-pyrrolidinoethyl-amino)-4-thiazolyl]-
3-Cyan-5-[2-(N-2-(3,4-dimethoxyphenyl)-ethyl-N-2-pyrrolidinoethyl-amino)-4-thiazolyl]-
3-Cyan-5-[2-(N-phenyl-N-2-pyrrolidinoethyl-amino)-4-thiazolyl]-
3-Cyan-5-[2-(N-p-methoxyphenyl-N-2-pyrrolidinoethyl-amino)-4-thiazolyl]-

3-Cyan-5-[2-(N-methyl-N-2-piperidinoethyl-amino)-4-thiazolyl]-
3-Cyan-5-[2-(N-ethyl-N-2-piperidinoethyl-amino)-4-thiazolyl]-
3-Cyan-5-[2-(N-isopropyl-N-2-piperidinoethyl-amino)-4-thiazolyl]-
3-Cyan-5-[2-(N-benzyl-N-2-piperidinoethyl-amino)-4-thiazolyl]-
3-Cyan-5-[2-(N-p-methoxybenzyl-N-2-piperidinoethyl-amino)-4-thiazolyl]-
3-Cyan-5-[2-(N-(3,4-dimethoxybenzyl)-N-2-piperidinoethyl-amino)-4-thiazolyl]-
3-Cyan-5-[2-(N-2-p-methoxyphenylethyl-N-2-piperidinoethyl-amino)-4-thiazolyl]-

3-Cyan-5-[2-(N-2-(3,4-dimethoxyphenyl)-ethyl-N-2-piperidinoethyl-amino)-4-thiazolyl]-
3-Cyan-5-[2-(N-phenyl-N-2-piperidinoethyl-amino)-4-thiazolyl]-
3-Cyan-5-[2-(N-p-methoxyphenyl-N-2-piperidinoethyl-amino)-4-thiazolyl]-

3-Cyan-5-[2-(N-methyl-N-2-(2-oxopyrrolidino)-ethyl-amino)-4-thiazolyl]-
3-Cyan-5-[2-(N-ethyl-N-2-(2-oxopyrrolidino)-ethyl-amino)-4-thiazolyl]-
3-Cyan-5-[2-(N-isopropyl-N-2-(2-oxopyrrolidino)-ethyl-amino)-4-thiazolyl]-
3-Cyan-5-[2-(N-benzyl-N-2-(2-oxopyrrolidino)-ethyl-amino)-4-thiazolyl]-
3-Cyan-5-[2-(N-p-methoxybenzyl-N-2-(2-oxopyrrolidino)-ethyl-amino)-4-thiazolyl]-
3-Cyan-5-[2-(N-(3,4-dimethoxybenzyl)-N-2-(2-oxopyrrolidino)-ethyl-amino)-4-thiazolyl]-
3-Cyan-5-[2-(N-2-p-methoxyphenylethyl-N-2-(2-oxopyrrolidino)-ethyl-amino)-4-thiazolyl]-
3-Cyan-5-[2-(N-2-(3,4-dimethoxyphenyl)-ethyl-N-2-(2-oxopyrrolidino)-ethyl-amino)-4-thiazolyl]-
3-Cyan-5-[2-(N-phenyl-N-2-(2-oxopyrrolidino)-ethyl-amino)-4-thiazolyl]-
3-Cyan-5-[2-(N-p-methoxyphenyl-N-2-(2-oxopyrrolidino)-ethyl-amino)-4-thiazolyl]-

3-Cyan-5-[2-(N-methyl-N-2-(2,5-dioxopyrrolidino)-ethyl-amino)-4-thiazolyl]-
3-Cyan-5-[2-(N-ethyl-N-2-(2,5-dioxopyrrolidino)-ethyl-amino)-4-thiazolyl]-
3-Cyan-5-[2-(N-isopropyl-N-2-(2,5-dioxopyrrolidino)-ethyl-amino)-4-thiazolyl]-

3-Cyan-5-[2-(N-benzyl-N-2-(2,5-dioxopyrrolidino)-ethyl-amino)-4-thiazolyl]-

3-Cyan-5-[2-(N-p-methoxybenzyl-N-2-(2,5-dioxopyrrolidino)-ethyl-amino)-4-thiazolyl]-

3-Cyan-5-[2-(N-(3,4-dimethoxybenzyl)-N-2-(2,5-dioxopyrrolidino)-ethyl-amino)-4-thiazolyl]-

3-Cyan-5-[2-(N-2-p-methoxyphenylethyl-N-2-(2,5-dioxopyrrolidino)-ethyl-amino)-4-thiazolyl]-

3-Cyan-5-[2-(N-2-(3,4-dimethoxyphenyl)-ethyl-N-2-(2,5-dioxopyrrolidino)-ethyl-amino)-4-thiazolyl]-

3-Cyan-5-[2-(N-phenyl-N-2-(2,5-dioxopyrrolidino)-ethyl-amino)-4-thiazolyl]-

3-Cyan-5-[2-(N-p-methoxyphenyl-N-2-(2,5-dioxopyrrolidino)-ethyl-amino)-4-thiazolyl]-

3-Cyan-5-[2-(N-methyl-N-2-(2-oxopiperidino)-ethyl-amino)-4-thiazolyl]-

3-Cyan-5-[2-(N-ethyl-N-2-(2-oxopiperidino)-ethyl-amino)-4-thiazolyl]-

3-Cyan-5-[2-(N-isopropyl-N-2-(2-oxopiperidino)-ethyl-amino)-4-thiazolyl]-

3-Cyan-5-[2-(N-benzyl-N-2-(2-oxopiperidino)-ethyl-amino)-4-thiazolyl]-

3-Cyan-5-[2-(N-p-methoxybenzyl-N-2-(2-oxopiperidino)-ethyl-amino)-4-thiazolyl]-

3-Cyan-5-[2-(N-(3,4-dimethoxybenzyl)-N-2-(2-oxopiperidino)-ethyl-amino)-4-thiazolyl]-

3-Cyan-5-[2-(N-2-p-methoxyphenylethyl-N-2-(2-oxopiperidino)-ethyl-amino)-4-thiazolyl]-

3-Cyan-5-[2-(N-2-(3,4-dimethoxyphenyl)-ethyl-N-2-(2-oxopiperidino)-ethyl-amino)-4-thiazolyl]-

3-Cyan-5-[2-(N-phenyl-N-2-(2-oxopiperidino)-ethyl-amino)-4-thiazolyl]-

3-Cyan-5-[2-(N-p-methoxyphenyl-N-2-(2-oxopiperidino)-ethyl-amino)-4-thiazolyl]-

3-Cyan-5-[2-(N-methyl-N-2-(2,6-dioxopiperidino)-ethylamino)-4-thiazolyl]-
3-Cyan-5-[2-(N-ethyl-N-2-(2,6-dioxopiperidino)-ethylamino)-4-thiazolyl]-
3-Cyan-5-[2-(N-isopropyl-N-2-(2,6-dioxopiperidino)-ethylamino)-4-thiazolyl]-
3-Cyan-5-[2-(N-benzyl-N-2-(2,6-dioxopiperidino)-ethylamino)-4-thiazolyl]-
3-Cyan-5-[2-(N-p-methoxybenzyl-N-2-(2,6-dioxopiperidino)-ethylamino)-4-thiazolyl]-
3-Cyan-5-[2-(N-(3,4-dimethoxybenzyl)-N-2-(2,6-dioxopiperidino)-ethylamino)-4-thiazolyl]-
3-Cyan-5-[2-(N-2-p-methoxyphenylethyl-N-2-(2,6-dioxopiperidino)-ethylamino)-4-thiazolyl]-
3-Cyan-5-[2-(N-2-(3,4-dimethoxyphenyl)-ethyl-N-2-(2,6-dioxopiperidino)-ethylamino)-4-thiazolyl]-
3-Cyan-5-[2-(N-phenyl-N-2-(2,6-dioxopiperidino)-ethylamino)-4-thiazolyl]-
3-Cyan-5-[2-(N-p-methoxyphenyl-N-2-(2,6-dioxopiperidino)-ethylamino)-4-thiazolyl]-

3-Cyan-5-[2-(N-methyl-N-2-morpholinoethyl-amino)-4-thiazolyl]-, F. 233°
3-Cyan-5-[2-(N-ethyl-N-2-morpholinoethyl-amino)-4-thiazolyl]-, F. 265-268°
3-Cyan-5-[2-(N-isopropyl-N-2-morpholinoethyl-amino)-4-thiazolyl]-
3-Cyan-5-[2-(N-benzyl-N-2-morpholinoethyl-amino)-4-thiazolyl]-, F. 179-182°
3-Cyan-5-[2-(N-p-methoxybenzyl-N-2-morpholinoethyl-amino)-4-thiazolyl]-, F. 144-148°
3-Cyan-5-[2-(N-phenyl-N-2-morpholinoethyl-amino)-4-thiazolyl]-
3-Cyan-5-[2-(N-p-methoxyphenyl-N-2-morpholinoethyl-amino)-4-thiazolyl]-

3-Cyan-5-[2-(N-methyl-N-2-(2,6-dioxopiperazino)-ethyl-amino-4-thiazolyl]-
3-Cyan-5-[2-(N-ethyl-N-2-(2,6-dioxopiperazino)-ethyl-amino-4-thiazolyl]-
3-Cyan-5-[2-(N-isopropyl-N-2-(2,6-dioxopiperazino)-ethyl-amino-4-thiazolyl]-
3-Cyan-5-[2-(N-benzyl-N-2-(2,6-dioxopiperazino)-ethyl-amino-4-thiazolyl]-
3-Cyan-5-[2-(N-p-methoxybenzyl-N-2-(2,6-dioxopiperazino)-ethyl-amino-4-thiazolyl]-
3-Cyan-5-[2-(N-phenyl-N-2-(2,6-dioxopiperazino)-ethyl-amino-4-thiazolyl]-
3-Cyan-5-[2-(N-p-methoxyphenyl-N-2-(2,6-dioxopiperazino)-ethyl-amino-4-thiazolyl]-

3-Cyan-5-[2-(N-methyl-N-2-(4-methyl-2,6-dioxopiperazino)-ethyl-amino)-4-thiazolyl]-
3-Cyan-5-[2-(N-ethyl-N-2-(4-methyl-2,6-dioxopiperazino)-ethyl-amino)-4-thiazolyl]-
3-Cyan-5-[2-(N-isopropyl-N-2-(4-methyl-2,6-dioxopiperazino)-ethyl-amino)-4-thiazolyl]-
3-Cyan-5-[2-(N-benzyl-N-2-(4-methyl-2,6-dioxopiperazino)-ethyl-amino)-4-thiazolyl]-
3-Cyan-5-[2-(N-p-methoxybenzyl-N-2-(4-methyl-2,6-dioxopiperazino)-ethyl-amino)-4-thiazolyl]-
3-Cyan-5-[2-(N-phenyl-N-2-(4-methyl-2,6-dioxopiperazino)-ethyl-amino)-4-thiazolyl]-
3-Cyan-5-[2-(N-p-methoxyphenyl-N-2-(4-methyl-2,6-dioxopiperazino)-ethyl-amino)-4-thiazolyl]-

3-Cyan-5-[2-(N-methyl-N-2-(4-ethyl-2,6-dioxopiperazino)-ethyl-amino)-4-thiazolyl]-
3-Cyan-5-[2-(N-ethyl-N-2-(4-ethyl-2,6-dioxopiperazino)-ethyl-amino)-4-thiazolyl]-

3-Cyan-5-[2-(N-isopropyl-N-2-(4-ethyl-2,6-dioxopiperazino)-ethyl-amino)-4-thiazolyl]-
3-Cyan-5-[2-(N-benzyl-N-2-(4-ethyl-2,6-dioxopiperazino)-ethyl-amino)-4-thiazolyl]-
3-Cyan-5-[2-(N-p-methoxybenzyl-N-2-(4-ethyl-2,6-dioxopiperazino)-ethyl-amino)-4-thiazolyl]-
3-Cyan-5-[2-(N-phenyl-N-2-(4-ethyl-2,6-dioxopiperazino)-ethyl-amino)-4-thiazolyl]-
3-Cyan-5-[2-(N-p-methoxyphenyl-N-2-(4-ethyl-2,6-dioxopiperazino)-ethyl-amino)-4-thiazolyl]-

3-Cyan-5-[2-(N-methyl-N-2-(3,5-dioxopiperazino)-ethyl-amino)-4-thiazolyl]-
3-Cyan-5-[2-(N-ethyl-N-2-(3,5-dioxopiperazino)-ethyl-amino)-4-thiazolyl]-
3-Cyan-5-[2-(N-isopropyl-N-2-(3,5-dioxopiperazino)-ethyl-amino)-4-thiazolyl]-
3-Cyan-5-[2-(N-benzyl-N-2-(3,5-dioxopiperazino)-ethyl-amino)-4-thiazolyl]-
3-Cyan-5-[2-(N-p-methoxybenzyl-N-2-(3,5-dioxopiperazino)-ethyl-amino)-4-thiazolyl]-
3-Cyan-5-[2-(N-phenyl-N-2-(3,5-dioxopiperazino)-ethyl-amino)-4-thiazolyl]-
3-Cyan-5-[2-(N-p-methoxyphenyl-N-2-(3,5-dioxopiperazino)-ethyl-amino)-4-thiazolyl]-

3-Cyan-5-[2-(N-methyl-N-2-(4-methyl-3,5-dioxopiperazino)-ethyl-amino)-4-thiazolyl]-
3-Cyan-5-[2-(N-ethyl-N-2-(4-methyl-3,5-dioxopiperazino)-ethyl-amino)-4-thiazolyl]-
3-Cyan-5-[2-(N-isopropyl-N-2-(4-methyl-3,5-dioxopiperazino)-ethyl-amino)-4-thiazolyl]-
3-Cyan-5-[2-(N-benzyl-N-2-(4-methyl-3,5-dioxopiperazino)-ethyl-amino)-4-thiazolyl]-

3-Cyan-5-[2-(N-p-methoxybenzyl-N-2-(4-methyl-3,5-dioxopiperazino)-ethyl-amino)-4-thiazolyl]-
3-Cyan-5-[2-(N-phenyl-N-2-(4-methyl-3,5-dioxopiperazino)-ethyl-amino)-4-thiazolyl]-
3-Cyan-5-[2-(N-p-methoxyphenyl-N-2-(4-methyl-3,5-dioxopiperazino)-ethyl-amino)-4-thiazolyl]-

3-Cyan-5-[2-(N-methyl-N-2-(4-ethyl-3,5-dioxopiperazino)-ethyl-amino)-4-thiazolyl]-
3-Cyan-5-[2-(N-ethyl-N-2-(4-ethyl-3,5-dioxopiperazino)-ethyl-amino)-4-thiazolyl]-
3-Cyan-5-[2-(N-isopropyl-N-2-(4-ethyl-3,5-dioxopiperazino)-ethyl-amino)-4-thiazolyl]-
3-Cyan-5-[2-(N-benzyl-N-2-(4-ethyl-3,5-dioxopiperazino)-ethyl-amino)-4-thiazolyl]-
3-Cyan-5-[2-(N-p-methoxybenzyl-N-2-(4-ethyl-3,5-dioxopiperazino)-ethyl-amino)-4-thiazolyl]-
3-Cyan-5-[2-(N-phenyl-N-2-(4-ethyl-3,5-dioxopiperazino)-ethyl-amino)-4-thiazolyl]-
3-Cyan-5-[2-(N-p-methoxyphenyl-N-2-(4-ethyl-3,5-dioxopiperazino)-ethyl-amino)-4-thiazolyl]-

3-Cyan-5-[2-(N-methyl-N-2-(4-(2-dimethylaminoethyl)-3,5-dioxopiperazino)-ethyl-amino)-4-thiazolyl]-
3-Cyan-5-[2-(N-ethyl-N-2-(4-(2-dimethylaminoethyl)-3,5-dioxopiperazino)-ethyl-amino)-4-thiazolyl]-
3-Cyan-5-[2-(N-isopropyl-N-2-(4-(2-dimethylaminoethyl)-3,5-dioxopiperazino)-ethyl-amino)-4-thiazolyl]-
3-Cyan-5-[2-(N-benzyl-N-2-(4-(2-dimethylaminoethyl)-3,5-dioxopiperazino)-ethyl-amino)-4-thiazolyl]-
3-Cyan-5-[2-(N-p-methoxybenzyl-N-2-(4-(2-dimethylaminoethyl)-3,5-dioxopiperazino)-ethyl-amino)-4-thiazolyl]-
3-Cyan-5-[2-(N-phenyl-N-2-(4-(2-dimethylaminoethyl)-

3,5-dioxopiperazino)-ethyl-amino-]-4-thiazolyl]-
3-Cyan-5-[2-(N-p-methoxyphenyl-N-2-(4-(2-dimethylaminoethyl)-3,5-dioxopiperazino)-ethyl-amino-]-4-thiazolyl]-

3-Cyan-5-[2-(N-methyl-N-2-(2-oxo-1-imidazolidinyl)-ethyl-amino)-4-thiazolyl]-
3-Cyan-5-[2-(N-ethyl-N-2-(2-oxo-1-imidazolidinyl)-ethyl-amino)-4-thiazolyl]-
3-Cyan-5-[2-(N-isopropyl-N-2-(2-oxo-1-imidazolidinyl)-ethyl-amino)-4-thiazolyl]-
3-Cyan-5-[2-(N-benzyl-N-2-(2-oxo-1-imidazolidinyl)-ethyl-amino)-4-thiazolyl]-
3-Cyan-5-[2-(N-p-methoxybenzyl-N-2-(2-oxo-1-imidazolidinyl)-ethyl-amino)-4-thiazolyl]-
3-Cyan-5-[2-(N-phenyl-N-2-(2-oxo-1-imidazolidinyl)-ethyl-amino)-4-thiazolyl]-
3-Cyan-5-[2-(N-p-methoxyphenyl-N-2-(2-oxo-1-imidazolidinyl)-ethyl-amino)-4-thiazolyl]-

3-Cyan-5-[2-(N-methyl-N-2-(2,5-dioxo-1-imidazolidinyl)-ethyl-amino)-4-thiazolyl]-
3-Cyan-5-[2-(N-ethyl-N-2-(2,5-dioxo-1-imidazolidinyl)-ethyl-amino)-4-thiazolyl]-
3-Cyan-5-[2-(N-isopropyl-N-2-(2,5-dioxo-1-imidazolidinyl)-ethyl-amino)-4-thiazolyl]-
3-Cyan-5-[2-(N-benzyl-N-2-(2,5-dioxo-1-imidazolidinyl)-ethyl-amino)-4-thiazolyl]-
3-Cyan-5-[2-(N-p-methoxybenzyl-N-2-(2,5-dioxo-1-imidazolidinyl)-ethyl-amino)-4-thiazolyl]-
3-Cyan-5-[2-(N-phenyl-N-2-(2,5-dioxo-1-imidazolidinyl)-ethyl-amino)-4-thiazolyl]-
3-Cyan-5-[2-(N-p-methoxyphenyl-N-2-(2,5-dioxo-1-imidazolidinyl)-ethyl-amino)-4-thiazolyl]-

3-Cyan-5-[2-(N-methyl-N-2-(3-methyl-2,5-dioxo-1-imidazolidinyl)-ethyl-amino)-4-thiazolyl]-
3-Cyan-5-[2-(N-ethyl-N-2-(3-methyl-2,5-dioxo-1-imidazolidinyl)-ethyl-amino)-4-thiazolyl]-
3-Cyan-5-[2-(N-isopropyl-N-2-(3-methyl-2,5-dioxo-1-imidazolidinyl)-ethyl-amino)-4-thiazolyl]-
3-Cyan-5-[2-(N-benzyl-N-2-(3-methyl-2,5-dioxo-1-imidazolidinyl)-ethyl-amino)-4-thiazolyl]-
3-Cyan-5-[2-(N-p-methoxybenzyl-N-2-(3-methyl-2,5-dioxo-1-imidazolidinyl)-ethyl-amino)-4-thiazolyl]-
3-Cyan-5-[2-(N-phenyl-N-2-(3-methyl-2,5-dioxo-1-imidazolidinyl)-ethyl-amino)-4-thiazolyl]-
3-Cyan-5-[2-(N-p-methoxyphenyl-N-2-(3-methyl-2,5-dioxo-1-imidazolidinyl)-ethyl-amino)-4-thiazolyl]-

3-Cyan-5-[2-(N-methyl-N-2-(3-ethyl-2,5-dioxo-1-imidazolidinyl)-ethyl-amino)-4-thiazolyl]-
3-Cyan-5-[2-(N-ethyl-N-2-(3-ethyl-2,5-dioxo-1-imidazolidinyl)-ethyl-amino)-4-thiazolyl]-
3-Cyan-5-[2-(N-isopropyl-N-2-(3-ethyl-2,5-dioxo-1-imidazolidinyl)-ethyl-amino)-4-thiazolyl]-
3-Cyan-5-[2-(N-benzyl-N-2-(3-ethyl-2,5-dioxo-1-imidazolidinyl)-ethyl-amino)-4-thiazolyl]-
3-Cyan-5-[2-(N-p-methoxybenzyl-N-2-(3-ethyl-2,5-dioxo-1-imidazolidinyl)-ethyl-amino)-4-thiazolyl]-
3-Cyan-5-[2-(N-phenyl-N-2-(3-ethyl-2,5-dioxo-1-imidazolidinyl)-ethyl-amino)-4-thiazolyl]-
3-Cyan-5-[2-(N-p-methoxyphenyl-N-2-(3-ethyl-2,5-dioxo-1-imidazolidinyl)-ethyl-amino)-4-thiazolyl]-

3-Cyan-5-[2-(N-methyl-N-2-(3-ethyl-2,5-dioxo-1-imidazolidinyl)-ethyl-amino)-4-thiazolyl]-
3-Cyan-5-[2-(N-ethyl-N-2-(3-ethyl-2,5-dioxo-1-imidazolidinyl)-ethyl-amino)-4-thiazolyl]-

3-Cyan-5-[2-(N-isopropyl-N-2-(3-ethyl-2,5-dioxo-1-imidazolidinyl)-ethyl-amino)-4-thiazolyl]-
3-Cyan-5-[2-(N-benzyl-N-2-(3-ethyl-2,5-dioxo-1-imidazolidinyl)-ethyl-amino)-4-thiazolyl]-
3-Cyan-5-[2-(N-p-methoxybenzyl-N-2-(3-ethyl-2,5-dioxo-1-imidazolidinyl)-ethyl-amino)-4-thiazolyl]-
3-Cyan-5-[2-(N-phenyl-N-2-(3-ethyl-2,5-dioxo-1-imidazolidinyl)-ethyl-amino)-4-thiazolyl]-
3-Cyan-5-[2-(N-p-methoxyphenyl-N-2-(3-ethyl-2,5-dioxo-1-imidazolidinyl)-ethyl-amino)-4-thiazolyl]-

3-Cyan-5-[2-N-methyl-N-2-methoxyethyl-amino-4-thiazolyl]-
3-Cyan-5-[2-N-ethyl-N-2-methoxyethyl-amino-4-thiazolyl]-
3-Cyan-5-[2-N-isopropyl-N-2-methoxyethyl-amino-4-thiazolyl]-
3-Cyan-5-[2-N-benzyl-N-2-methoxyethyl-amino-4-thiazolyl]-
3-Cyan-5-[2-N-p-methoxybenzyl-N-2-methoxyethyl-amino-4-thiazolyl]-
3-Cyan-5-[2-N-(3,4-dimethoxybenzyl)-N-2-methoxyethyl-amino-4-thiazolyl]-
3-Cyan-5-[2-N-2-p-methoxyphenylethyl-N-2-methoxyethyl-amino-4-thiazolyl]-
3-Cyan-5-[2-N-2-(3,4-dimethoxyphenyl)-ethyl-N-2-methoxyethyl-amino-4-thiazolyl]-
3-Cyan-5-[2-N-phenyl-N-2-methoxyethyl-amino-4-thiazolyl]-
3-Cyan-5-[2-N-p-methoxyphenyl-N-2-methoxyethyl-4-thiazolyl]-
3-Cyan-5-(2-pyrrolidino-4-thiazolyl)-, F. > 310°
3-Cyan-5-(2-piperidino-4-thiazolyl)-
3-Cyan-5-(2-morpholino-4-thiazolyl)-, F. 302-305°
3-Cyan-5-(2-piperazino-4-thiazolyl)-, F. 243-246°

3-Cyan-5-[2-(4-methylpiperazino)-4-thiazolyl]-, F. 266-268°
3-Cyan-5-[2-(4-(2-hydroxyethyl)-piperazino)-4-thiazolyl]-, F. 255-257°
3-Cyan-5-[2-(4-(3-hydroxypropyl)-piperazino)-4-thiazolyl]-, F. 206-208°
3-Cyan-5-[2-(4-(2,3-dihydroxypropyl)-piperazino)-4-thiazolyl]-, Dihydrochlorid, F. 260-263°
3-Cyan-5-[2-(4-(N-isopropylcarbamoylmethyl)-piperazino)-4-thiazolyl]-, Dihydrochlorid, F. 261-264°
3-Cyan-5-[2-(4-o-methoxyphenyl-piperazino)-4-thiazolyl]-, F. 244-247°
3-Cyan-5-[2-(4-m-methoxyphenyl-piperazino)-4-thiazolyl]-
3-Cyan-5-[2-(4-p-methoxyphenyl-piperazino)-4-thiazolyl], F. > 300°
3-Cyan-5-[2-(4-(2-thienyl)-piperazino)-4-thiazolyl]-
3-Cyan-5-[2-(4-benzyl-piperazino)-4-thiazolyl]-, F. 240-243°
3-Cyan-5-[2-(4-methoxycarbonyl-piperazino)-4-thiazolyl]-
3-Cyan-5-[2-(4-benzoyl-piperazino)-4-thiazolyl]-
3-Cyan-5-[2-(4-p-methoxybenzoyl-piperazino)-4-thiazolyl]-
3-Cyan-5-[2-(4-(2-furoyl)-piperazino)-4-thiazolyl]-, F. 294-299° (Zers.)
3-Cyan-5-[2-(4-(2-thenoyl)-piperazino)-4-thiazolyl]-
3-Cyan-5-[2-(4-carbamoyl-piperazino)-4-thiazolyl]-
3-Cyan-5-[2-(4-N-methylcarbamoyl-piperazino)-4-thiazolyl]-
3-Cyan-5-[2-(4-N-phenylcarbamoyl-piperazino)-4-thiazolyl]-
3-Cyan-5-[2-(4-N-p-methoxyphenylcarbamoyl-piperazino)-4-thiazolyl]-
3-Cyan-5-[2-(4-acetoxymethyl-piperazino)-4-thiazolyl]-
3-Cyan-5-[2-(4-oxopiperidino)-4-thiazolyl]-
3-Cyan-5-[2-(4-methylpiperidino)-4-thiazolyl]-, F. 270-272°

3-Cyan-5-[2-(4-benzylpiperidino)-4-thiazolyl]-
3-Cyan-5-[2-(2-carboxypyrrolidino)-4-thiazolyl]-
3-Cyan-5-[2-(2-ethoxycarbonylpyrrolidino)-4-thiazolyl]-
3-Cyan-5-[2-(2-benzyloxycarbonylpyrrolidino)-4-thiazolyl]-.

Analog erhält man aus 3-Carbamoyl-5-bromacetyl-6-methyl-2-pyridon die folgenden 6-Methyl-2-pyridone:

3-Carbamoyl-5-(2-amino-4-thiazolyl)-, F. > 300°
3-Carbamoyl-5-(2-methylamino-4-thiazolyl)-
3-Carbamoyl-5-(2-ethylamino-4-thiazolyl)-
3-Carbamoyl-5-(2-isopropylamino-4-thiazolyl)-
3-Carbamoyl-5-(2-hexylamino-4-thiazolyl)-
3-Carbamoyl-5-(2-allylamino-4-thiazolyl)-
3-Carbamoyl-5-[2-(N-2-hydroxyethylamino)-4-thiazolyl]-
3-Carbamoyl-5-[2-(N-2-dimethylaminoethyl-amino)-4-thiazolyl]-
3-Carbamoyl-5-[2-(N-3-dimethylaminopropyl-amino)-4-thiazolyl]-
3-Carbamoyl-5-[2-(N-2-diethylaminopropyl-amino)-4-thiazolyl]-
3-Carbamoyl-5-[2-(N-2-pyrrolidinoethyl-amino)-4-thiazolyl]-
3-Carbamoyl-5-[2-(N-2-piperidinoethyl-amino)-4-thiazolyl]-
3-Carbamoyl-5-[2-(N-3-(2-methylpiperidino)-propyl-amino)-4-thiazolyl]-
3-Carbamoyl-5-[2-(N-3-(2-oxopyrrolidino)-propyl-amino)-4-thiazolyl]-
3-Carbamoyl-5-[2-(N-2-morpholinoethyl-amino)-4-thiazolyl]-
3-Carbamoyl-5-[2-(N-3-morpholinopropyl-amino)-4-thiazolyl]-

3-Carbamoyl-5-[2-(N-2-piperazinoethyl-amino)-4-thiazolyl]-

3-Carbamoyl-5-[2-(N-2-methoxyethyl-amino)-4-thiazolyl]-

3-Carbamoyl-5-(2-benzylamino-4-thiazolyl)-

3-Carbamoyl-5-(2-p-methoxybenzylamino-4-thiazolyl)-

3-Carbamoyl-5-[2-(2-p-methoxyphenylethyl-amino)-4-thiazolyl]-

3-Carbamoyl-5-[2-(2-(3,4-dimethoxyphenyl)-ethyl-amino)-4-thiazolyl]-

3-Carbamoyl-5-[2-(N-1-indanylamino)-4-thiazolyl]-

3-Carbamoyl-5-[2-(N-2-(2-pyridyl)-ethylamino)-4-thiazolyl]-

3-Carbamoyl-5-[2-(N-2-(3-pyridyl)-ethylamino)-4-thiazolyl]-

3-Carbamoyl-5-[2-(N-2-(4-pyridyl)-ethylamino)-4-thiazolyl]-

3-Carbamoyl-5-[2-(N-(1-benzyl-4-piperidyl)-amino)-4-thiazolyl]-

3-Carbamoyl-5-[2-(N-(1-ethoxycarbonyl-4-piperidyl)-amino)-4-thiazolyl]-

3-Carbamoyl-5-[2-(N-2-(4-methyl-5-imidazolylmethyl-thio)-ethylamino)-4-thiazolyl]-

3-Carbamoyl-5-(2-dimethylamino-4-thiazolyl)-3-Carbamoyl-5-(2-diisopropylamino)-4-thiazolyl)-

3-Carbamoyl-5-[2-(bis-2-hydroxyethyl-amino)-4-thiazolyl]-

3-Carbamoyl-5-[2-(N-ethyl-N-2-dimethylaminoethyl-amino)-4-thiazolyl]-

3-Carbamoyl-5-[2-(N-isopropyl-N-2-dimethylaminoethyl-amino)-4-thiazolyl]-

3-Carbamoyl-5-[2-(N-benzyl-N-2-dimethylaminoethyl-amino)-4-thiazolyl]-

3-Carbamoyl-5-[2-(N-p-methoxyphenyl-N-2-dimethylamino-ethyl-amino)-4-thiazolyl]-

3-Carbamoyl-5-[2-(N-ethyl-N-2-diethylaminoethyl-amino)-4-thiazolyl]-
3-Carbamoyl-5-(2-pyrrolidino-4-thiazolyl)-
3-Carbamoyl-5-(2-morpholino-4-thiazolyl)-
3-Carbamoyl-5-(2-piperazino-4-thiazolyl)-
3-Carbamoyl-5-[2-(4-(2-hydroxyethyl)-piperazino)-4-thiazolyl]-
3-Carbamoyl-5-[2-(4-(3-hydroxypropyl)-piperazino)-4-thiazolyl]-
3-Carbamoyl-5-[2-(4-(2,3-dihydroxypropyl)-piperazino)-4-thiazolyl]-
3-Carbamoyl-5-[2-(4-(N-isopropylcarbamoylmethyl)-piperazino)-4-thiazolyl]-
3-Carbamoyl-5-[2-(4-o-methoxyphenyl-piperazino)-4-thiazolyl]-
3-Carbamoyl-5-[2-(4-p-methoxyphenyl-piperazino)-4-thiazolyl]-
3-Carbamoyl-5-[2-(4-(2-furoyl)-piperazino)-4-thiazolyl]-.

Beispiel 22

Analog Beispiel 21 erhält man aus 3-Cyan-5-(2-brompropionyl)-6-methyl-2-pyridon und N-(2-Dimethylaminoethyl)-thioharnstoff das 3-Cyan-5-[2-(2-Dimethylaminoethyl-amino)-5-methyl-4-thiazolyl]-6-methyl-2-pyridon, F. 219° (Zers.).

Analog erhält man mit den entsprechenden Thioharnstoffen die nachstehenden 6-Methyl-2-pyridone:

3-Cyan-5-(2-amino-5-methyl-4-thiazolyl)-
3-Cyan-5-(2-methylamino-5-methyl-4-thiazolyl)-
3-Cyan-5-(2-ethylamino-5-methyl-4-thiazolyl)-
3-Cyan-5-(2-p-methoxybenzylamino-5-methyl-4-thiazolyl)-, F. 197-198°

3-Cyan-5-(2-p-methoxyanilino-5-methyl-4-thiazolyl)-, F. 256-257°
3-Carbamoyl-5-[2-(2-Dimethylaminoethyl-amino)-5-methyl-4-thiazolyl]-
3-Carbamoyl-5-[2-p-methoxybenzylamino-5-methyl-4-thiazolyl]-
3-Carbamoyl-5-[2-p-methoxyanilino-5-methyl-4-thiazolyl]-.

Beispiel 23

Analog Beispiel 21 erhält man aus 3-Cyan-5-brom-acetyl-6-ethyl-2-pyridon und Thioharnstoff das 3-Cyan-5-(2-amino-4-thiazolyl)-6-ethyl-2-pyridon, F. > 300°.

Analog erhält man mit den entsprechenden Thioharnstoffen:

3-Cyan-5-(2-methylamino-4-thiazolyl)-6-ethyl-2-pyridon
3-Cyan-5-(2-ethylamino-4-thiazolyl)-6-ethyl-2-pyridon
3-Cyan-5-(2-propylamino-4-thiazolyl)-6-ethyl-2-pyridon
3-Cyan-5-(2-isopropylamino-4-thiazolyl)-6-ethyl-2-pyridon
3-Cyan-5-[2-(2-dimethylaminoethylamino)-4-thiazolyl]-6-ethyl-2-pyridon, F. 261-262°
3-Cyan-5-(2-benzylamino-4-thiazolyl)-6-ethyl-2-pyridon
3-Cyan-5-(2-p-methoxybenzylamino-4-thiazolyl)-6-ethyl-2-pyridon, F. 230-231°
3-Cyan-5-(2-anilino-4-thiazolyl)-6-ethyl-2-pyridon
3-Cyan-5-(2-p-methoxyanilino-4-thiazolyl)-6-ethyl-2-pyridon, F. 249°
3-Cyan-5-(2-p-hydroxyanilino-4-thiazolyl)-6-ethyl-2-pyridon

3-Cyan-5-(2-dimethylamino-4-thiazolyl)-6-ethyl-2-pyridon
3-Carbamoyl-5-(2-amino-4-thiazolyl)-6-ethyl-2-pyridon
3-Carbamoyl-5-[2-(2-dimethylaminoethyl-amino)-4-thiazolyl]-6-ethyl-2-pyridon
3-Carbamoyl-5-(2-p-methoxybenzylamino-4-thiazolyl)-6-ethyl-2-pyridon
3-Carbamoyl-5-(2-p-methoxyanilino-4-thiazolyl)-6-ethyl-2-pyridon.

Beispiel 24

Analog Beispiel 21 erhält man aus 3-Cyan-6-brom-1,2,5,6,7,8-hexahydro-chinolin-2,5-dion und Thioharnstoff das 2-Amino-8-cyan-4,5,6,7-tetrahydro-thiazolo[4,5-f]chinolin-7-on, F. > 300 °

Analog erhält man mit den entsprechenden Thioharnstoffen die folgenden 8-Cyan-4,5,6,7-tetrahydro-thiazolo[4,5-f]chinolin-7-one:

2-Methylamino-, F. > 300 °
2-Ethylamino-, F > 300 °
2-Propylamino-
2-Isopropylamino-, F. > 300 °
2-Butylamino-
2-Isobutylamino-
2-sek.-Butylamino-
2-tert.-Butylamino-
2-Pentylamino-
2-Hexylamino-, F. 221 - 222 °
2-Allylamino-, Hydrobromid, F. 292 - 294 °
2-(2-Hydroxyethylamino)-, F. 260 - 261 °
2-(3-Hydroxypropylamino)-

2-(2,3-Dihydroxypropylamino)-
2-(2-Dimethylaminoethylamino)-, F. 233 - 235 °
2-(3-Dimethylaminopropylamino)-, F. 244 - 247 °
2-(2-Diethylaminoethylamino)-
2-(2-Pyrrolidinoethylamino)-
2-(2-Piperidinoethylamino)-, Zers. 143°
2-[2-(2-Methylpiperidino)-ethylamino]-
2-[2-(2-Oxopyrrolidino)-ethylamino]-
2-(2-Morpholinoethylamino)-
2-(3-Morpholinopropylamino)-, F. 220 - 222 °
2-(2-Methoxyethylamino)-
2-Benzylamino-, F. 262 - 267 °
2-p-Methoxybenzylamino-, Hydrobromid, F. > 300 °
2-(2-Phenylethylamino)-
2-(2-p-Methoxyphenylethylamino)-
2-[2-(3,4-Dimethoxyphenyl)-ethylamino]-
2-(1-Indanylamino)-
2-[2-(2-Pyridyl)-ethylamino]-, F. 252 - 256 °
2-[2-(3-Pyridyl)-ethylamino]-
2-[2-(4-Pyridyl)-ethylamino]-, F. 237 - 241 °
2-(4-Piperidylamino)-
2-(1-Benzyl-4-piperidylamino)-, F. 281 - 282 °
2-(1-Ethoxycarbonyl-4-piperidylamino)-, F. 298 °
2-Dimethylamino-, Hydrobromid, F. > 300 °
2-Diethylamino-
2-Dipropylamino-
2-Diisopropylamino-, F. > 300 °
2-Bis-(2-hydroxyethyl)-amino-, F. 285 ° (Zers.)
2-(N-Methyl-N-2-dimethylaminoethyl-amino)-
2-(N-Ethyl-N-2-dimethylaminoethyl-amino)-, F. 233 - 236 °
2-(N-Isopropyl-N-2-dimethylaminoethyl-amino)-, F. 245 - 248 °
2-(N-Benzyl-N-2-dimethylaminoethyl-amino)-, F. 252-256 °
2-(N-p-Methoxybenzyl-N-2-dimethylaminoethyl-amino)-, F. 240-245°

2-[N-(3,4-Dimethoxybenzyl)-N-2-dimethylaminoethyl-amino]-
2-(N-2-p-Methoxyphenylethyl-N-2-dimethylaminoethyl-amino)-, F. 278-280°
2-[N-2-(3,4-Dimethoxyphenyl)-ethyl-N-2-dimethylaminoethyl-amino]-
2-(N-Phenyl-N-2-dimethylaminoethyl-amino)-
2-(N-p-Methoxyphenyl-N-2-dimethylaminoethyl-amino)-, F. 242 - 245 °
2-(N-Methyl-N-2-diethylaminoethyl-amino)-
2-(N-Ethyl-N-2-diethylaminoethyl-amino)-, F. 267 - 268 °
2-(N-Isopropyl-N-2-diethylaminoethyl-amino)-
2-(N-Benzyl-N-2-diethylaminoethyl-amino)-
2-(N-p-Methoxybenzyl-N-2-diethylaminoethyl-amino)-, F. 168° (Zers.)
2-[N-(3,4-Dimethoxybenzyl)-N-2-diethylaminoethyl-amino]-
2-(N-2-p-Methoxyphenylethyl-N-2-diethylaminoethyl-amino)-, F. 282-284°
2-[N-2-(3,4-Dimethoxyphenyl)-ethyl-N-2-diethylaminoethyl-amino]-, F. 245 (Zers.)
2-(N-Phenyl-N-2-diethylaminoethyl-amino)-
2-(N-p-Methoxyphenyl-N-2-diethylaminoethyl-amino)-
2-(N-Methyl-N-2-methoxyethyl-amino)-
2-(N-Ethyl-N-2-methoxyethyl-amino)-
2-(N-Isopropyl-N-2-methoxyethyl-amino)-
2-(N-Benzyl-N-2-methoxyethyl-amino)-
2-(N-p-Methoxybenzyl-N-2-methoxyethyl-amino)-
2-[N-(3,4-Dimethoxybenzyl)-N-2-methoxyethyl-amino]-
2-(N-2-p-Methoxyphenylethyl-N-2-methoxyethyl-amino)-
2-[N-2-(3,4-Dimethoxyphenyl)-ethyl-N-2-methoxyethyl-amino]-
2-(N-Phenyl-N-2-methoxyethyl-amino)-
2-(N-p-Methoxyphenyl-N-2-methoxyethyl-amino)-
2-Pyrrolidino-, F. > 300 °
2-Piperidino-
2-Morpholino-, F. > 300 °
2-(4-Methylpiperidino)-, F. 288 - 291 °
2-(4-Methylpiperazino)-

2-[4-(2-Hydroxyethyl)-piperazino]-, F. 268 - 271 °
2-[4-(3-Hydroxypropyl)-piperazino]-, F. 272 - 275 °
2-[4-(2,3-Dihydroxypropyl)-piperazino]-, F. 259 - 261 °
2-[4-(N-Isopropylcarbamoylmethyl)-piperazino]-, F. > 300 °
2-(4-o-Methoxyphenyl-piperazino)-, F. 299 - 300 °
2-(4-m-Methoxyphenyl-piperazino)-
2-(4-p-Methoxyphenyl-piperazino)-, F. > 300 °
2-(4-Furoyl-piperazino)-
2-Anilino-
2-p-Methylanilino-
2-o-Fluoranilino-
2-m-Fluoranilino-
2-p-Fluoranilino-
2-o-Chloranilino-
2-m-Chloranilino-, F. > 300 °
2-p-Chloranilino-
2-o-Bromanilino-
2-m-Bromanilino-
2-p-Bromanilino-
2-p-Jodanilino-
2-p-Trifluormethylanilino-
2-o-Hydroxyanilino-
2-m-Hydroxyanilino-
2-p-Hydroxyanilino-
2-o-Methoxyanilino-
2-m-Methoxyanilino-
2-p-Methoxyanilino-, F. > 300 °
2-o-Nitroanilino-
2-m-Nitroanilino-
2-p-Nitroanilino-
2-o-Methylthioanilino-
2-m-Methylthioanilino-
2-p-Methylthioanilino-
2-o-Methylsulfinylanilino-
2-m-Methylsulfinylanilino-

2-p-Methylsulfinylanilino-
2-(2,4-Dimethoxyanilino)-, F. > 300 °
2-(3,4-Dimethoxyanilino)-
2-(2-Pyridylamino)-
2-(3-Pyridylamino)-
2-(4-Pyridylamino)-
2-(2-Methoxy-5-pyridylamino)-, F. > 300 °
2-[5-(4-Pyridyl)-2-pyridylamino]-, F. > 300 °

sowie aus 3-Carbamoyl-6-brom-1,2,5,6,7,8-hexahydro-chinolin-2,5-dion die folgenden 8-Carbamoyl-4,5,6,7-tetrahydro-thiazolo[4,5-f]chinolin-7-one:

2-Amino-
2-Methylamino-
2-Ethylamino-
2-Isopropylamino-
2-Hexylamino-
2-Allylamino-
2-(2-Hydroxyethylamino)-
2-(2-Dimethylaminoethylamino)-
2-(3-Dimethylaminopropylamino)-
2-(2-Piperidinoethylamino)-
2-(3-Morpholinopropylamino)-
2-Benzylamino-
2-p-Methoxybenzylamino-
2-[2-(2-Pyridyl)-ethylamino]-

2-[2-(4-Pyridyl)-ethylamino]-
2-(1-Benzyl-4-piperidylamino)-
2-(1-Ethoxycarbonyl-4-piperidylamino)-
2-Dimethylamino-
2-Diisopropylamino-
2-Bis-(2-hydroxyethyl)-amino-

2-Pyrrolidino-
2-Morpholino-
2-(4-Methylpiperidino)-
2-[4-(2-Hydroxyethyl)-piperazino]-
2-[4-(3-Hydroxypropyl)-piperazino]-
2-[4-(2,3-Dihydroxypropyl)-piperazino]-
2-[4-(N-Isopropylcarbamoylmethyl)-piperazino]-
2-(4-o-Methoxyphenyl-piperazino)-
2-(4-p-Methoxyphenyl-piperazino)-
2-m-Chloranilino-
2-p-Methoxyanilino-
2-(2,4-Dimethoxyanilino)-
2-(2-Methoxy-5-pyridylamino)-
2-[5-(4-pyridyl)-2-pyridylamino]-.

Beispiel 25

Eine Suspension von 2,55 g 3-Cyan-5-bromacetyl-6-methyl-2-pyridon und 1,82 g p-Methoxyphenylharnstoff in 50 ml Ethanol wird 45 Min. gekocht. Das in der Kälte ausgefallene Salz wird in wenig Wasser suspendiert und mit gesättigter $NaHCO_3$-Lösung behandelt. Man erhält 3-Cyan-5-(2-p-methoxyanilino-4-thiazolyl)-6-methyl-2-pyridon, F. 289 - 293 °.

Analog erhält man mit den entsprechenden Thioharnstoffen die nachstehenden 6-Methyl-2-pyridone:

3-Cyan-5-(2-anilino-4-thiazolyl)-, F. 315 - 317 °
3-Cyan-5-(2-p-methylanilino-4-thiazolyl)-
3-Cyan-5-(2-o-fluoranilino-4-thiazolyl)-
3-Cyan-5-(2-m-fluoranilino-4-thiazolyl)-
3-Cyan-5-(2-p-fluoranilino-4-thiazolyl)-
3-Cyan-5-(2-o-chloranilino-4-thiazolyl)-

3-Cyan-5-(2-m-chloranilino-4-thiazolyl)-, F. 278 - 280 °
3-Cyan-5-(2-p-chloranilino-4-thiazolyl)-
3-Cyan-5-(2-o-bromanilino-4-thiazolyl)-
3-Cyan-5-(2-m-bromanilino-4-thiazolyl)-
3-Cyan-5-(2-p-bromanilino-4-thiazolyl)-
3-Cyan-5-(2-p-jodanilino-4-thiazolyl)-
3-Cyan-5-(2-p-trifluormethylanilino-4-thiazolyl)-
3-Cyan-5-(2-o-hydroxyanilino-4-thiazolyl)-
3-Cyan-5-(2-m-hydroxyanilino-4-thiazolyl)-
3-Cyan-5-(2-p-hydroxyanilino-4-thiazolyl)-, F. 297 - 299 °
3-Cyan-5-(2-o-methoxyanilino-4-thiazolyl)-
3-Cyan-5-(2-m-methoxyanilino-4-thiazolyl)-
3-Cyan-5-(2-o-nitroanilino-4-thiazolyl)-
3-Cyan-5-(2-m-nitroanilino-4-thiazolyl)-
3-Cyan-5-(2-p-nitroanilino-4-thiazolyl)-
3-Cyan-5-(2-o-methylthioanilino-4-thiazolyl)-
3-Cyan-5-(2-m-methylthioanilino-4-thiazolyl)-
3-Cyan-5-(2-p-methylthioanilino-4-thiazolyl)-, F. 221 - 225 °
3-Cyan-5-(2-o-methylsulfinylanilino-4-thiazolyl)-
3-Cyan-5-(2-m-methylsulfinylanilino-4-thiazolyl)-
3-Cyan-5-(2-p-methylsulfinylanilino-4-thiazolyl)-, F. 291 - 293 °
3-Cyan-5-(2-p-methylsulfonylanilino-4-thiazolyl)-
3-Cyan-5-[2-(2,4-dimethoxyanilino)-4-thiazolyl]-, F. 275 °
3-Cyan-5-[2-(3,4-dimethoxyanilino)-4-thiazolyl]-, F. 280 - 282 °
3-Cyan-5-[2-(2-pyridylamino)-4-thiazolyl]-, F. > 300 °
3-Cyan-5-[2-(3-pyridylamino)-4-thiazolyl]-, F. 226 - 230 ° (Zers.)
3-Cyan-5-[2-(4-pyridylamino)-4-thiazolyl],- F. > 300 °
3-Cyan-5-[2-(2-methoxy-5-pyridylamino)-4-thiazolyl]-, F. 245 °
3-Cyan-5-[2-(5-(4-pyridyl)-2-pyridylamino)-4-thiazolyl]-, Hydrobromid, F. 360 °

sowie

3-Carbamoyl-5-(2-anilino-4-thiazolyl)-
3-Carbamoyl-5-(2-m-chloranilino-4-thiazolyl)-
3-Carbamoyl-5-(2-p-hydroxyanilino-4-thiazolyl)-
3-Carbamoyl-5-(2-p-methoxyanilino-4-thiazolyl)-
3-Carbamoyl-5-(2-p-methylthioanilino-4-thiazolyl)-
3-Carbamoyl-5-(2-p-methylsulfinylanilino-4-thiazolyl)-
3-Carbamoyl-5-[2-(2,4-dimethoxyanilino)-4-thiazolyl]-
3-Carbamoyl-5-[2-(3,4-dimethoxyanilino)-4-thiazolyl]-
3-Carbamoyl-5-[2-(2-pyridylamino)-4-thiazolyl]-
3-Carbamoyl-5-[2-(3-pyridylamino)-4-thiazolyl]-
3-Carbamoyl-5-[2-(4-pyridylamino)-4-thiazolyl]-
3-Carbamoyl-5-[2-(2-methoxy-5-pyridylamino)-4-thiazolyl]-
3-Carbamoyl-5-[2-(5-(4-pyridyl)-2-pyridylamino)-4-thiazolyl]-.

Beispiel 26

Ein Gemisch von 2,85 g 3-Cyan-4-methoxy-5-bromacetyl-6-methyl-2-pyridon, 1,82 g 4-Methoxyphenylthioharnstoff und 100 ml Propanol wird 1 Std. gekocht. Beim Abkühlen fällt 3-Cyan-4-methoxy-5-(2-p-methoxyanilino-4-thiazolyl)-6-methyl-2-pyridon aus; F. > 300 °.

Analog erhält man mit den entsprechenden Thioharnstoffen die folgenden 6-Methyl-2-pyridone:

3-Cyan-4-methoxy-5-(2-amino-4-thiazolyl)-, F. 265 °
3-Cyan-4-methoxy-5-(2-methylamino-4-thiazolyl)-
3-Cyan-4-methoxy-5-(2-ethylamino-4-thiazolyl)-
3-Cyan-4-methoxy-5-(2-propylamino-4-thiazolyl)-
3-Cyan-4-methoxy-5-(2-isopropylamino-4-thiazolyl)-
3-Cyan-4-methoxy-5-[2-(2-dimethylaminoethylamino)-4-thiazolyl]-

3-Cyan-4-methoxy-5-(2-benzylamino-4-thiazolyl)-
3-Cyan-4-methoxy-5-(2-p-methoxybenzylamino-4-thiazolyl)-
3-Cyan-4-methoxy-5-(2-anilino-4-thiazolyl)-
3-Cyan-4-methoxy-5-(2-p-methoxyanilino-4-thiazolyl)-
3-Cyan-4-methoxy-5-(2-p-hydroxyanilino-4-thiazolyl)-
3-Cyan-4-methoxy-5-(2-dimethylamino-4-thiazolyl)-
3-Carbamoyl-4-methoxy-5-(2-amino-4-thiazolyl)-
3-Carbamoyl-4-methoxy-5-(2-p-methoxyanilino-4-thiazolyl)-.

Beispiel 27

Man erhitzt 2,67 g 3-Cyan-6-brom-1,2,5,6,7,8-hexahydrochinolin-2,5-dion mit 1,04 g Ethylthioharnstoff in 25 ml DMF 4 Std. auf 110 - 120 °, dampft ein und fällt mit Methanol das 2-Ethylamino-8-cyan-6,7-dihydro-thiazolo-[4,5-f]chinolin-7-on, F. > 300 °.

Analog sind die 4,5-Dehydroderivate der übrigen in Beispiel 24 genannten Verbindungen erhältlich.

Beispiel 28

Eine Suspension von 2,55 g 3-Cyan-5-bromacetyl-6-methyl-2-pyridon und 1,69 g 4-Thioureido-pyridin-N-oxid [F. 199 ° (Zers.); erhältlich durch Reaktion von 4-Nitropyridin-N-oxid mit Na-cyanamid zu 4-Cyanamino-pyridin-N-oxid und Umsetzung mit $H_2S$] in 100 ml Ethanol wird 20 Min. gekocht. Nach dem Abkühlen fällt 3-Cyan-5-[2-(4-pyridylamino)-4-thiazolyl]-6-methyl-2-pyridon-N-oxid-hydrobromid aus; F. 310 ° (Zers.).

Analog erhält man die folgenden 2-Pyridon-N-oxid-hydrobromide:

3-Cyan-5-[2-(4-pyridylamino)-4-thiazolyl]-6-ethyl-
3-Carbamoyl-5-[2-(4-pyridylamino)-4-thiazolyl]-6-methyl-.

Beispiel 29

Man kocht 2,55 g 3-Cyan-4-bromacetyl-6-methyl-2-pyridon und 1,02 g N,N'-Ethylenthioharnstoff (2-Thioxo-imidazolidin) 3 Std. in 50 ml Ethanol, kühlt ab und filtriert das erhaltene 3-Cyan-5-[(2-imidazolinylthio)-acetyl]-6-methyl-2-pyridon-hydrobromid ab. F. 209 - 212 °.

Analog erhält man mit 2-Mercaptobenzimidazol:

3-Cyan-6-[(2-benzimidazolylthio)-acetyl]-6-methyl-2-pyridon, Hydrobromid, F. 240 - 245 °.

Analog erhält man die folgenden 6-Methyl-2-pyridone:

3-Carbamoyl-5-[(2-imidazolinylthio)-acetyl]-
3-Carbamoyl-5-[(2-benzimidazolylthio)-acetyl]-.

Beispiel 30

Ein Gemisch von 2,55 g 3-Cyan-5-bromacetyl-6-methyl-2-pyridon, 1,65 g Guanidiniumsulfat, 0,5 g NaH (80 %ig) und 55 ml DMF wird 24 Std. bei 20 ° gerührt. Man filtriert ab, wäscht mit Ethanol, dann mit Ether und kocht den Rückstand mit Wasser aus. Nach Filtration wird der wäßrige Extrakt eingedampft, der Rückstand mit Wasser ausgeschlämmt und filtriert. Man erhält 3-Cyan-5-(2-amino-5-imidazolyl)-6-methyl-2-pyridon, F. > 300 °.

Analog erhält man mit Methylguanidiniumsulfat:

3-Cyan-5-(2-methylamino-5-imidazolyl)-6-methyl-2-pyridon.

Analog erhält man die folgenden 6-Methyl-2-pyridone:

3-Carbamoyl-5-(2-amino-5-imidazolyl)-
3-Carbamoyl-5-(2-methylamino-5-imidazolyl)-.

Beispiel 31

Ein Gemisch von 2,55 g 3-Cyan-5-bromacetyl-6-methyl-2-pyridon, 0,94 g 2-Aminopyridin und 90 ml Ethanol wird 5 Std. gekocht und wie üblich aufgearbeitet. Man erhält 3-Cyan-5-(imidazo[1,2-a]pyridin-2-yl)-6-methyl-2-pyridon, F. > 320 ° (Zers.). Hydrobromid, F. 307 - 310 ° (Zers.).

Analog erhält man aus den entsprechenden Aminopyridinen die nachstehenden 6-Methyl-2-pyridone:

3-Cyan-5-(5-methyl-imidazo[1,2-a]pyridin-2-yl)-
3-Cyan-5-(6-methyl-imidazo[1,2-a]pyridin-2-yl)-, Zers. ab 316 °
3-Cyan-5-(7-methyl-imidazo[1,2-a]pyridin-2-yl)-, Zers. ab 327 °
3-Cyan-5-(8-methyl-imidazo[1,2-a]pyridin-2-yl)-, F. 340 - 350 ° (Zers.)
3-Cyan-5-(5-carbamoyl-imidazo[1,2-a]pyridin-2-yl)-
3-Cyan-5-(6-carbamoyl-imidazo[1,2-a]pyridin-2-yl)-
3-Cyan-5-(7-carbamoyl-imidazo[1,2-a]pyridin-2-yl)-
3-Cyan-5-(8-carbamoyl-imidazo]1,2-a]pyridin-2-yl)-, Zers. ab. 350 °
3-Cyan-5-(5-cyan-imidazo[1,2-a]pyridin-2-yl)-
3-Cyan-5-(6-cyan-imidazo[1,2-a]pyridin-2-yl)-

3-Cyan-5-(7-cyan-imidazo[1,2-a]pyridin-2-yl)-
3-Cyan-5-(8-cyan-imidazo[1,2-a]pyridin-2-yl)-, F. > 350 °
3-Cyan-5-(6-fluor-imidazo[1,2-a]pyridin-2-yl)-
3-Cyan-5-(5-chlor-imidazo[1,2-a]pyridin-2-yl)-
3-Cyan-5-(6-chlor-imidazo[1,2-a]pyridin-2-yl)-, Hydrobromid, F. 340 °
3-Cyan-5-(7-chlor-imidazo[1,2-a]pyridin-2-yl)-
3-Cyan-5-(8-chlor-imidazo[1,2-a]pyridin-2-yl)-
3-Cyan-5-(6-brom-imidazo[1,2-a]pyridin-2-yl)-
3-Cyan-5-(6-jod-imidazo[1,2-a]pyridin-2-yl)-
3-Cyan-5-(5-hydroxy-imidazo[1,2-a]pyridin-2-yl)-
3-Cyan-5-(6-hydroxy-imidazo[1,2-a]pyridin-2-yl)-
3-Cyan-5-(7-hydroxy-imidazo[1,2-a]pyridin-2-yl)-
3-Cyan-5-(8-hydroxy-imidazo[1,2-a]pyridin-2-yl)-, Hydrobromid, Zers. ab 330 °
3-Cyan-5-(5-amino-imidazo[1,2-a]pyridin-2-yl)-
3-Cyan-5-(6-amino-imidazo[1,2-a]pyridin-2-yl)-
3-Cyan-5-(7-amino-imidazo[1,2-a]pyridin-2-yl)-
3-Cyan-5-(8-amino-imidazo[1,2-a]pyridin-2-yl)-, Hydrochlorid, F. 330 - 335 °
3-Cyan-4-methoxy-5-(8-carbamoyl-imidazo[1,2-a]pyridin-2-yl)-
3-Carbamoyl-5-(imidazo[1,2-a]pyridin-2-yl)-
3-Carbamoyl-5-(6-methylimidazo[1,2-a]pyridin-2-yl)-
3-Carbamoyl-5-(7-methylimidazo[1,2-a]pyridin-2-yl)-
3-Carbamoyl-5-(8-methylimidazo[1,2-a]pyridin-2-yl)-
3-Carbamoyl-5-(8-carbamoyl-imidazo[1,2-a]pyridin-2-yl)-
3-Carbamoyl-5-(8-cyan-imidazo[1,2-a]pyridin-2-yl)-
3-Carbamoyl-5-(6-chlor-imidazo[1,2-a]pyridin-2-yl)-
3-Carbamoyl-5-(8-hydroxy-imidazo[1,2-a]pyridin-2-yl)-
3-Carbamoyl-5-(8-amino-imidazo[1,2-a]pyridin-2-yl)-;

sowie

3-Cyan-5-(8-carbamoyl-imidazo[1,2-a]pyridin-2-yl)-6-ethyl-2-pyridon
3-Carbamoyl-5-(8-carbamoyl-imidazo[1,2-a]pyridin-2-yl)-6-ethyl-2-pyridon.

Beispiel 32

Eine Suspension von 1,92 g 3-Cyan-5-hydroxyacetyl-6-methyl-2-pyridon, 3,15 g Trifluoracetanhydrid, 1,1 g Triethylamin und 100 ml $CH_2Cl_2$ wird 4 Std. bei 25 ° gerührt. Man filtriert, dampft das Filtrat ein und erhält 3-Cyan-5-trifluoracetoxyacetyl-6-methyl-2-pyridon.

Analog erhält man mit Methan-, Hexan-, Benzol- oder p-Toluolsulfochlorid:

3-Cyan-5-methansulfonyloxyacetyl-6-methyl-2-pyridon
3-Cyan-5-hexansulfonyloxyacetyl-6-methyl-2-pyridon
3-Cyan-5-benzolsulfonyloxyacetyl-6-methyl-2-pyridon
3-Cyan-5-p-toluolsulfonyloxyacetyl-6-methyl-2-pyridon.

Beispiel 33

Man rührt eine Suspension von 2,18 g 3-Cyan-5-(1,3-dioxobutyl)-6-methyl-2-pyridon und 0,46 g Methylhydrazin in 80 ml Ethanol 24 Std. bei 20 °, filtriert und erhält durch fraktioniertes Kristallisieren aus Methanol 3-Cyan-5-(1,3-dimethyl-5-pyrazolyl)-6-methyl-2-pyridon vom F. > 300 ° und 3-Cyan-5-(1,5-dimethyl-3-pyrazolyl)-6-methyl-2-pyridon vom F. 286 - 288 ° (Zers.).

Analog erhält man:

mit Hydrazin: 3-Cyan-5-(3-methyl-5-pyrazolyl)-6-methyl-2-pyridon vom F. > 280 °;

mit Phenylhydrazin: 3-Cyan-5-(1-phenyl-3-methyl-5-pyrazolyl)-6-methyl-2-pyridon vom F. 286 - 287 ° und 3-Cyan-5-(1-phenyl-5-methyl-3-pyrazolyl)-6-methyl-2-pyridon vom F. 267 - 273 °;

aus 3-Cyan-5-[1,3-dioxo-3-(4-pyridyl)-propyl]-6-methyl-2-pyridon und Hydrazin: 3-Cyan-5-[3-(4-pyridyl)-5-pyrazolyl]-6-methyl-2-pyridon, F. > 350 °;

aus 3-Cyan-5-formylacetyl-6-methyl-2-pyridon und Methylhydrazin: 3-Cyan-5-(1-methyl-3-pyrazolyl)-6-methyl-2-pyridon, F. 265 - 270 ° (Zers.);

Analog erhält man aus den entsprechenden 3-Carbamoylverbindungen die folgenden 6-Methyl-2-pyridone:

3-Carbamoyl-5-(1,3-dimethyl-5-pyrazolyl)-
3-Carbamoyl-5-(1,5-dimethyl-3-pyrazolyl)-
3-Carbamoyl-5-(1-phenyl-3-methyl-5-pyrazolyl)-
3-Carbamoyl-5-(1-phenyl-5-methyl-3-pyrazolyl)-
3-Carbamoyl-5-(3-methyl-5-pyrazolyl)-
3-Carbamoyl-5-[3-(4-pyridyl)-5-pyrazolyl]-
3-Carbamoyl-5-(1-methyl-3-pyrazolyl)-.

Beispiel 34

Man kocht ein Gemisch von 2,93 g 3-Cyan-6-(4-pyridylcarbonyl)-1,2,5,6,7,8-hexahydro-chinolin-2,5-dion und 0,5 g Hydrazinhydrat in 200 ml Isopropanol in Gegenwart von 1,9 g p-Toluolsulfonsäure 6 Std., arbeitet wie üblich auf und erhält 1H-3-(4-Pyridyl)-8-cyan-4,5,6,7-tetrahydro-pyrazolo[3,4-f]chinolin-7-on, F. > 300 °.

Analog erhält man aus den entsprechenden Dioxoverbindungen mit den entsprechenden Hydrazinen die folgenden 8-Cyan-4,5,6,7-tetrahydro-pyrazolo[3,4-f]-chinolin-7-one:

1H-1-Methyl-, F. > 300 °
1H-3-Methyl-, F. > 300 °
1H-1,3-Dimethyl-, F. > 300 °
1H-1-Methyl-3-(4-pyridyl)-, F. > 300 °
1H-1-Phenyl-3-methyl-, F. > 300 °
1H-1-p-Methoxyphenyl-3-methyl-, F. 195 - 198 °.

Analog erhält man aus den entsprechenden 3-Carbamoyl-1,2,5,6,7,8-hexahydro-6-acyl-chinolin-2,5-dionen die folgenden 8-Carbamoyl-4,5,6,7-tetrahydro-pyrazolo-[3,4-f]chinolin-7-one:

1H-3-(4-Pyridyl)-
1H-1-Methyl-
1H-3-Methyl-
1H-1,3-Dimethyl-
1H-1-Methyl-3-(4-pyridyl)-
1H-1-Phenyl-3-methyl-
1H-1-p-Methoxyphenyl-3-methyl-.

Beispiel 35

Man suspendiert 2,48 g 3-Cyan-5-ethoxycarbonylacetyl-6-methyl-2-pyridon in 30 ml Ethanol und versetzt unter Rühren mit 0,5 g Hydrazinhydrat. Nach 22 Std. Kochen filtriert man heiß und erhält 3-Cyan-5-(3-hydroxy-5-pyrazolyl)-6-methyl-2-pyridon, F. > 300 °.

Analog erhält man aus 3-Carbamoyl-5-ethoxycarbonyl-acetyl-6-methyl-2-pyridon das 3-Carbamoyl-5-(3-hydroxy-5-pyrazolyl)-6-methyl-2-pyridon.

Beispiel 36

Ein Gemisch von 2,18 g 3-Cyan-5-(1,3-dioxobutyl)-6-methyl-2-pyridon, 0,69 g Hydroxylaminhydrochlorid und 50 ml Ethanol wird 5 Std. gekocht. 3-Cyan-5-(3-methyl-5-isoxazolyl)-6-methyl-2-pyridon, F. > 300 °, fällt aus.

Analog erhält man

3-Cyan-5-(3-ethyl-5-isoxazolyl)-6-methyl-2-pyridon
3-Cyan-5-(3-phenyl-5-isoxazolyl)-6-methyl-2-pyridon
3-Cyan-5-(3-p-methoxyphenyl-5-isoxazolyl)-6-methyl-2-pyridon
3-Cyan-5-[3-(2,4-dimethoxyphenyl)-5-isoxazolyl]-6-methyl-2-pyridon
3-Cyan-5-[3-(4-pyridyl)-5-isoxazolyl]-6-methyl-2-pyridon
3-Cyan-5-[3-(2-furyl)-5-isoxazolyl]-6-methyl-2-pyridon
3-Cyan-5-[3-(2-thienyl)-5-isoxazolyl]-6-methyl-2-pyridon
3-Cyan-5-(3-methyl-5-isoxazolyl)-6-ethyl-2-pyridon
3-Carbamoyl-5-(3-methyl-5-isoxazolyl)-6-methyl-2-pyridon.

Beispiel 37

Analog Beispiel 36 erhält man aus 3-Cyan-6-acetyl-1,2,5,6,7,8-hexahydrochinolin-2,5-dion und Hydroxylaminhydrochlorid das 3-Methyl-8-cyan-4,5,6,7-tetrahydro-isoxazolo[5,4-f]chinolin-7-on, F. > 300 °.

Analog erhält man

8-Cyan-4,5,6,7-tetrahydro-isoxazolo[5,4-f]chinolin-7-on, F. 247 - 248 °
3-Ethyl-8-cyan-4,5,6,7-tetrahydro-isoxazolo[5,4-f]-chinolin-7-on
3-Phenyl-8-cyan-4,5,6,7-tetrahydro-isoxazolo[5,4-f]-chinolin-7-on
3-p-Methoxyphenyl-8-cyan-4,5,6,7-tetrahydro-isoxazolo-[5,4-f]chinolin-7-on
3-(2,4-Dimethoxyphenyl)-8-cyan-4,5,6,7-tetrahydro-isoxazolo[5,4-f]chinolin-7-on
3-(4-Pyridyl)-8-cyan-4,5,6,7-tetrahydro-isoxazolo[5,4-f]-chinolin-7-on
3-(2-Furyl)-8-cyan-4,5,6,7-tetrahydro-isoxazolo[5,4-f]-chinolin-7-on
3-(2-Thienyl)-8-cyan-4,5,6,7-tetrahydro-isoxazolo[5,4-f]-chinolin-7-on
8-Carbamoyl-4,5,6,7-tetrahydro-isoxazolo[5,4-f]chinolin-7-on
3-Methyl-8-carbamoyl-4,5,6,7-tetrahydro-isoxazolo[5,4-f]-chinolin-7-on.

Beispiel 38

Man rührt 2,34 g 3-Cyan-5-acetoxyacetyl-6-methyl-2-pyridon und 1,36 g Isonicotinimidsäuremethylester in 100 ml flüssigem $NH_3$ 10 Std. bei 68 - 70 ° im Autoklaven. Als Zwischenprodukt entsteht Isonicotinamidin, es wird nicht isoliert. Man dampft ein und erhält 3-Cyan-5-[2-(4-pyridyl)-5-imidazolyl]-6-methyl-2-pyridon, F. > 300 °.

0154190

Analog sind erhältlich:

3-Cyan-5-(2-phenyl-5-imidazolyl)-6-methyl-2-pyridon
3-Cyan-5-(2-p-fluorphenyl-5-imidazolyl)-6-methyl-2-pyridon
3-Cyan-5-(2-p-methoxyphenyl-5-imidazolyl)-6-methyl-2-pyridon
3-Cyan-5-[2-(2-pyridyl)-5-imidazolyl]-6-methyl-2-pyridon
3-Cyan-5-[2-(3-pyridyl)-5-imidazolyl]-6-methyl-2-pyridon
3-Cyan-5-[2-(4-pyridyl)-5-imidazolyl]-6-ethyl-2-pyridon
3-Carbamoyl-5-[2-(4-pyridyl)-5-imidazolyl]-6-methyl-2-pyridon.

Beispiel 39

Ein Gemisch von 2,7 g 3-Cyan-4-methoxy-5-p-methoxyphenyl-6-methyl-2-pyridon, 0,36 g NaH, 3 g $CH_3J$ und 50 ml DMF wird bei 20 ° 16 Std. gerührt. Nach üblicher Aufarbeitung erhält man 1,6-Dimethyl-3-cyan-4-methoxy-5-p-methoxyphenyl-2-pyridon.

Analog erhält man mit Ethyljodid 1-Ethyl-3-cyan-4-methoxy-5-p-methoxyphenyl-6-methyl-2-pyridon.

Beispiel 40

Analog Beispiel 39 erhält man aus 3-Cyan-4-methyl-5-p-methoxyphenyl-2-pyridon mit Benzylchlorid das 1-Benzyl-3-cyan-4-methyl-5-p-methoxyphenyl-2-pyridon.

Analog erhält man:

1-Benzyl-3-carbamoyl-4-methyl-5-p-methoxyphenyl-2-pyridon.

Beispiel 41

Man rührt ein Gemisch von 3,84 g 3-Cyan-5-[2-(N-2-dimethylaminoethyl-amino)-4-thiazolyl]-6-methyl-2-pyridon-hydrobromid, 13,6 ml Ameisensäure und 32,8 ml Acetanhydrid 75 Min. bei 50 °, dann 1 Std. bei 20 °, filtriert und löst den Niederschlag in wäßrigem Ethanol. Mit verdünnter Natronlauge fällt man 3-Cyan-5-[2-(N-2-dimethylaminoethyl-formamido)-4-thiazolyl]-6-methyl-2-pyridon, F. 205 - 208 ° (Zers.).

Analog erhält man durch Formylierung die folgenden 6-Methyl-2-pyridone:

3-Cyan-5-(2-formamido-4-thiazolyl)-, F. 294 - 297 °
3-Cyan-5-(2-N-methyl-formamido-4-thiazolyl)-, F. 285 - 288 °
3-Cyan-5-(2-N-ethyl-formamido-4-thiazolyl)-, F. 273 - 274 °
3-Cyan-5-(2-N-propyl-formamido-4-thiazolyl)-
3-Cyan-5-(2-N-isopropyl-formamido-4-thiazolyl)-
3-Cyan-5-(2-N-butyl-formamido-4-thiazolyl)-
3-Cyan-5-(2-N-isobutyl-formamido-4-thiazolyl)-
3-Cyan-5-(2-N-sek.-butyl-formamido-4-thiazolyl)-
3-Cyan-5-(2-N-tert.-butyl-formamido-4-thiazolyl)-
3-Cyan-5-(2-N-pentyl-formamido-4-thiazolyl)-
3-Cyan-5-(2-N-hexyl-formamido-4-thiazolyl)-
3-Cyan-5-(2-N-allyl-formamido-4-thiazolyl)-
3-Cyan-5-[2-(N-2-hydroxyethyl-formamido)-4-thiazolyl]-
3-Cyan-5-[2-(N-2-dimethylaminopropyl-formamido)-4-thiazolyl]-
3-Cyan-5-[3-(N-3-dimethylaminopropyl-formamido)-4-thiazolyl]-, F. 275 - 277 °

3-Cyan-5-[2-(N-2-diethylaminoethyl-formamido)-4-thiazolyl]-, Hydrobromid, F. 247 - 248 °
3-Cyan-5-[2-(N-2-pyrrolidinoethyl-formamido)-4-thiazolyl]-, F. 273 - 274 °
3-Cyan-5-[2-(N-2-piperidinoethyl-formamido)-4-thiazolyl]-, F. 266 - 267 °
3-Cyan-5-[2-(N-3-(2-methylpiperidino)-propyl)-formamido-4-thiazolyl]-
3-Cyan-5-[2-(N-3-(2-oxopyrrolidino)-propyl)-formamido-4-thiazolyl]-, F. 235 - 237 °
3-Cyan-5-[2-(N-2-morpholinoethyl-formamido)-4-thiazolyl]-, Hydrobromid, F. 259 - 261 °
3-Cyan-5-[2-(N-3-morpholinopropyl-formamido)-4-thiazolyl]-, F. 188 ° (Zers.).
3-Cyan-5-[2-(N-2-(4-formylpiperazino)-ethyl-formamido)-4-thiazolyl]-
3-Cyan-5-[2-(N-2-methoxyethyl-formamido)-4-thiazolyl]-, F. 249 - 251 °
3-Cyan-5-(2-N-benzyl-formamido-4-thiazolyl)-
3-Cyan-5-[2-(N-p-methoxybenzyl-formamido)-4-thiazolyl]-, F. 241 - 243 °
3-Cyan-5-[2-(N-2-phenylethyl-formamido)-4-thiazolyl]-
3-Cyan-5-[2-(N-2-p-methoxyphenylethyl-formamido)-4-thiazoyl]-
3-Cyan-5-[2-(N-2-(3,4-dimethoxyphenyl)-ethyl-formamido)-4-thiazolyl]-
3-Cyan-5-[2-(N-1-indanyl-formamido)-4-thiazolyl]-
3-Cyan-5-[2-(N-2-(2-pyridyl)-ethyl-formamido)-4-thiazolyl]-, F. 240 - 242 °
3-Cyan-5-[2-(N-2-(3-pyridyl)-ethyl-formamido)-4-thiazolyl]-
3-Cyan-5-[2-(N-2-(4-pyridyl)-ethyl-formamido)-4-thiazolyl]-, F. 252 - 255 °
3-Cyan-5-[2-(N-4-piperidyl-formamido)-4-thiazolyl]-
3-Cyan-5-[2-(N-(1-benzyl-4-piperidyl)-formamido)-4-thiazolyl]-

3-Cyan-5-[2-(N-(1-ethoxycarbonyl-4-piperidyl)-formamido)-4-thiazolyl]-

3-Cyan-5-[2-(N-2-(4-methyl-5-imidazolylmethylthio)-ethyl-formamido)-4-thiazolyl]-

3-Cyan-5-[2-(4-formylpiperazino)-4-thiazolyl]-, F. 304 - 305 °

3-Carbamoyl-5-(2-formamido-4-thiazolyl)-

3-Carbamoyl-5-(2-N-methyl-formamido-4-thiazolyl)-

3-Carbamoyl-5-(2-N-ethyl-formamido-4-thiazolyl)-

3-Carbamoyl-5-[2-(N-2-dimethylaminoethyl-formamido)-4-thiazolyl]-

3-Carbamoyl-5-[2-(N-3-dimethylaminopropyl-formamido)-4-thiazolyl]-

3-Carbamoyl-5-[2-(N-2-diethylaminoethyl-formamido)-4-thiazolyl]-

3-Carbamoyl-5-[2-(N-2-pyrrolidinoethyl-formamido)-4-thiazolyl]-

3-Carbamoyl-5-[2-(N-2-piperidinoethyl-formamido)-4-thiazolyl]-

3-Carbamoyl-5-[2-(N-3-oxopyrrolidinopropyl-formamido)-4-thiazolyl]-

3-Carbamoyl-5-[2-(N-2-morpholinoethyl-formamido)-4-thiazolyl]-

3-Carbamoyl-5-[2-(N-3-morpholinopropyl-formamido)-4-thiazolyl]-

3-Carbamoyl-5-[2-(N-2-methoxyethyl-formamido)-4-thiazolyl]-

3-Carbamoyl-5-[2-(N-p-methoxybenzyl-formamido)-4-thiazolyl]-

3-Carbamoyl-5-[2-(N-2-(2-pyridyl)-ethyl-formamido)-4-thiazolyl]-

3-Carbamoyl-5-[2-(N-2-(3-pyridyl)-ethyl-formamido)-4-thiazolyl]-

3-Carbamoyl-5-[2-(N-2-(4-pyridyl)-ethyl-formamido)-4-thiazolyl]-
3-Carbamoyl-5-[2-(4-formylpiperazino)-4-thiazolyl]-

sowie die folgenden 8-Cyan-4,5,6,7-tetrahydro-thiazolo-[4,5-f]chinolin-7-one:

2-(N-2-Dimethylaminoethyl-formamido)-, Hydrobromid, F. > 300 °
2-(N-3-Dimethylaminopropyl-formamido)-, F. 279 - 281 °
2-(N-Benzyl-formamido)-, F. > 300 °
2-(N-p-Methoxybenzyl-formamido)-, F. > 300 ° (Zers.);

die folgenden 8-Carbamoyl-4,5,6,7-tetrahydro-thiazolo-[4,5-f]chinolin-7-one:

2-(N-2-Dimethylaminoethyl-formamido)-
2-(N-3-Dimethylaminopropyl-formamido)-
2-(N-Benzyl-formamido)-
2-(N-p-Methoxybenzyl-formamido)-;

die folgenden 8-Cyan-6,7-dihydro-thiazolo[4,5-f]chinolin-7-one:

2-(N-2-Dimethylaminoethyl-formamido)-, F. 252 - 253 °
2-(N-3-Dimethylaminopropyl-formamido)-, F. 297 ° (Zers.)
2-(N-Benzyl-formamido)-, F. > 300 °
2-(N-p-Methoxybenzyl-formamido)-, F. 298 ° (Zers.)

die folgenden 8-Carbamoyl-6,7-dihydro-thiazolo[4,5-f]-chinolin-7-one:

2-(N-2-Dimethylaminoethyl-formamido)-
2-(N-3-Dimethylaminopropyl-formamido)-

2-(N-Benzyl-formamido)-
2-(N-p-Methoxybenzyl-formamido)-.

Beispiel 42

Ein Gemisch von 2,46 g 3-Cyan-5-(2-methylamino-4-thiazolyl)-6-methyl-2-pyridon, 2 ml Acetylchlorid und 20 ml 2n NaOH wird über Nacht bei 20 ° gerührt. Nach üblicher Aufarbeitung erhält man 3-Cyan-5-(2-N-methyl-acetamido-4-thiazolyl)-6-methyl-2-pyridon, F. 320 - 322 °.

Analog erhält man durch Acetylierung die folgenden 6-Methyl-2-pyridone:

3-Cyan-5-(2-acetamido-4-thiazolyl)-
3-Cyan-5-(2-N-ethyl-acetamido-4-thiazolyl)-, F. 261 - 262 °
3-Cyan-5-(2-N-propyl-acetamido-4-thiazolyl)-
3-Cyan-5-(2-N-isopropyl-acetamido-4-thiazolyl)-, F. 205 - 206 °
3-Cyan-5-(2-N-butyl-acetamido-4-thiazolyl)-
3-Cyan-5-(2-N-isobutyl-acetamido-4-thiazolyl)-
3-Cyan-5-(2-N-sek.-butyl-acetamido-4-thiazolyl)-
3-Cyan-5-(2-N-tert.-butyl-acetamido-4-thiazolyl)-
3-Cyan-5-(2-N-pentyl-acetamido-4-thiazolyl)-
3-Cyan-5-(2-N-hexyl-acetamido-4-thiazolyl)-
3-Cyan-5-(2-N-allyl-acetamido-4-thiazolyl)-
3-Cyan-5-[2-(N-2-hydroxyethyl-acetamido)-4-thiazolyl]-
3-Cyan-5-[2-(N-2-dimethylaminoethyl-acetamido)-4-thiazolyl]-, F. 263 - 267 °
3-Cyan-5-[2-(N-3-dimethylaminopropyl-acetamido)-4-thiazolyl]-, F. 273 - 275 °
3-Cyan-5-[2-(N-2-diethylaminoethyl-acetamido)-4-thiazolyl]-, Hydrobromid, F. 264 - 266 °
3-Cyan-5-[2-(N-2-pyrrolidinoethyl-acetamido)-4-thiazolyl]-, F. 285 - 288 °

3-Cyan-5-[2-(N-2-piperidinoethyl-acetamido)-4-thiazolyl]-, F. 270 °

3-Cyan-5-[2-(N-3-(2-methylpiperidino)-propyl-acetamido-4-thiazolyl]-

3-Cyan-5-[2-(N-3-(2-oxopyrrolidino)-propyl-acetamido-4-thiazolyl]-, F. 247 - 249 °

3-Cyan-5-[2-(N-2-morpholinoethyl-acetamido)-4-thiazolyl]-, Hydrobromid, F. 258 - 260 °

3-Cyan-5-[2-(N-3-morpholinopropyl-acetamido)-4-thiazolyl]-, F. 230 - 232 °

3-Cyan-5-[2-(N-2-(4-acetylpiperazino)-ethyl-acetamido)-4-thiazolyl]-

3-Cyan-5-[2-(N-2-methoxyethyl-acetamido)-4-thiazolyl]-, F. 243 - 245 °

3-Cyan-5-[2-(N-benzyl-acetamido)-4-thiazolyl]-, F. 250 - 253 °

3-Cyan-5-[2-(N-p-methoxybenzyl-acetamido)-4-thiazolyl]-, F. 186 - 188 °

3-Cyan-5-[2-(N-2-phenylethyl-acetamido)-4-thiazolyl]-

3-Cyan-5-[2-(N-2-p-methoxyphenylethyl-acetamido)-4-thiazolyl]-

3-Cyan-5-[2-(N-2-(3,4-dimethoxyphenyl)-ethyl-acetamido)-4-thiazolyl]-

3-Cyan-5-[2-(N-1-indanyl-acetamido)-4-thiazolyl]-

3-Cyan-5-[2-(N-2-(2-pyridyl)-ethyl-acetamido)-4-thiazolyl]-, F. 239 - 242 °

3-Cyan-5-[2-(N-2-(3-pyridyl)-ethyl-acetamido)-4-thiazolyl]-

3-Cyan-5-[2-(N-2-(4-pyridyl)-ethyl-acetamido)-4-thiazolyl]-, F. > 300°

3-Cyan-5-[2-(N-4-piperidyl-acetamido)-4-thiazolyl]-

3-Cyan-5-[2-(N-1-benzyl-4-piperidyl-acetamido)-4-thiazolyl]-

3-Cyan-5-[2-(N-1-ethoxycarbonyl-4-piperidyl-acetamido)-4-thiazolyl]-

3-Cyan-5-[2-(N-2-(4-methyl-5-imidazolylmethylthio)-ethyl-acetamido)-4-thiazolyl]-

3-Cyan-5-[2-(N-phenyl-acetamido)-4-thiazolyl]-
3-Cyan-5-[2-(N-p-methoxyphenyl-acetamido)-4-thiazolyl]-, F. 304 - 306 °
3-Cyan-5-(2-acetamido-4-methyl-5-thiazolyl)-, F. > 355 °
3-Carbamoyl-5-(2-N-methyl-acetamido-4-thiazolyl)-
3-Carbamoyl-5-(2-N-ethyl-acetamido-4-thiazolyl)-
3-Carbamoyl-5-(2-N-isopropyl-acetamido-4-thiazolyl)-
3-Carbamoyl-5-[2-(N-2-dimethylaminoethyl-acetamido)-4-thiazolyl]-
3-Carbamoyl-5-[2-(N-3-dimethylaminopropyl-acetamido)-4-thiazolyl]-
3-Carbamoyl-5-[2-(N-2-diethylaminoethyl-acetamido)-4-thiazolyl]-
3-Carbamoyl-5-[2-(N-2-pyrrolidinoethyl-acetamido)-4-thiazolyl]-
3-Carbamoyl-5-[2-(N-2-piperidinoethyl-acetamido)-4-thiazolyl]-
3-Carbamoyl-5-[2-(N-3-(2-oxopyrrolidino)-propyl-acetamido)-4-thiazolyl]-
3-Carbamoyl-5-[2-(N-2-morpholinoethyl-acetamido)-4-thiazolyl]-
3-Carbamoyl-5-[2-(N-2-morpholinopropyl-acetamido)-4-thiazolyl]-
3-Carbamoyl-5-[2-(N-2-methoxyethyl-acetamido)-4-thiazolyl]-
3-Carbamoyl-5-[2-(N-benzyl-acetamido)-4-thiazolyl]-
3-Carbamoyl-5-[2-(N-p-methoxybenzyl-acetamido)-4-thiazolyl]-
3-Carbamoyl-5-[2-(N-(2-pyridyl)-ethyl-acetamido)-4-thiazolyl]-
3-Carbamoyl-5-[2-(N-(3-pyridyl)-ethyl-acetamido)-4-thiazolyl]-
3-Carbamoyl-5-[2-(N-(4-pyridyl)-ethyl-acetamido)-4-thiazolyl]-

3-Carbamoyl-5-[2-(N-p-methoxyphenyl-acetamido)-4-thiazolyl]-
3-Carbamoyl-5-(2-acetamido-4-methyl-5-thiazolyl)-;

sowie die folgenden 8-Cyan-4,5,6,7-tetrahydro-thiazolo-[4,5-f]chinolin-7-one:

2-Acetamido-
2-(N-2-Dimethylaminoethyl-acetamido)-
2-(N-3-Dimethylaminopropyl-acetamido)-
2-(N-Benzyl-acetamido)-
2-(N-p-Methoxybenzyl-acetamido)-;

die folgenden 8-Carbamoyl-4,5,6,7-tetrahydro-thiazolo-[4,5-f]chinolin-7-one:

2-Acetamido-
2-(N-2-Dimethylaminoethyl-acetamido)-
2-(N-3-Dimethylaminopropyl-acetamido)-
2-(N-Benzyl-acetamido)-
2-(N-p-Methoxybenzyl-acetamido)-;

die folgenden 8-Cyan-6,7-dihydro-thiazolo[4,5-f]chinolin-7-one:

2-Acetamido-, F. > 300 °
2-(N-2-Dimethylaminoethyl-acetamido)-, F. 293 - 294 °
2-(N-3-Dimethylaminopropyl-acetamido)-, F. > 300 °
2-(N-Benzyl-acetamido)-
2-(N-p-Methoxybenzyl-acetamido)-, F. 302 ° (Zers.);

die folgenden 8-Carbamoyl-6,7-dihydro-thiazolo[4,5-f]-chinolin-7-one:

2-Acetamido-
2-(N-2-Dimethylaminoethyl-acetamido)-
2-(N-3-Dimethylaminopropyl-acetamido)-
2-(N-Benzyl-acetamido)-
2-(N-p-Methoxybenzyl-acetamido)-.

Beispiel 43

Analog Beispiel 42 erhält man aus den entsprechenden Aminen durch Acylierung die folgenden 6-Methyl-2-pyridone:

3-Cyan-5-(2-propionamido-4-thiazolyl)-
3-Cyan-5-(2-N-methyl-propionamido-4-thiazolyl)-
3-Cyan-5-(2-N-ethyl-propionamido-4-thiazolyl)-, F. 263 - 265 °
3-Cyan-5-(2-N-propyl-propionamido-4-thiazolyl)-
3-Cyan-5-(2-N-isopropyl-propionamido-4-thiazolyl)-, F. 235 - 238 °
3-Cyan-5-[2-(N-2-dimethylaminoethyl-propionamido)-4-thiazolyl]-, F. 275 - 278 °
3-Cyan-5-[2-(N-3-dimethylaminopropyl-propionamido)-4-thiazolyl]-
3-Cyan-5-[2-(N-2-diethylaminoethyl-propionamido)-4-thiazolyl]-
3-Cyan-5-[2-(N-2-pyrrolidinoethyl-propionamido)-4-thiazolyl]-, F. 287 - 289 °
3-Cyan-5-[2-(N-2-piperidinoethyl-propionamido)-4-thiazolyl]-, F. 281 - 282 °
3-Cyan-5-[2-(N-3-(2-methylpiperidino)-propyl-propionamido)-4-thiazolyl]-
3-Cyan-5-[2-(N-3-(2-oxopyrrolidino)-propyl-propionamido)-4-thiazolyl]-, F. 216 - 217 °
3-Cyan-5-[2-(N-2-morpholinoethyl-propionamido)-4-thiazolyl]-

3-Cyan-5-[2-(N-2-(2-pyridyl)-ethyl-butyramido)-4-thiazolyl]-, F. 219 - 221 °
3-Cyan-5-(2-benzamido-4-thiazolyl)-
3-Cyan-5-(2-N-methyl-benzamido-4-thiazolyl)-
3-Cyan-5-(2-N-ethyl-benzamido-4-thiazolyl)-
3-Cyan-5-(2-N-propyl-benzamido-4-thiazolyl)-
3-Cyan-5-(2-N-isopropyl-benzamido-4-thiazolyl)-
3-Cyan-5-[2-(N-2-dimethylaminoethyl-benzamido)-4-thiazolyl]-, Hydrochlorid, F. 259 - 261 °
3-Cyan-5-[2-(N-3-dimethylaminopropyl-benzamido)-4-thiazolyl]-, F. 246 - 248 °
3-Cyan-5-[2-(N-2-diethylaminoethyl-benzamido)-4-thiazolyl]-, Hydrochlorid, F. 275 - 276 °
3-Cyan-5-[2-(N-2-pyrrolidinoethyl-benzamido)-4-thiazolyl]-
3-Cyan-5-[2-(N-2-piperidinoethyl-benzamido)-4-thiazolyl]-
3-Cyan-5-[2-(N-3-(2-methylpiperidino)-propyl-benzamido)-4-thiazolyl]-
3-Cyan-5-[2-(N-3-(2-oxopyrrolidino)-propyl-benzamido)-4-thiazolyl]-
3-Cyan-5-[2-(N-2-morpholinoethyl-benzamido)-4-thiazolyl]-, F. 281 - 283 °
3-Cyan-5-[2-(N-2-methoxyethyl-benzamido)-4-thiazolyl]-, F. 272 - 273 °
3-Cyan-5-(2-p-methoxybenzamido-4-thiazolyl)-
3-Cyan-5-[2-(N-2-dimethylaminoethyl-p-methoxybenzamido)-4-thiazolyl]-, Hydrochlorid, F. 229 - 230 °
3-Cyan-5-[2-(N-3-dimethylaminopropyl-p-methoxybenzamido)-4-thiazolyl]-, Hydrochlorid, F. 243 - 245 °
3-Cyan-5-[2-(N-2-morpholinoethyl-p-methoxybenzamido)-4-thiazolyl]-, F. 249 - 251 °
3-Cyan-5-[2-(N-2-methoxyethyl-p-methoxybenzamido)-4-thiazolyl]-, F. 223 - 224 °

3-Cyan-5-[2-(N-2-dimethylaminoethyl-p-methoxyphenyl-acetamido)-4-thiazolyl]-, F. 263 - 265 °
3-Cyan-5-[2-(N-3-dimethylaminopropyl-p-methoxyphenyl-acetamido)-4-thiazolyl]-, F. 146 - 148 °
3-Cyan-5-[2-(N-2-diethylaminoethyl-p-methoxyphenyl-acetamido)-4-thiazolyl]-, F. 202 - 204 °
3-Cyan-5-[2-(N-2-methoxyethyl-p-methoxyphenylacetamido)-4-thiazolyl]-, F. 241 - 245 °
3-Cyan-5-(2-nicotinamido-4-thiazolyl)-
3-Cyan-5-[2-(N-2-dimethylaminoethyl-nicotinamido)-thiazolyl]-, Hydrochlorid, F. 235 - 237 °
3-Cyan-5-[2-(N-3-dimethylaminopropyl-nicotinamido)-4-thiazolyl]-, Dihydrochlorid, F. 176 - 179 °
3-Cyan-5-[2-(N-2-diethylaminoethyl-nicotinamido)-4-thiazolyl]-, F. 254 - 256 °
3-Cyan-5-[2-(N-2-methoxyethyl-nicotinamido)-4-thiazolyl]-6-methyl-2-pyridon
3-Cyan-5-(2-isonicotinamido-4-thiazolyl)-
3-Cyan-5-[2-(N-2-dimethylaminoethyl-isonicotinamido)-4-thiazolyl]-, F. 271 - 273 °
3-Cyan-5-[2-(N-3-dimethylaminopropyl-isonicotinamido)-4-thiazolyl]-, Dihydrochlorid, F. 228 - 231 °
3-Cyan-5-[2-(N-2-diethylaminoethyl-isonicotinamido)-4-thiazolyl]-, Dihydrochlorid, F. 258 - 260 °
3-Cyan-5-[2-methoxyethyl-isonicotinamido)-4-thiazolyl]-
3-Cyan-5-[2-(N-ethoxycarbonyl-N-ethyl-amino)-4-thiazolyl]-
3-Carbamoyl-5-(2-N-ethyl-propionamido-4-thiazolyl)-
3-Carbamoyl-5-(2-N-isopropyl-propionamido-4-thiazolyl)-
3-Carbamoyl-5-[2-(N-2-dimethylaminoethyl-propionamido)-4-thiazolyl]-
3-Carbamoyl-5-[2-(N-2-pyrrolidinoethyl-propionamido)-4-thiazolyl]-
3-Carbamoyl-5-[2-(N-2-piperidinoethyl-propionamido)-4-thiazolyl]-

3-Carbamoyl-5-[2-(N-3-(2-oxopyrrolidino)-propyl-propionamido)-4-thiazolyl]-
3-Carbamoyl-5-[2-(N-2-(2-pyridyl)-ethyl-butyramido)-4-thiazolyl]-
3-Carbamoyl-5-[2-(N-2-dimethylaminoethyl-benzamido)-4-thiazolyl]-
3-Carbamoyl-5-[2-(N-3-dimethylaminopropyl-benzamido)-4-thiazolyl]-
3-Carbamoyl-5-[2-(N-2-diethylaminoethyl-benzamido)-4-thiazolyl]-
3-Carbamoyl-5-[2-(N-2-morpholinoethyl-benzamido)-4-thiazolyl]-
3-Carbamoyl-5-[2-(N-2-methoxyethyl-benzamido)-4-thiazolyl]-
3-Carbamoyl-5-[2-(N-2-dimethylaminoethyl-p-methoxybenzamido)-4-thiazolyl]-
3-Carbamoyl-5-[2-(N-3-dimethylaminopropyl-p-methoxybenzamido)-4-thiazolyl]-
3-Carbamoyl-5-[2-(N-2-morpholinoethyl-p-methoxybenzamido)-4-thiazolyl]-
3-Carbamoyl-5-[2-(N-2-methoxyethyl-p-methoxybenzamido)-4-thiazolyl]-
3-Carbamoyl-5-[2-(N-2-dimethylaminoethyl-p-methoxyphenylacetamido)-4-thiazolyl]-
3-Carbamoyl-5-[2-(N-3-dimethylaminopropyl-p-methoxyphenylacetamido)-4-thiazolyl]-
3-Carbamoyl-5-[2-(N-2-diethylaminoethyl-p-methoxyphenylacetamido)-4-thiazolyl]-
3-Carbamoyl-5-[2-(N-2-methoxyethyl-p-methoxyphenylacetamido)-4-thiazolyl]-
3-Carbamoyl-5-[2-(N-2-dimethylaminoethyl-nicotinamido)-4-thiazolyl]-
3-Carbamoyl-5-[2-(N-3-dimethylaminopropyl-nicotinamido)-4-thiazolyl]-
3-Carbamoyl-5-[2-(N-2-diethylaminoethyl-nicotinamido)-4-thiazolyl]-

3-Carbamoyl-5-[2-(N-2-methoxyethyl-nicotinamido)-4-thiazolyl]-
3-Carbamoyl-5-[2-(N-2-dimethylaminoethyl-isonicotinamido)-4-thiazolyl]-
3-Carbamoyl-5-[2-(N-3-dimethylaminopropyl-isonicotinamido)-4-thiazolyl]-
3-Carbamoyl-5-[2-(N-2-diethylaminoethyl-isonicotinamido)-4-thiazolyl]-
3-Carbamoyl-5-[2-(N-2-methoxyethyl-isonicotinamido)-4-thiazolyl]-.

Beispiel 44

Man rührt 4,12 g 3-Cyan-5-[2-(N-2-diethylaminoethylamino)-4-thiazolyl]-6-methyl-2-pyridon-hydrobromid, 5,1 g p-Methoxy-benzoylchlorid, 4,44 g Triethylamin und 120 ml Acetonitril 90 Min. bei 20 °, filtriert und dampft das Filtrat ein. Nach Kristallisation des Rückstands aus Methanol erhält man 2-p-Methoxybenzoyloxy-3-cyan-5-[2-(N-2-diethylaminoethyl-p-methoxybenzamido)-4-thiazolyl]-6-methylpyridin, F. 195 - 200 ° (Zers.)

Analog sind durch Acylierung die folgenden 6-Methylpyridine erhältlich:

2-Benzoyloxy-3-cyan-5-[2-(N-2-dimethylaminoethyl-benzamido)-4-thiazolyl]-, F. 172 - 175 °
2-Benzoyloxy-3-cyan-5-[2-(N-2-diethylaminoethyl-benzamido)-4-thiazolyl]-, F. 210 - 212 °
2-Benzoyloxy-3-cyan-5-[2-(N-3-dimethylaminopropyl-benzamido)-4-thiazolyl]-, F. 188 - 190 °
2-Benzoyloxy-3-cyan-5-[2-(N-2-morpholinoethyl-benzamido)-4-thiazolyl]-, F. 100 - 102 °
2-p-Methoxybenzoyloxy-3-cyan-5-[2-(N-2-dimethylaminoethyl-p-methoxybenzamido)-4-thiazolyl]-, F. 134 - 137 °

2-p-Methoxybenzoyloxy-3-cyan-5-[2-(N-3-dimethylaminopropyl-p-methoxybenzamido)-4-thiazolyl]-, F. 136 - 139 °
2-p-Methoxybenzoyloxy-3-cyan-5-[2-(N-2-morpholinoethyl-p-methoxybenzamido)-4-thiazolyl]-, F. 128 - 130 °.

Durch 2 Std. Kochen von 3-Cyan-5-(2-benzylamino-4-thiazolyl)-6-methyl-pyridon mit überschüssigem Acetanhydrid ist analog erhältlich das 2-Acetoxy-3-cyan-5-(2-N-benzyl-acetamido-4-thiazolyl)-6-methyl-pyridin, F. 134 - 136 °.

Beispiel 45

Man rührt 2,32 g 3-Cyan-5-(2-amino-4-thiazolyl)-6-methyl-2-pyridon, 2,28 g Methylisocyanat und 1 g Triethylamin in 25 ml DMF 8 Std. bei 20 °, arbeitet wie üblich auf und erhält 3-Cyan-5-(2-N'-methylureido-4-thiazolyl)-6-methyl-2-pyridon, F. > 300 °.

Analog erhält man die folgenden 6-Methyl-2-pyridone:

3-Cyan-5-(2-N'-ethylureido-4-thiazolyl)-
3-Carbamoyl-5-(2-N'-methylureido-4-thiazolyl)-
3-Carbamoyl-5-(2-N'-ethylureido-4-thiazolyl)-.

Beispiel 46

Man erwärmt 1 g 3-Cyan-5-(2-amino-4-thiazolyl)-6-methyl-2-pyridon mit 10 ml 80 %iger $H_2SO_4$ 3 Std. auf 80 °, kühlt ab, gießt auf Eis und erhält 3-Carbamoyl-5-(2-amino-4-thiazolyl)-6-methyl-2-pyridon, Hydrogensulfat, F. > 300 °. Durch Behandeln mit gesättigter $NaHCO_3$-Lösung kann das Salz in die freie Base, F. > 300 °, übergeführt werden.

Analog erhält man durch partielle Hydrolyse der entsprechenden Nitrile die folgenden 6-Methyl-2-pyridone:

3-Carbamoyl-5-(2-methyl-4-thiazolyl)-
3-Carbamoyl-5-(2-carbamoylmethyl-4-thiazolyl)-
3-Carbamoyl-5-[2-N-methyl-N-(2-dimethylaminoethyl)-amino-4-thiazolyl]-

sowie die folgenden 8-Carbamoyl-4,5,6,7-tetrahydro-thiazolo[4,5-f]chinolin-7-one:

2-Amino-, F. > 290 °
2-(N-Methyl-N-2-dimethylaminoethyl-amino)-
2-(N-Ethyl-N-2-dimethylaminoethyl-amino)-
2-(N-Isoproypl-N-2-dimethylaminoethyl-amino)-
2-(N-Benzyl-N-2-dimethylaminoethyl-amino)-
2-(N-p-Methoxyphenyl-N-2-dimethylaminoethyl-amino)-
2-(N-Ethyl-N-2-diethylaminoethyl-amino)-.

Beispiel 47

Man erhitzt 2,32 g 3-Cyan-5-(2-amino-4-thiazolyl)-6-methyl-2-pyridon in 40 ml 50 %iger $H_2SO_4$ 5 Std. auf 100 - 105 °, kühlt ab, gießt auf Eis und erhält 5-(2-Amino-4-thiazolyl)-6-methyl-2-pyridon-2-carbonsäure, F. > 300 °.

Analog erhält man durch Hydrolyse der entsprechenden Nitrile die nachstehenden 6-Methyl-2-pyridon-3-carbonsäuren:

5-(2-p-Methoxyanilino-4-thiazolyl)-
5-(2-p-Diethylphosphonomethylphenyl-4-thiazolyl)-
5-(2-Benzylamino-4-thiazolyl)-

5-(2-p-Methoxybenzylamino-4-thiazolyl)-
5-(2-Dimethylamino-4-thiazolyl)-
5-[2-(N-Isopropyl-N-2-dimethylaminoethyl-amino)-4-thiazolyl]-
5-[2-(N-p-Methoxyphenyl-N-2-dimethylaminoethyl-amino)-4-thiazolyl]-
sowie
2,5-Dioxo-1,2,5,6,7,8-hexahydrochinolin-3-carbonsäure, F. 276 - 280 ° (Zers.)
2-Dimethylamino-6,7-dihydro-thiazolo[4,5-f]chinolin-7-on-8-carbonsäure.

Beispiel 48

Man erhitzt 1 g 3-Carbamoyl-5-(2-amino-4-thiazolyl)-6-methyl-2-pyridon in 20 ml 50 %iger $H_2SO_4$ 5 Std. auf 100 °, kühlt ab, gießt auf Eis und erhält 5-(2-Amino-4-thiazolyl)-6-methyl-2-pyridon-3-carbonsäure, F. > 300 °.

Beispiel 49

Man kocht 1 g 2,5-Dioxo-1,2,5,6,7,8-hexahydrochinolin-3-carbonsäuremethylester 6 Std. mit einer Lösung von 0,3 g NaOH in 10 ml $CH_3OH$ und 10 ml Wasser, konzentriert die Lösung, säuert an und erhält 2,5-Dioxo-1,2,5,6,7,8-hexahydrochinolin-3-carbonsäure, F. 276 - 280 ° (Zers.).

Beispiel 50

Man kocht 6 g 2-p-Methoxybenzoyloxy-3-cyan-5-[2-(N-2-diethylaminoethyl-p-methoxybenzamido-4-thiazolyl]-6-methylpyridin mit 50 ml methanolischer HCl-Lösung 1 Std., kühlt ab und filtriert das ausgefallene 3-Cyan-5-[2-(N-2-diethylaminoethyl-p-methoxybenzamido-4-thiazolyl]-6-methyl-2-pyridon-hydrochlorid ab; F. 241 - 243 °.

Analog erhält man durch Hydrolyse der übrigen in Beispiel 44 genannten Lactimester (2-Hydroxypyridylester) die zugrundeliegenden 2-Pyridone.

Beispiel 51

Man suspendiert 1 g 2-p-Diethylphosphonomethylphenyl-8-cyan-4,5,6,7-tetrahydro-thiazolo[4,5-f]chinolin-7-on in 50 ml Ethanol, leitet HBr bis zur Sättigung ein und läßt 16 Std. bei 20 ° stehen. Nach dem Eindampfen und Waschen des Rückstands mit Wasser erhält man 2-p-Monoethylphosphonomethylphenyl-8-cyan-4,5,6,7-tetrahydrothiazolo[4,5-f]chinolin-7-on, F. 299 - 301 °.

Analog erhält man durch partielle Verseifung der entsprechenden Diester mit methanolischer bzw. ethanolischer HBr:

3-Cyan-5-(2-o-monomethylphosphonomethylphenyl-4-thiazolyl)-6-methyl-2-pyridon
3-Cyan-5-(2-m-monomethylphosphonomethylphenyl-4-thiazolyl)-6-methyl-2-pyridon
3-Cyan-5-(2-p-monomethylphosphonomethylphenyl-4-thiazolyl)-6-methyl-2-pyridon, F. > 300 °
3-Cyan-5-(2-o-monoethylphosphonomethylphenyl-4-thiazolyl)-6-methyl-2-pyridon
3-Cyan-5-(2-m-monoethylphosphonomethylphenyl-4-thiazolyl)-6-methyl-2-pyridon
3-Cyan-5-(2-p-monoethylphosphonomethylphenyl-4-thiazolyl)-6-methyl-2-pyridon

2-o-Monomethylphosphonomethylphenyl-8-cyan-4,5,6,7-tetrahydrothiazolo[4,5-f]chinolin-7-on
2-m-Monomethylphosphonomethylphenyl-8-cyan-4,5,6,7-tetrahydrothiazolo[4,5-f]chinolin-7-on
2-p-Monomethylphosphonomethylphenyl-8-cyan-4,5,6,7-tetrahydrothiazolo[4,5-f]chinolin-7-on
2-o-Monoethylphosphonomethylphenyl-8-cyan-4,5,6,7-tetrahydrothiazolo[4,5-f]chinolin-7-on
2-m-Monoethylphosphonomethylphenyl-8-cyan-4,5,6,7-tetrahydrothiazolo[4,5-f]chinolin-7-on
2-o-Monomethylphosphonomethylphenyl-8-cyan-6,7-dihydrothiazolo[4,5-f]chinolin-7-on
2-m-Monomethylphosphonomethylphenyl-8-cyan-6,7-dihydrothiazolo[4,5-f]chinolin-7-on
2-p-Monomethylphosphonomethylphenyl-8-cyan-6,7-dihydrothiazolo[4,5-f]chinolin-7-on
2-o-Monoethylphosphonomethylphenyl-8-cyan-6,7-dihydrothiazolo[4,5-f]chinolin-7-on
2-m-Monoethylphosphonomethylphenyl-8-cyan-6,7-dihydrothiazolo[4,5-f]chinolin-7-on
2-p-Monoethylphosphonomethylphenyl-8-cyan-6,7-dihydrothiazolo[4,5-f]chinolin-7-on.

Beispiel 52

Man suspendiert 1 g 2-o-Diethylphosphonomethylphenyl-8-cyan-4,5,6,7-tetrahydro-thiazolo[4,5-f]chinolin-7-on in 50 ml gesättigter ethanolischer HBr-Lösung, kocht 16 Std. unter Einleitung von HBr und dampft ein. Das erhaltene 2-o-Phosphonomethylphenyl-8-cyan-4,5,6,7-tetrahydro-thiazolo[4,5-f]chinolin-7-on wird mit Wasser gewaschen. F > 300 °.

Analog erhält man durch Verseifung der entsprechenden Diester oder Monoester:

3-Cyan-5-(2-o-phosphonomethylphenyl-4-thiazolyl)-6-methyl-2-pyridon
3-Cyan-5-(2-m-phosphonomethylphenyl-4-thiazolyl)-6-methyl-2-pyridon
3-Cyan-5-(2-p-phosphonomethylphenyl-4-thiazolyl)-6-methyl-2-pyridon
2-m-Phosphonomethylphenyl-8-cyan-4,5,6,7-tetrahydro-thiazolo[4,5-f]chinolin-7-on
2-p-Phosphonomethylphenyl-8-cyan-4,5,6,7-tetrahydro-thiazolo[4,5-f]chinolin-7-on
2-o-Phosphonomethylphenyl-8-cyan-6,7-dihydro-thiazolo-[4,5-f]chinolin-7-on
2-m-Phosphonomethylphenyl-8-cyan-6,7-dihydro-thiazolo-[4,5-f]chinolin-7-on
2-p-Phosphonomethylphenyl-8-cyan-6,7-dihydro-thiazolo-[4,5-f]chinolin-7-on.

Beispiel 53

Man löst 5,7 g Na in 60 ml Methanol, versetzt mit 2,22 g 3-Cyan-4-methylthio-5-acetyl-6-methyl-2-pyridon, kocht das Gemisch 1 Std. und dampft ein. Der Rückstand wird in Wasser gelöst. Man gibt Salzsäure bis pH 4 hinzu, wäscht mit Ethylacetat, gibt erneut Salzsäure bis pH 1 hinzu und filtriert ab. Man erhält 3-Cyan-4-hydroxy-5-acetyl-6-methyl-2-pyridon, F. 273 °.

Analog erhält man aus den entsprechenden 4-Methylthioverbindungen die folgenden 6-Methyl-2-pyridone:

3-Cyan-4-hydroxy-5-p-methoxyphenyl-, F. 281 °
3-Cyan-4-hydroxy-5-(3,4-dimethoxyphenyl)-, F. 289 °

3-Cyan-4-hydroxy-5-(4-pyridyl)-, F. > 310 °
3-Carbamoyl-4-hydroxy-5-acetyl-
3-Carbamoyl-4-hydroxy-5-p-methoxyphenyl-
3-Carbamoyl-4-hydroxy-5-(4-pyridyl)-.

Beispiel 54

Ein Gemisch von 1 g 3-Cyan-5-(2-acetamido-4-methyl-5-thiazolyl)-6-methyl-2-pyridon, 7 ml 2n NaOH und 30 ml Methanol wird 16 Std. gekocht und anschließend eingedampft. Man löst den Rückstand in Wasser und neutralisiert mit 1n HCl, wobei 3-Cyan-5-(2-amino-4-methyl-5-thiazolyl)-6-methyl-2-pyridon ausfällt. F. 286 - 290 ° (Zers.).

Analog erhält man durch Hydrolyse der entsprechenden Acetamidoverbindungen:

3-Cyan-5-(2-isopropylamino-4-methyl-5-thiazolyl)-6-methyl-2-pyridon
3-Cyan-5-(2-p-methoxyanilino-4-methyl-5-thiazolyl)-6-methyl-2-pyridon.

Beispiel 55

Man erhitzt im Autoklaven 10 g 3-Cyan-4-methylthio-5-acetyl-6-methyl-2-pyridon mit 500 ml 25 %iger wäßriger $NH_3$-Lösung 12 Std. auf 140 °, kühlt ab, konzentriert die Lösung und erhält 3-Cyan-4-amino-5-acetyl-6-methyl-2-pyridon, F. > 300 °.

Analog erhält man aus den entsprechenden 4-Methoxy- oder 4-Methylthioverbindungen mit Ammoniak bzw. den entsprechenden Aminen die folgenden 6-Methyl-2-pyridone:

3-Cyan-4-amino-5-p-methoxyphenyl-, F. 273 - 274 °
3-Cyan-4-amino-5-(2,4-dimethoxyphenyl)-, F. 234 - 235 °
3-Cyan-4-amino-5-(3,4-dimethoxyphenyl)-, F. 290 - 292 °
3-Cyan-4-amino-5-(4-pyridyl)-, F. > 355 °
3-Cyan-4-dimethylamino-5-acetyl-, F. 228 °
3-Cyan-4-dimethylamino-5-p-methoxyphenyl-, F. 266 - 267 °
3-Cyan-4-dimethylamino-5-(2,4-dimethoxyphenyl)-, F. 286 - 287 °
3-Cyan-4-dimethylamino-5-(3,4-dimethoxyphenyl)-, F. 322 - 323 °
3-Cyan-4-dimethylamino-5-(4-pyridyl)-, F. 335 - 337 °
3-Cyan-4-dimethylamino-5-(2-p-methoxyanilino-4-thiazolyl)-, F. > 290 °
3-Carbamoyl-4-amino-5-acetyl-
3-Carbamoyl-4-amino-5-methoxyphenyl-
3-Carbamoyl-4-amino-5-(2,4-dimethoxyphenyl)-
3-Carbamoyl-4-amino-5-(3,4-dimethoxyphenyl)-
3-Carbamoyl-4-amino-5-(4-pyridyl)-
3-Carbamoyl-4-dimethylamino-5-acetyl-
3-Carbamoyl-4-dimethylamino-5-p-methoxyphenyl-
3-Carbamoyl-4-dimethylamino-5-(2,4-dimethoxyphenyl)-
3-Carbamoyl-4-dimethylamino-5-(3,4-dimethoxyphenyl)-
3-Carbamoyl-4-dimethylamino-5-(4-pyridyl)-
3-Carbamoyl-4-dimethylamino-5-(2-p-methoxyanilino-4-thiazolyl)-.

Beispiel 56

Man erhitzt 2,86 g 3-Cyan-4-methylthio-5-p-methoxyphenyl-6-methyl-2-pyridon mit 10 ml p-Methoxyphenethylamin 24 Std. auf 120 °, kühlt ab und filtriert das erhaltene 3-Cyan-4-(2-p-methoxyphenylethylamino)-5-p-methoxyphenyl-6-methyl-2-pyridon ab. F. 219 °.

Analog erhält man aus den entsprechenden 4-Methylthioverbindungen und den entsprechenden Aminen die folgenden 6-Methyl-2-pyridone:

3-Cyan-4-pyrrolidino-5-acetyl-, F. 252 - 253 °
3-Cyan-4-(2-hydroxyethylamino)-5-p-methoxyphenyl-, F. 239 - 240 °
3-Cyan-4-(2-hydroxyethylamino)-5-(3,4-dimethoxyphenyl)-, F. 268 - 269 °
3-Cyan-4-(2-p-methoxyphenylethylamino)-, F. 225 - 228 °
3-Cyan-4-[2-(3,4-dimethoxyphenylethylamino)]-, F. 264 - 265 °
3-Cyan-4-[2-(3,4-dimethoxyphenylethylamino)]-5-(3,4-dimethoxyphenyl)-, F. 243 °
3-Carbamoyl-4-(2-p-methoxyphenylethylamino)-5-p-methoxyphenyl-
3-Carbamoyl-4-pyrrolidino-5-acetyl-
3-Carbamoyl-4-(2-hydroxyethylamino)-5-p-methoxyphenyl-
3-Carbamoyl-4-(2-hydroxyethylamino)-5-(3,4-dimethoxyphenyl)-
3-Carbamoyl-4-(2-p-methoxyphenylethylamino)-5-(3,4-dimethoxyphenyl)-
3-Carbamoyl-4-[2-(3,4-dimethoxyphenylethylamino)]-
3-Carbamoyl-4-[2-(3,4-dimethoxyphenylethylamino)-5-(3,4-dimethoxyphenyl)]-.

Beispiel 57

Man löst 5 g Na in 100 ml Methanol, versetzt mit 2,22 g 3-Cyan-4-methylthio-5-acetyl-6-methyl-2-pyridon, kocht 4 Std. und dampft ein. Nach Zugabe von Wasser und verdünnter Salzsäure bis pH 4 extrahiert man mit Ethylacetat und erhält 3-Cyan-4-methoxy-5-acetyl-6-methyl-2-pyridon, F. 214 - 216 ° (Zers.).

Analog erhält man aus den entsprechenden 4-Methylthioverbindungen folgende 6-Methyl-2-pyridone:

3-Cyan-4-ethoxy-5-acetyl-
3-Cyan-4-isobutoxy-5-acetyl-
3-Cyan-4-hexoxy-5-acetyl-
3-Cyan-4-methoxy-5-p-methoxyphenyl-
3-Carbamoyl-4-methoxy-5-acetyl-.

Beispiel 58

Man erhitzt 2,51 g 5-(2-Amino-4-thiazolyl)-6-methyl-2-pyridon-3-carbonsäure in 30 ml Chinolin in Gegenwart von 6,3 mg Cu-Pulver 3 Std. auf 205 - 210 °. Nach üblicher Aufarbeitung erhält man 5-(2-Amino-4-thiazolyl)-6-methyl-2-pyridon, F. 255 - 258° (Zers.).

Analog erhält man durch Decarboxylierung der entsprechenden Carbonsäure die nachfolgenden 6-Methyl-2-pyridone:

5-(2-p-Methoxyanilino-4-thiazolyl)-
5-(2-p-Diethylphosphonomethylphenyl-4-thiazolyl)-
5-(2-Benzylamino-4-thiazolyl)-
5-(2-p-Methoxybenzylamino-4-thiazolyl)-
5-(2-Dimethylamino-4-thiazolyl)-
5-[2-(N-Isopropyl-N-2-dimethylaminoethyl-amino)-4-thiazolyl]-
5-[2-(N-p-Methoxyphenyl-N-2-dimethylaminoethyl-amino)-4-thiazolyl]-
sowie
2-Dimethylamino-6,7-dihydro-thiazolo[4,5-f]chinolin-7-on.

Beispiel 59

Man kocht 2,55 g 3-Cyan-4-amino-5-p-methoxyphenyl-6-methyl-2-pyridon mit 30 ml 2n Natronlauge 1 Std., kühlt ab und filtriert das erhaltene 4-Amino-5-p-methoxyphenyl-6-methyl-2-pyridon ab. F. 299 - 300 °.

Analog erhält man durch Hydrolyse der entsprechenden 3-Cyan-Verbindungen:

4-Amino-5-(3,4-dimethoxyphenyl)-6-methyl-2-pyridon, F. 293 - 294 °
4-Amino-5-(4-pyridyl)-6-methyl-2-pyridon, F. > 355 °
5-(2-Amino-4-thiazolyl)-6-methyl-2-pyridon, F. 255 - 258 ° (Zers.)
2-Isopropylamino-4,5,6,7-tetrahydro-thiazolo[4,5-f]-chinolin-7-on, F. 264 - 267 °
2-Isopropylamino-6,7-dihydro-thiazolo[4,5-f]chinolin-7-on, F. 237 - 238 °.

Beispiel 60

Man erhitzt 1 g 3-Methyl-8-cyan-4,5,6,7-tetrahydro-isoxazolo[5,4-f]chinolin-7-on mit 20 ml DMF 24 Std. auf 100 - 110 ° und erhält nach üblicher Aufarbeitung 3-Methyl-8-cyan-6,7-dihydro-isoxazolo[5,4-f]chinolin-7-on, F. > 300 °.

Analog erhält man durch Dehydrierung der entsprechenden 4,5,6,7-Tetrahydroverbindungen die folgenden 8-Cyan-6,7-dihydrothiazolo[4,5-f]chinolin-7-one:

2-Methyl-
2-Ethoxycarbonylmethyl-
2-Carbamoylmethyl-

2-Cyanmethyl-
2-Phenyl-
2-p-Tolyl-
2-p-Ethylphenyl-
2-p-Methoxyphenyl-
2-p-Ethoxyphenyl-
2-p-Fluorphenyl-
2-p-Chlorphenyl-
2-p-Bromphenyl-
2-p-Jodphenyl-
2-m-Trifluormethylphenyl-
2-o-Hydroxyphenyl-
2-m-Hydroxyphenyl-
2-p-Hydroxyphenyl-
2-o-Nitrophenyl-
2-m-Nitrophenyl-
2-p-Nitrophenyl-
2-o-Aminophenyl-
2-m-Aminophenyl-
2-p-Aminophenyl-
2-p-Dimethylaminophenyl-
2-o-Dimethylphosphonomethylphenyl-
2-m-Dimethylphosphonomethylphenyl-
2-p-Dimethylphosphonomethylphenyl-
2-o-Diethylphosphonomethylphenyl-
2-m-Diethylphosphonomethylphenyl-
2-p-Diethylphosphonomethylphenyl-
2-(2-Pyridyl)-
2-(3-Pyridyl)-
2-(4-Pyridyl)-
2-(4-Methyl-3-pyridyl)-
2-Amino-, F. > 300 °
2-Methylamino-, F. > 300 °
2-Ethylamino-, F. > 300 °

2-Propylamino-
2-Isopropylamino-, F. > 300 °
2-Butylamino-
2-Isobutylamino-
2-sek.-Butylamino-
2-tert.-Butylamino-
2-Pentylamino-
2-Hexylamino-, F. 245 - 246 °
2-Allylamino-, F. 283 - 286 °
2-(2-Hydroxyethylamino)-, F. 273 ° (Zers.)
2-(3-Hydroxypropylamino)-, F. 278 - 280 °
2-(2-Dimethylaminoethylamino)-, F. 294 ° (Zers.)
2-(3-Dimethylaminopropylamino)-, F. 290 - 292 °
2-(2-Diethylaminoethylamino)-
2-(2-Pyrrolidinoethylamino)-
2-(2-Piperidinoethylamino)-, F. 267 - 268 °
2-[2-(2-Methylpiperidino)-ethylamino]-
2-[2-(2-Oxopyrrolidino)-ethylamino]-
2-(2-Morpholinoethylamino)-
2-(3-Morpholinopropylamino)-, F. 269 - 272 °
2-(2-Methoxyethylamino)-
2-Benzylamino-, F. 298 ° (Zers.)
2-p-Methoxybenzylamino-, F. 250 - 253 °
2-(2-Phenylethylamino)-
2-(2-p-Methoxyphenylethylamino)-
2-[2-(3,4-Dimethoxyphenyl)-ethylamino]-
2-(1-Indanylamino)-
2-[2-(2-Pyridyl)-ethylamino]-, F. 272 - 273 °
2-[2-(3-Pyridyl)-ethylamino]-
2-[2-(4-Pyridyl)-ethylamino]-, F. 243 - 245 °
2-(4-Piperidylamino)-
2-(1-Benzyl-4-piperidylamino)-, F. 243 - 246 °
2-(1-Ethoxycarbonyl-4-piperidylamino)-, F. > 300 °
2-Dimethylamino-, F. > 300 °

2-Diethylamino-
2-Dipropylamino-
2-Diisopropylamino-, F. 250 ° (Zers.)
2-Bis-(2-hydroxyethyl)-amino-, F. 264 - 267 °
2-(N-Methyl-N-2-dimethylaminoethyl-amino)-, F. 301-304°
2-(N-Ethyl-N-2-dimethylaminoethyl-amino)-, F. 240-242°
2-(N-Isopropyl-N-2-dimethylaminoethyl-amino)-, F. 215 - 218 °
2-(N-Benzyl-N-2-dimethylaminoethyl-amino)-, F. 267°
2-(N-p-Methoxybenzyl-N-2-dimethylaminoethyl-amino)-, F. 210-214°
2-[N-(3,4-Dimethoxybenzyl)-N-2-dimethylaminoethyl-amino]-
2-(N-2-p-Methoxyphenylethyl-N-2-dimethylaminoethyl-amino)-, F. 257-259°
2-[N-2-(3,4-Dimethoxyphenyl)-ethyl-N-2-dimethylaminoethyl-amino]-
2-(N-Phenyl-N-2-dimethylaminoethyl-amino)-
2-(N-p-Methoxyphenyl-N-2-dimethylaminoethyl-amino)-, F. 242 - 245 °
2-(N-Methyl-N-2-diethylaminoethyl-amino)-
2-(N-Ethyl-N-2-diethylaminoethyl-amino)-, F. > 300°
2-(N-Isopropyl-N-2-diethylaminoethyl-amino)-
2-(N-Benzyl-N-2-diethylaminoethyl-amino)-
2-(N-p-Methoxybenzyl-N-2-diethylaminoethyl-amino)-
2-[N-(3,4-Dimethoxybenzyl)-N-2-diethylaminoethyl-amino]-
2-(N-2-p-Methoxyphenylethyl-N-2-diethylaminoethyl-amino)-, F. 240° (Zers.)
2-[N-2-(3,4-Dimethoxyphenyl)-ethyl-N-2-diethylaminoethyl-amino]-, F. 204°
2-(N-Phenyl-N-2-diethylaminoethyl-amino)-
2-(N-p-Methoxyphenyl-N-2-diethylaminoethyl-amino)-
2-(N-Methyl-N-2-methoxyethyl-amino)-
2-(N-Ethyl-N-2-methoxyethyl-amino)-
2-(N-Isopropyl-N-2-methoxyethyl-amino)-
2-(N-Benzyl-N-2-methoxyethyl-amino)-
2-(N-p-Methoxybenzyl-N-2-methoxyethyl-amino)-

2-[N-(3,4-Dimethoxybenzyl)-N-2-methoxyethyl-amino]-
2-(N-2-p-Methoxyphenylethyl-N-2-methoxyethyl-amino)-
2-[N-2-(3,4-Dimethoxyphenyl)-ethyl-N-2-methoxyethyl-amino]-
2-(N-Phenyl-N-2-methoxyethyl-amino)-
2-(N-p-Methoxyphenyl-N-2-methoxyethyl-amino)-
2-Pyrrolidino-
2-Piperidino-
2-Morpholino-, F. > 300°
2-(4-Methylpiperidino)-
2-(4-Methylpiperazino)-
2-[4-(2-Hydroxyethyl)-piperazino]-, F. 273 - 274 °
2-[4-(3-Hydroxypropyl)-piperazino]-
2-[4-(2,3-Dihydroxypropyl)-piperazino]-, F. 265 - 267 °
2-[4-(N-Isopropylcarbamoylmethyl)-piperazino]-, F. > 300 °
2-(4-o-Methoxyphenyl-piperazino)-
2-(4-m-Methoxyphenyl-piperazino)-
2-(4-p-Methoxyphenyl-piperazino)-, F. 332 °
2-(4-Furoyl-piperazino)-
2-Anilino-
2-p-Methylanilino-
2-o-Fluoranilino-
2-m-Fluoranilino-
2-p-Fluoranilino-
2-o-Chloranilino-
2-m-Chloranilino-, F. > 300 °
2-p-Chloranilino-
2-o-Bromanilino-
2-m-Bromanilino-
2-p-Bromanilino-
2-p-Jodanilino-
2-p-Trifluormethylanilino
2-o-Hydroxyanilino-
2-m-Hydroxyanilino-
2-p-Hydroxyanilino-

2-o-Methoxyanilino-
2-m-Methoxyanilino-
2-p-Methoxyanilino-, F. > 300 °
2-o-Nitroanilino-
2-m-Nitroanilino-
2-p-Nitroanilino-
2-o-Methylthioanilino-
2-m-Methylthioanilino-
2-p-Methylthioanilino-
2-o-Methylsulfinylanilino-
2-m-Methylsulfinylanilino-
2-p-Methylsulfinylanilino-
2-(2,4-Dimethoxyanilino)-, F. > 300 °
2-(3,4-Dimethoxyanilino)-
2-(2-Pyridylanilino)-
2-(3-Pyridylanilino)-
2-(4-Pyridylanilino)-
2-(2-Methoxy-5-pyridylanilino)-, F. > 300 °
2-[5-(4-Pyridyl)-2-pyridylamino]-;

ferner 3-Methyl-8-carbamoyl-6,7-dihydro-isoxazolo[5,4-f]-chinolin-7-on

sowie die folgenden 8-Carbamoyl-6,7-dihydro-thiazolo-[4,5-f]chinolin-7-one:

2-Amino-
2-Methylamino-
2-Ethylamino-
2-Isopropylamino-
2-Hexylamino-
2-Allylamino-
2-(2-Hydroxyethylamino)-
2-(3-Hydroxypropylamino)-

2-(2-Dimethylaminoethylamino)-
2-(3-Dimethylaminopropylamino)-
2-(2-Piperidinoethylamino)-
2-(3-Morpholinopropylamino)-
2-Benzylamino-
2-p-Methoxybenzylamino-
2-[2-(2-Pyridyl)-ethylamino]-
2-[2-(3-Pyridyl)-ethylamino]-
2-[2-(4-Pyridyl)-ethylamino]-
2-(1-Benzyl-4-piperidylamino)-
2-(1-Ethoxycarbonyl-4-piperidylamino)-
2-Dimethylamino-
2-Diisopropylamino-
2-Bis-(2-hydroxyethyl)-amino-
2-[4-(2-Hydroxyethyl)-piperazino]-
2-[4-(2,3-Dihydroxypropyl)-piperazino]-
2-[4-(N-Isopropylcarbamoylmethyl)-piperazino]-
2-(4-p-Methoxyphenyl-piperazino)-
2-m-Chloranilino-
2-p-Methoxyanilino-
2-(2,4-Dimethoxyanilino)-
2-(2-Methoxy-5-pyridylamino)-;

ferner die folgenden 8-Cyan-6,7-dihydro-pyrazolo[3,4-f]-chinolin-7-one:

1H-1-Methyl-, F. > 300 °
1H-1,3-Dimethyl-, F. > 300 °
1H-1-Methyl-3-(4-pyridyl)-, F. > 300 °
1H-1-Phenyl-3-methyl-, F. > 300 °
1H-1-p-Methoxyphenyl-3-methyl-, F. > 300 °;

ferner die folgenden 8-Carbamoyl-6,7-dihydro-pyrazolo-[3,4-f]chinolin-7-one:

1H-1-Methyl-
1H-1,3-Dimethyl-
1H-1-Methyl-3-(4-pyridyl)-
1H-1-Phenyl-3-methyl-
1H-1-p-Methoxyphenyl-3-methyl-.

Beispiel 61

Zu einer Suspension von 2,22 g 3-Cyan-4-methylthio-5-acetyl-6-methyl-2-pyridon in 100 ml Chloroform gibt man 3,05 g m-Chlorperbenzoesäure unter Rühren und kocht das Gemisch über Nacht. Der erhaltene Niederschlag von 3-Cyan-4-methylsulfinyl-5-acetyl-6-methyl-2-pyridon wird abfiltriert und mit Methanol ausgekocht; F. 276 ° (Zers.).

Analog erhält man durch Oxydation der entsprechenden Thioether:

3-Cyan-5-(2-methylsulfinyl-4-thiazolyl)-6-methyl-2-pyridon
3-Carbamoyl-4-methylsulfinyl-5-acetyl-6-methyl-2-pyridon
3-Carbamoyl-5-(2-methylsulfinyl-4-thiazolyl)-6-methyl-2-pyridon.

Beispiel 62

Eine Lösung von 2,63 g 3-Cyan-5-(2-methylthio-4-thiazolyl)-6-methyl-2-pyridon in 80 ml Essigsäure wird portionsweise unter Rühren bei 50 ° mit 6,16 g m-Chlorperbenzoesäure versetzt. Man rührt noch 2,5 Std. bei 50 - 55 °, saugt ab, wäscht mit $NaHCO_3$-Lösung und mit Wasser und kristallisiert das erhaltene 3-Cyan-5-(2-methylsulfonyl-4-thiazolyl)-6-methyl-2-pyridon aus DMF/Wasser um; F. 292 - 300 °.

Analog erhält man durch Oxydation der entsprechenden Thioether:

3-Cyan-4-methylsulfonyl-5-acetyl-6-methyl-2-pyridon
3-Carbamoyl-5-(2-methylsulfonyl-4-thiazolyl)-6-methyl-2-pyridon
3-Carbamoyl-4-methylsulfonyl-5-acetyl-6-methyl-2-pyridon.

Beispiel 63

Ein Gemisch von 2,07 g 2,5-Dioxo-1,2,5,6,7,8-hexahydrochinolin-3-carbonsäure, 1,8 g $SOCl_2$ und 75 ml DMF wird 3 Std. bei 40 ° gerührt, abgekühlt, mit 100 ml Methanol versetzt und erneut 2 Std. bei 20 ° gerührt. Man konzentriert, gibt Wasser zu und erhält 2,5-Dioxo-1,2,5,6,7,8-hexahydrochinolin-3-carbonsäuremethylester.

Analog erhält man durch Veresterung:

2,5-Dioxo-1,2,5,6,7,8-hexahydrochinolin-3-carbonsäureethylester
2,5-Dioxo-1,2,5,6,7,8-hexahydrochinolin-3-carbonsäurehexylester
2,5-Dioxo-1,2,5,6,7,8-hexahydrochinolin-3-carbonsäurebenzylester.

Beispiel 64

Man kocht ein Gemisch von 4,06 g 3-Cyan-5-[2-(4-pyridylamino)-4-thiazolyl]-6-methyl-2-pyridon-N-oxid-hydrobromid, 2,7 g Eisenpulver, 300 ml Essigsäure und 100 ml Wasser 1 Std., filtriert heiß, konzentriert das Filtrat, verrührt den Niederschlag mit verdünnter Natronlauge, filtriert und kristallisiert das erhaltene 3-Cyan-5-[2-(4-pyridylamino)-4-thiazolyl]-6-methyl-2-pyridon aus DMF; F. > 300 °.

Analog erhält man 3-Carbamoyl-5-[2-(4-pyridylamino)-4-thiazolyl]-6-methyl-2-pyridon.

Beispiel 65

Man löst 2,32 g 3-Cyan-5-(2-amino-4-thiazolyl)-6-methyl-2-pyridon in 50 ml DMF, versetzt mit 2,94 g Dimethylformamid-diethylacetal und rührt 2 Std. bei 140 - 150 °. Beim Abkühlen fällt 2-Dimethylaminomethylamino-8-cyan-6,7-dihydro-thiazolo[4,5-f]chinolin-7-on aus; F. > 300 °. Diese Verbindung wird abfiltriert und das Filtrat mit 250 ml Wasser versetzt. Das ausgefallene Gemisch wird ebenfalls abfiltriert, das Filtrat eingedampft und der Rückstand mit methanolischem HCl behandelt. Man erhält 3-Cyan-5-(2-dimethylaminomethylenamino-4-thiazolyl)-6-methyl-2-pyridon-hydrochlorid, F. 258 - 260 °.

Analog erhält man

2-Dimethylaminomethylenamino-8-carbamoyl-6,7-dihydro-thiazolo[4,5-f]chinolin-7-on
3-Carbamoyl-5-(2-dimethylaminomethylenamino-4-thiazolyl)-6-methyl-2-pyridon.

Beispiel 66

Man kocht ein Gemisch von 2,6 g 3-Cyan-5-(2-dimethylamino-4-thiazolyl)-6-methyl-2-pyridon, 55 ml Essigsäure, 1,5 ml Acetanhydrid und 2,3 g Orthoameisensäuretriethylester 8 Std., konzentriert, gießt in Wasser und erhält 2-Dimethylamino-8-cyan-6,7-dihydrothiazolo[4,5-f]chinolin-7-on, F. > 300 °.

Beispiel 67

Eine Lösung von 1,88 g 3-Cyan-1,2,5,6,7,8-hexahydrochinolin-2,5-dion und 2,2 g Dimethylformamid-diethylacetal in 40 ml DMF wird 5 Std. bei 70 ° gerührt. Man filtriert heiß und wäscht das so erhaltene 3-Cyan-6-dimethylaminomethylen-1,2,5,6,7,8-hexahydrochinolin-2,5-dion mit Ethanol; Zers. ab 300 °.

Analog erhält man 3-Carbamoyl-6-dimethylaminomethylen-1,2,5,6,7,8-hexahydrochinolin-2,5-dion.

Beispiel 68

Ein Gemisch von 3,03 g 3-Cyan-4-(2-ethoxycarbonylmethyl-4-thiazolyl)-6-methyl-2-pyridon, 1,36 g Anisaldehyd, 20 ml Essigsäure und 20 ml Acetanhydrid wird 2 Std. gekocht, abgekühlt und auf Eis gegossen. Man erhält 3-Cyan-4-[2-(1-ethoxycarbonyl-2-p-methoxyphenyl-vinyl)-4-thiazolyl]-6-methyl-2-pyridon, F. 239 - 241 °.

Analog sind erhältlich:
3-Cyan-4-[2-(1-ethoxycarbonyl-2-p-dimethylaminophenyl-vinyl)-4-thiazolyl]-6-methyl-2-pyridon
3-Carbamoyl-4-[2-(1-ethoxycarbonyl-2-p-methoxyphenyl-vinyl)-4-thiazolyl]-6-methyl-2-pyridon.

Beispiel 69

Unter Eiskühlung werden bei 4 ° 2 g Brom zu einer Lösung von 2,55 g NaOH in 21 ml Wasser gegeben. Anschließend

gibt man portionsweise 3,56 g 3-Carbamoyl-5-(2-p-methoxyanilino-4-thiazolyl)-6-methyl-2-pyridon hinzu, rührt 17 Std. bei 4 ° und erwärmt 30 Min. auf 60 °. Es wird abgekühlt und filtriert. Man versetzt das Filtrat mit Salzsäure bis pH 5, filtriert, wäscht mit Wasser, behandelt das Produkt 16 Std. bei 100 ° mit verdünnter Natronlauge, kühlt ab, filtriert und wäscht erneut mit Wasser. Man erhält 3-Amino-5-(2-p-methoxyanilino-4-thiazolyl)-6-methyl-2-pyridon.

Analog erhält man die nachstehenden 6-Methyl-2-pyridone:

3-Amino-5-(2-p-diethylphosphonomethylphenyl-4-thiazolyl)-
3-Amino-5-(2-benzylamino-4-thiazolyl)-
3-Amino-5-(2-p-methoxybenzylamino-4-thiazolyl)-
3-Amino-5-(2-dimethylamino-4-thiazolyl)-
3-Amino-5-[2-(N-2-dimethylaminoethyl-N-isopropyl-amino)-4-thiazolyl]-
3-Amino-5-[2-(N-2-dimethylaminoethyl-p-methoxyanilino)-4-thiazolyl]-

sowie

2-Dimethylamino-8-amino-6,7-dihydro-thiazolo[4,5-f]-chinolin-7-on.

Beispiel 70

Analog Beispiel 2 erhält man aus 2-(1-Dimethylaminomethylen-2-oxopropyl)-4-p-methoxyphenylthiazol (erhältlich durch Reaktion von 4-p-Methoxyphenylthiazol-2-essigsäure mit Acetanhydrid/Pyridin, nachfolgende Ketonspaltung und Umsetzung mit Dimethylformamiddiethylacetal) das 3-Cyan-5-(4-p-methoxyphenyl-2-thiazolyl)-6-methyl-2-pyridon, F. 317-319°.

Beispiel 71

Analog Beispiel 21 erhält man mit den entsprechenden Thioharnstoffen die folgenden 6-Methyl-2-pyridone:

3-Cyan-5-(2-o-methoxybenzylamino-4-thiazolyl)-
3-Cyan-5-(2-m-methoxybenzylamino-4-thiazolyl)-
3-Cyan-5-(2-p-methoxybenzylamino-4-thiazolyl)-
3-Cyan-5-(2-N-methyl-N-o-methoxybenzylamino-4-thiazolyl)-
3-Cyan-5-(2-N-methyl-N-m-methoxybenzylamino-4-thiazolyl)-
3-Cyan-5-(2-N-methyl-N-p-methoxybenzylamino-4-thiazolyl)-, F. 210-213°
3-Cyan-5-(2-N-ethyl-N-o-methoxybenzylamino-4-thiazolyl)-
3-Cyan-5-(2-N-ethyl-N-m-methoxybenzylamino-4-thiazolyl)-
3-Cyan-5-(2-N-ethyl-N-p-methoxybenzylamino-4-thiazolyl)-, F. 190-192°

3-Cyan-5-[2-(2-p-methoxyphenylethyl-amino)-4-thiazolyl]-
3-Cyan-5-[2-(N-methyl-N-(2-p-methoxyphenylethyl)-amino)-4-thiazolyl]-, F. 236-239°
3-Cyan-5-[2-(N-ethyl-N-(2-p-methoxyphenylethyl)-amino)-4-thiazolyl]-
3-Cyan-5-[2-(2-(3,4-dimethoxyphenyl)-ethyl-amino)-4-thiazolyl]-
3-Cyan-5-[2-(N-methyl-N-2-(3,4-dimethoxyphenyl)-ethyl-amino)-4-thiazolyl]-

3-Cyan-5-[2-(N-ethyl-N-2-(3,4-dimethoxyphenyl)-ethyl-amino)-4-thiazolyl]-

3-Cyan-5-[2-(2-pyridylmethyl-amino)-4-thiazolyl]-, Hydrobromid, F. 253°

3-Cyan-5-[2-(N-methyl-N-2-pyridylmethyl-amino)-4-thiazolyl]-

3-Cyan-5-[2-(N-ethyl-N-2-pyridylmethyl-amino)-4-thiazolyl]-

3-Cyan-5-[2-(3-pyridylmethyl-amino)-4-thiazolyl]-, F. 184°

3-Cyan-5-[2-(N-methyl-N-3-pyridylmethyl-amino)-4-thiazolyl]-, F. 230-233°

3-Cyan-5-[2-(N-ethyl-N-3-pyridylmethyl-amino)-4-thiazolyl]-

3-Cyan-5-[2-(4-pyridylmethyl-amino)-4-thiazolyl]-, Hydrobromid, F. 256-260°

3-Cyan-5-[2-(N-methyl-N-4-pyridylmethyl-amino)-4-thiazolyl]-

3-Cyan-5-[2-(N-ethyl-N-4-pyridylmethyl-amino)-4-thiazolyl]-

3-Cyan-5-[2-(2-(2-pyridyl)-ethylamino)-4-thiazolyl]-

3-Cyan-5-[2-(N-methyl-N-2-(2-pyridyl)-ethyl-amino)-4-thiazolyl]-, F. 215-218°

3-Cyan-5-[2-(N-ethyl-N-2-(2-pyridyl)-ethyl-amino-4-thiazolyl]-

3-Cyan-5-[2-(2-(3-pyridyl)-ethylamino)-4-thiazolyl]-

3-Cyan-5-[2-(N-methyl-N-2-(3-pyridyl)-ethyl-amino)-4-thiazolyl]-

3-Cyan-5-[2-(N-ethyl-N-2-(3-pyridyl)-ethyl-amino)-4-thiazolyl]-

3-Cyan-5-[2-(2-(4-pyridyl)-ethylamino)-4-thiazolyl]-

3-Cyan-5-[2-(N-methyl-N-2-(4-pyridyl)-ethyl-amino)-4-thiazolyl]-

3-Cyan-5-[2-(N-ethyl-N-2-(4-pyridyl)-ethyl-amino)-4-thiazolyl]-

3-Cyan-5-[2-(p-4-pyridylmethyl-anilino)-4-thiazolyl]-
3-Cyan-5-[2-(N-methyl-N-2-morpholinoethyl-amino)-4-thiazolyl]-
3-Cyan-5-[2-(N-ethyl-N-2-morpholinoethyl-amino)-4-thiazolyl]-
3-Cyan-5-[2-(N-benzyl-N-2-morpholinoethyl-amino)-4-thiazolyl]-
3-Cyan-5-[2-(N-p-methoxybenzyl-N-2-morpholinoethyl-amino)-4-thiazolyl]-
3-Cyan-5-[2-(N-2-p-methoxyphenyl-ethyl-N-2-morpholinoethyl-amino)-4-thiazolyl]-, F. 192-196°
3-Cyan-5-[2-(N-2-(3,4-dimethoxyphenyl)-ethyl-N-2-morpholinoethyl-amino)-4-thiazolyl]-, F. 199-202°

Beispiel 72

Analog Beispiel 24 erhält man mit den entsprechenden Thioharnstoffen die folgenden 8-Cyan-4,5,6,7-tetrahydro-thiazolo[4,5-f]chinolin-7-one:

2-[N-Methyl-N-(2-morpholinoethyl)-amino]-, F. 240°
2-[N-p-Methoxybenzyl-N-(2-morpholinoethyl)-amino]-, F. 240° (Zers.)
2-[N-(2-p-Methoxyphenylethyl)-N-(2-morpholinoethyl)-amino]-, F. 265° (Zers.)
2-[N-Methyl-N-(2-(2-pyridyl)-ethyl)-amino]-, F. 250-253°.

Beispiel 73

Analog Beispiel 25 erhält man mit den entsprechenden Thioharnstoffen die nachstehenden 6-Methyl-2-pyridone:

3-Cyan-5-(2-p-carboxymethylanilino-4-thiazolyl)-, F. 250° (Zers.)

3-Cyan-5-(2-p-ethoxycarbonylmethylanilino-4-thiazolyl)-, F. 290-293° (Zers.)
3-Cyan-5-[2-p-(4-pyridylmethyl)-anilino-4-thiazolyl]-, F. 236-238°
3-Cyan-5-[2-(5-methyl-2-pyridyl-amino)-4-thiazolyl]-, F. > 300°.

Beispiel 74

Analog Beispiel 43 erhält man aus den entsprechenden Aminen durch Acylierung die nachstehenden 6-Methyl-2-pyridone:

3-Cyan-5-(2-N-p-methoxybenzyl-propionamido-4-thiazolyl)-, F. 233-236°
3-Cyan-5-[2-N-(2-(4-pyridyl)-ethyl)-propionamido-4-thiazolyl]-, F. > 300°
3-Cyan-5-(2-N-p-methoxybenzyl-trimethylacetamido-4-thiazolyl)-, F. 220-222°
3-Cyan-5-[2-N-(2-piperidinoethyl)-trimethylacetamido-4-thiazolyl]-, F. 243-244°
3-Cyan-5-(2-N-p-methoxybenzyl-N-methoxycarbonyl-amino-4-thiazolyl)-, F. 231-234°.

Beispiel 75

Umsetzung von 3-Cyan-5-(2-p-methoxybenzylamino-4-thiazolyl)-6-methyl-2-pyridon mit p-Methoxybenzoylchlorid analog Beispiel 44 liefert 2-p-Methoxybenzoyloxy-3-cyan-5-(2-p-methoxybenzylamino-4-thiazolyl)-6-methylpyridin, Harz.

Analog erhält man mit Chlorameisensäureethylester 2-Ethoxycarbonyloxy-3-cyan-5-(2-N-p-methoxybenzyl-N-ethoxycarbonyl-amino)-6-methylpyridin, F. 101-103°.

Beispiel 76

Analog Beispiel 49 erhält man aus dem entsprechenden Methyl- oder Ethylester durch Verseifung 3-Cyan-5-(2-p-carboxyphenyl-4-thiazolyl)-6-methyl-2-pyridon, F. > 300°.

Beispiel 77

Analog Beispiel 60 erhält man durch Dehydrierung die folgenden 8-Cyan-6,7-dihydro-thiazolo[4,5-f]chinolin-7-one:

2-[N-Methyl-N-(2-morpholinoethyl)-amino]-, F. 235°
2-[N-p-Methoxybenzyl-N-(2-morpholinoethyl)-amino]-, F. 247-249°
2-[N-(2-p-Methoxyphenylethyl)-N-(2-morpholinoethyl)-amino]-, F. 243°
2-[N-Methyl-N-(2-(2-pyridyl)-ethyl)-amino]-, F. 253°.

Beispiel 78

Analog Beispiel 16 erhält man mit den entsprechenden Thioamiden die nachstehenden 6-Methyl-2-pyridone:

3-Cyan-5-(2-methoxymethyl-4-thiazolyl)-, F. 258-259°
3-Cyan-5-[2-(2-methoxyethyl)-4-thiazolyl]-, F. 191-193°
3-Cyan-5-(2-o-methoxybenzyl-4-thiazolyl)-, F. 238-241° (Zers.)
3-Cyan-5-(2-p-methoxybenzyl-4-thiazolyl)-, F. 232-235° (Zers.)
3-Cyan-5-(2-phenoxymethyl-4-thiazolyl)-, F. 244°.

Beispiel 79

Analog Beispiel 17 erhält man mit den entsprechenden Thioamiden die nachstehenden 6-Methyl-2-pyridone:

3-Cyan-5-[2-p-(N-2-dimethylaminoethyl-carbamoyl)-phenyl-4-thiazolyl]-, F. 282-283°
3-Cyan-5-[2-p-(N-methyl-N-2-dimethylaminoethyl-carbamoyl)-phenyl-4-thiazolyl]-, F. 205-210°.

Beispiel 80

Analog Beispiel 16 erhält man aus Thiophenoxyacetamid und 3-Cyan-5-(2-brom-3-ethoxy-1,3-dioxopropyl)-6-methyl-2-pyridon [F. 35-40° (Zers.); erhältlich durch Bromierung von 3-Cyan-5-ethoxycarbonylacetyl-6-methyl-2-pyridon] 3-Cyan-5-(2-phenoxymethyl-5-ethoxycarbonyl-4-thiazolyl)-6-methyl-2-pyridon, F. 223-224°.

Analog sind mit den entsprechenden Thioamiden bzw. Thioharnstoffen die folgenden 6-Methyl-2-pyridone erhältlich:

3-Cyan-5-(2-p-carbamoylphenyl-5-ethoxycarbonyl-4-thiazolyl)-, F. 324° (Zers.)
3-Cyan-5-(2-p-ethoxycarbonylphenyl-5-ethoxycarbonyl-4-thiazolyl)-, F. 312°
3-Cyan-5-[2-(2-methoxyethylamino)-5-ethoxycarbonyl-4-thiazolyl]-, F. 220-221°
3-Cyan-5-(2-o-methoxybenzylamino-5-ethoxycarbonyl-4-thiazolyl)-
3-Cyan-5-(2-m-methoxybenzylamino-5-ethoxycarbonyl-4-thiazolyl)-
3-Cyan-5-(2-p-methoxybenzylamino-5-ethoxycarbonyl-4-thiazolyl)-, F. 235-237°
3-Cyan-5-(2-N-methyl-N-o-methoxybenzyl-amino-5-ethoxycarbonyl-4-thiazolyl)-
3-Cyan-5-(2-N-methyl-N-m-methoxybenzyl-amino-5-ethoxycarbonyl-4-thiazolyl)-
3-Cyan-5-(2-N-methyl-N-p-methoxybenzyl-amino-5-ethoxycarbonyl-4-thiazolyl)-, F. 240-242°

3-Cyan-5-(2-N-ethyl-N-o-methoxybenzyl-amino-5-ethoxycarbonyl-4-thiazolyl)-

3-Cyan-5-(2-N-ethyl-N-m-methoxybenzyl-amino-5-ethoxycarbonyl-4-thiazolyl)-

3-Cyan-5-(2-N-ethyl-N-p-methoxybenzyl-amino-5-ethoxycarbonyl-4-thiazolyl)-, F. 201-203°

3-Cyan-5-[2-(2-p-methoxyphenylethyl-amino)-5-ethoxycarbonyl-4-thiazolyl]-

3-Cyan-5-[2-(N-methyl-N-(2-p-methoxyphenylethyl)-amino-5-ethoxycarbonyl-4-thiazolyl]-

3-Cyan-5-[2-(N-ethyl-N-(2-p-methoxyphenylethyl)-amino-5-ethoxycarbonyl-4-thiazolyl]-

3-Cyan-5-[2-(2-(3,4-dimethoxyphenyl)-ethyl-amino)-5-ethoxycarbonyl-4-thiazolyl]-

3-Cyan-5-[2-(N-methyl-N-2-(3,4-dimethoxyphenyl)-ethyl-amino)-5-ethoxycarbonyl-4-thiazolyl]-

3-Cyan-5-[2-(N-ethyl-N-2-(3,4-dimethoxyphenyl)-ethyl-amino)-5-ethoxycarbonyl-4-thiazolyl]-

3-Cyan-5-[2-(2-pyridylmethylamino)-5-ethoxycarbonyl-4-thiazolyl]-, Hydrobromid, F. 230° (Zers.)

3-Cyan-5-[2-(N-methyl-N-2-pyridylmethylamino)-5-ethoxycarbonyl-4-thiazolyl]-

3-Cyan-5-[2-(N-ethyl-N-2-pyridylmethylamino)-5-ethoxycarbonyl-4-thiazolyl]-

3-Cyan-5-[2-(3-pyridylmethylamino)-5-ethoxycarbonyl-4-thiazolyl]-, Hydrobromid, F. 182° (Zers.)

3-Cyan-5-[2-(N-methyl-N-3-pyridylmethylamino)-5-ethoxycarbonyl-4-thiazolyl]-, Hydrobromid, F. 242-243° (Zers.)

3-Cyan-5-[2-(N-ethyl-N-3-pyridylmethylamino)-5-ethoxycarbonyl-4-thiazolyl]-

3-Cyan-5-[2-(4-pyridylmethylamino)-5-ethoxycarbonyl-4-thiazolyl]-, Hydrobromid, F. 192° (Zers.)

3-Cyan-5-[2-(N-methyl-N-4-pyridylmethyl-amino)-5-ethoxycarbonyl-4-thiazolyl]-

3-Cyan-5-[2-(N-ethyl-N-4-pyridylmethyl-amino)-5-ethoxycarbonyl-4-thiazolyl]-

3-Cyan-5-[2-(2-(2-pyridyl)-ethyl-amino)-5-ethoxycarbonyl-4-thiazolyl]-

3-Cyan-5-[2-(N-methyl-N-2-(2-pyridyl)-ethyl-amino)-5-ethoxycarbonyl-4-thiazolyl]- Hydrobromid, F. 186° (Zers.)

3-Cyan-5-[2-(N-ethyl-N-2-(2-pyridyl)-ethyl-amino)-5-ethoxycarbonyl-4-thiazolyl]-

3-Cyan-5-[2-(2-(3-pyridyl)-ethylamino)-5-ethoxycarbonyl-4-thiazolyl]-

3-Cyan-5-[2-(N-methyl-N-2-(3-pyridyl)-ethyl-amino)-5-ethoxycarbonyl-4-thiazolyl]-

3-Cyan-5-[2-(N-ethyl-N-2-(3-pyridyl)-ethyl-amino)-5-ethoxycarbonyl-4-thiazolyl]-

3-Cyan-5-[2-(2-(4-pyridyl)-ethylamino)-5-ethoxycarbonyl-4-thiazolyl]-

3-Cyan-5-[2-(N-methyl-N-2-(4-pyridyl)-ethyl-amino)-5-ethoxycarbonyl-4-thiazolyl]-

3-Cyan-5-[2-(N-ethyl-N-2-(4-pyridyl)-ethyl-amino)-5-ethoxycarbonyl-4-thiazolyl]-

3-Cyan-5-[2-(p-4-pyridylmethyl-anilino)-5-ethoxycarbonyl-4-thiazolyl]-, Hydrobromid, F. 253-256°

3-Cyan-5-[2-(N-methyl-N-2-morpholinoethyl-amino)-5-ethoxycarbonyl-4-thiazolyl]-

3-Cyan-5-[2-(N-ethyl-N-2-morpholinoethyl-amino)-5-ethoxycarbonyl-4-thiazolyl]-, Hydrobromid, F. 200° (Zers.)

3-Cyan-5-[2-(N-benzyl-N-2-morpholinoethyl-amino)-5-ethoxycarbonyl-4-thiazolyl]-, Hydrobromid, F. 121° (Zers.)

3-Cyan-5-[2-(N-p-methoxybenzyl-N-2-morpholinoethyl-amino)-5-ethoxycarbonyl-4-thiazolyl]-, Hydrobromid, F. 160° (Zers.)

3-Cyan-5-[2-(N-2-p-methoxyphenyl-ethyl-N-2-morpholinoethyl-amino)-5-ethoxycarbonyl-4-thiazolyl]-, Hydrobromid, F. 145° (Zers.)

3-Cyan-5-[2-(N-2-(3,4-dimethoxyphenyl)-ethyl-N-(2-morpholinoethyl-amino)-5-ethoxycarbonyl-4-thiazolyl]-, F. 95°.

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1 kg 3-Cyan-5-(2-p-diethylphosphonomethylphenyl-4-thiazolyl)-6-methyl-2-pyridon, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 50 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 10 kg 3-Cyan-5-(8-carbamoyl-imidazo[1,2-a]-pyridin-2-yl)-6-methyl-2-pyridon in üblicher Weise in Hartgelatinekapseln, so daß jede Kapsel 50 mg Wirkstoff enthält.

Beispiel D: Ampullen

Eine Lösung von 1 kg 3-Cyan-5-[2-(N-ethyl-N-2-dimethylaminoethyl-amino)-4-thiazolyl]-6-methyl-2-pyridon in 30 l 1,2-Propandiol wird steril filtriert, in Ampullen abgefüllt und steril verschlossen. Jede Ampulle enthält 20 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

Merck Patent Gesellschaft
mit beschränkter Haftung
D a r m s t a d t

Patentansprüche

1. Pyridone der allgemeinen Formel I

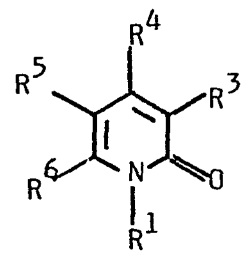

I

worin

$R^1$ H, A oder Benzyl,

$R^3$ H, CN, $CONH_2$, COOH, COOA, $COOCH_2C_6H_5$ oder $NH_2$,

$R^4$ H, A, $A_2N$-CH=CH-, Ar-alkyl, OH, OA, SA, SO-A, $SO_2$-A oder $NR^7R^8$,

$R^5$ H, $R^9$-$CHR^{10}$-CO-, $A_2N$-CH=$CR^{10}$-CO-, Ar, Py, unsubstituiertes oder ein- oder mehrfach durch $R^{10}$ und/oder $R^{11}$ substituiertes Thiazolyl, Imidazolyl, Pyrazolyl, Isoxazolyl oder Imidazopyridyl,

$R^6$ H, A, COOA oder zusammen mit $R^{10}$ eine $-CH_2CH_2-$ oder -CH=CH-Gruppe,

A Alkyl,

Ar Phenyl oder ein- bis dreifach durch A, OA, F, Cl, Br, J, $CF_3$, OH, $NO_2$, $NH_2$, ANH, $A_2N$, CN, $CONR^7R^8$, COOH, COOA, $CH_2CONR^7R^8$, $CH_2COOH$, $CH_2COOA$, $OCH_2COOH$, $OCH_2COOA$, SA, SO-A, $SO_2A$, Phosphonomethyl, Alkylphosphonomethyl, Dialkylphosphonomethyl, AO-alkyl, AS-alkyl, Ar', Ar'O, Ar'-thioalkyl, Hydroxyalkyl und/oder Imidazolyl substituiertes Phenyl,

| | |
|---|---|
| Ar' | Phenyl oder Fluorphenyl, |
| Py | 2-, 3- oder 4-Pyridyl oder einfach durch A, OA, F, Cl, Br, J, $NO_2$, CN, $CONH_2$, COOH, N-Oxidosauerstoff oder Pyridyl substituiertes 2-, 3- oder 4-Pyridyl, |
| $R^7$ | H, A, Alkenyl, Hydroxyalkyl, Dihydroxyalkyl, AO-alkyl, Ar, Py, $R^{12}R^{13}$N-alkyl, Ar-alkyl, Ar-hydroxyalkyl, Indanyl, Py-alkyl, 1-$R^{14}$-piperidyl oder 2-(4-Methyl-5-imidazolylmethylthio)-ethyl, |
| $R^8$ | H, A, Hydroxyalkyl, Ar, Ar-alkyl, H-CO-, A-CO-, Ar-CO-, Py-CO-, Ar-alkyl-CO-, COOA oder CONHA, |
| $R^7$ und $R^8$ | zusammen auch $A_2$N-CH= oder eine Alkylengruppe mit 3 bis 5 C-Atomen, die durch -O- oder -$NR^{15}$- unterbrochen und/oder ein- oder mehrfach durch Carbonylsauerstoff, A, Ar-alkyl, COOH, COOA oder $ArCH_2OCO$- substituiert sein kann, |
| $R^9$ | H, H-CO-, AO-CO-, A-CO-, Cl, Br, OH, OA, A-COO-, $CF_3COO$-, A-$SO_2$-O-, Ar-$SO_2$-O-, SCN, 2-Imidazolylthio, 2-Benzimidazolylthio, Ar-CO-, Py-CO, Furoyl oder Thenoyl, |
| $R^{10}$ | H, A oder zusammen mit $R^6$ eine $-CH_2CH_2-$ oder -CH=CH-Gruppe, |
| $R^{11}$ | H, A, Alkenyl, AO-alkyl, $R^7R^8$N-CO-, $R^7R^8$N-CO-$CH_2$-, AOOC-$CH_2$-, CN, CN-$CH_2$-, Ar, Ar-alkyl, ArO-alkyl, ArS-alkyl, ArSO-alkyl, $ArSO_2$-alkyl, Ar-alkyl-O-alkyl, Ar-alkenyl, Py, Py-alkyl, Pyrimidyl, F, Cl, Br, J, OH, SH, OA, SA, SO-A, $SO_2$-A, $NR^7R^8$ oder 2-Oxo-1,2-dihydro-4-(A-CO)-5-A-pyridyl, |

$R^{12}$ und $R^{13}$ jeweils H, A, Ar-alkyl, H-CO-, A-CO-, Ar-CO- oder Ar-alkyl-CO- oder zusammen eine Alkylengruppe mit 4 oder 5 C-Atomen, die durch -O- oder $-NR^{15}-$ unterbrochen und/oder ein- oder mehrfach durch Carbonylsauerstoff, A, Ar-alkyl, COOH, COOA oder $Ar-CH_2O-CO-$ substituiert sein kann,

$R^{14}$ H, Formyl, COOA oder Benzyl,

$R^{15}$ H, A, Hydroxyalkyl, Dihydroxyalkyl, $A_2N$-alkyl, A-CO-, Ar, Thienyl, Ar-alkyl, $R^{12}R^{13}N-CO-CH_2-$, Ar-CO-, Furoyl, Thenoyl, $H_2N-CO-$, ANH-CO-, ArNH-CO- oder A-COO-alkyl

bedeuten,

worin die Alkyl- und Alkenylgruppen jeweils bis zu 6 C-Atome besitzen,

worin ferner

(a) nur zwei der Reste $R^3$ bis $R^6$ H bedeuten können,

(b) $R^5$ nur dann H sein kann, wenn gleichzeitig $R^4$ Ar-alkyl oder $NR^7R^8$ bedeutet,

(c) $R^5$ nur dann Ar, Py, unsubstituiertes Thiazolyl, unsubstituiertes Imidazolyl, unsubstituiertes Pyrazolyl oder unsubstituiertes Isoxazolyl sein kann, wenn gleichzeitig $R^4$ A, $A_2N-CH=CH-$, Ar-alkyl, OH, OA, SO-A, $SO_2-A$ oder $NR^7R^8$ und/oder $R^6$ COOA bedeuten,

(d) $R^5$ nur dann $CH_3$-CO- oder A-$CH_2$-CO- sein kann, wenn gleichzeitig $R^4$ $A_2N$-CH=CH-, Ar-alkyl, OH, OA, SO-A, $SO_2$-A oder $NR^7R^8$ und/oder $R^6$ COOA bedeuten,

(e) $R^5$ und $R^6$ zusammen nur dann $-COCH_2CH_2CH_2-$ sein können, wenn gleichzeitig $R^3$ CN, COOH, COOA, $COOCH_2C_6H_5$ oder $NH_2$ und/oder $R^4$ A, $A_2N$-CH=CH-, Ar-alkyl, OH, OA, SO-A, $SO_2$-A oder $NR^7R^8$ bedeuten,

sowie die entsprechenden Lactimester und die Salze der Verbindungen der Formel I.

2. a) 3-Cyan-1,2,5,6,7,8-hexahydro-chinolin-2,5-dion;

b) 3-Cyan-5-bromacetyl-6-methyl-2-pyridon;

c) 3-Cyan-5-chloracetyl-6-methyl-2-pyridon;

d) 3-Cyan-5-(2-brompropionyl)-6-methyl-2-pyridon;

e) 3-Cyan-5-(2-chlorpropionyl)-6-methyl-2-pyridon;

f) 3-Cyan-5-bromacetyl-6-ethyl-2-pyridon;

g) 3-Cyan-5-chloracetyl-6-ethyl-2-pyridon;

h) 3-Cyan-6-brom-1,2,5,6,7,8-hexahydro-chinolin-2,5-dion;

i) 3-Cyan-6-chlor-1,2,5,6,7,8-hexahydro-chinolin-2,5-dion;

j) 3-Cyan-5-/2-(3-pyridyl)-4-thiazolyl/-6-methyl-2-pyridon;

k) 3-Cyan-5-(2-p-diethylphosphonomethylphenyl-4-thiazolyl)-6-methyl-2-pyridon;

l) 3-Cyan-5-(2-p-methoxyanilino-4-thiazolyl)-6-methyl-2-pyridon;

m) 3-Cyan-5-(2-N-ethyl-formamido-4-thiazolyl)-6-methyl-2-pyridon;

n) 3-Cyan-5-(2-N-benzyl-acetamido-4-thiazolyl)-6-methyl-2-pyridon;

o) 3-Cyan-5-(2-N-ethyl-propionamido-4-thiazolyl)-6-methyl-2-pyridon;

p) 3-Cyan-5-(2-acetamido-4-methyl-5-thiazolyl)-6-methyl-2-pyridon;

q) 3-Cyan-5-(8-carbamoyl-imidazo/1,2-a/pyridin-2-yl)-6-methyl-2-pyridon;

r) 2-Dimethylamino-8-cyan-6,7-dihydro-thiazo/4,5-f/chinolin-7-on;

s) 3-Cyan-5-(2-dimethylaminomethylenamino-4-thiazolyl)-6-methyl-2-pyridon.

3. Verfahren zur Herstellung von Pyridonen der Formel I nach Anspruch 1 sowie ihrer Lactimester und der Säureadditionssalze der basischen unter diesen Verbindungen, dadurch gekennzeichnet, daß man

3.1 eine Dicarbonylverbindung der Formel II

$$R^6CO\text{-}CHR^5\text{-}CO\text{-}R^4 \qquad II$$

worin
$R^4$, $R^5$ und $R^6$ die in Anspruch 1 angegebenen Bedeutungen haben

oder eines ihrer reaktionsfähigen Derivate mit einer Verbindung der Formel III

$$R^{16}\text{-}CH_2\text{-}R^3 \qquad III$$

worin
$R^{16}$ $R^1NHCO$- oder CN bedeutet und
$R^1$ und $R^3$ die in Anspruch 1 angegebenen Bedeutungen haben

umsetzt, oder daß man

3.2 ein Amid der Formel IV

$$R^5\text{-}CH_2\text{-}CR^4\text{=}CR^3\text{-}CONH_2 \qquad IV$$

worin
$R^3$, $R^4$ und $R^5$ die in Anspruch 1 angegebenen Bedeutungen haben

oder eines seiner reaktionsfähigen Derivate mit einer Verbindung der Formel V

$X-CR^6(OA)_2$ V

worin

X AO oder $A_2N$ bedeuten und

$R^6$ und A die in Anspruch 1 angegebenen Bedeutungen haben

umsetzt, oder daß man

3.3 ein Keton der Formel VI

$R^6-CO-CH_2-R^5$ VI

worin
$R^5$ und $R^6$ die in Anspruch 1 angegebenen Bedeutungen haben

oder eines seiner reaktionsfähigen Derivate mit einem Nitril der Formel VII

$Z=C=C(CN)-R^{16}$ VII

worin
Z $(AS)_2$ oder (H,Y) und
Y HO, AO, A-CO-O-, $Ar-SO_2-O-$, $A_2N$ oder $H_2N-CO-NH-$

bedeuten und
A, Ar und $R^{16}$ die in Anspruch 1 bzw. oben angegebenen Bedeutungen haben

umsetzt, und/oder daß man

3.4 ein Keton der Formel I ($R^5 = R^{10}-CH_2-CO-$)

3.4.1 zu einem Haloketon der Formel I ($R^5$ = $Cl-CHR^{10}-CO-$ oder $Br-CHR^{10}-CO-$) halogeniert oder

3.4.2 mit einem Ester der Formel $R^{17}-COOA$ (worin $R^{17}$, H, AO, A, Ar, Py, Furyl oder Thienyl bedeutet) zu einer Dicarbonylverbindung der Formel I ($R^5 = R^{17}-CO-CHR^{10}-CO-$) kondensiert, und/oder daß man

3.5 ein Haloketon der Formel I ($R^5$ = $Cl-CHR^{10}-CO-$ oder $Br-CHR^{10}-CO-$)

3.5.1 mit einer Verbindung der Formel $R^{18}-H$ (worin $R^{18}$ OH, OA, A-COO- oder SCN bedeutet) oder einem ihrer reaktionsfähigen Derivate zu einer Verbindung der Formel I ($R^5 = R^{18}-CHR^{10}-CO-$) umsetzt oder

3.5.2 mit einem Thioamid der Formel $R^{11}-CS-NH_2$ oder einem seiner reaktionsfähigen Derivate zu einem Thiazolderivat der Formel I ($R^5$ = unsubstituiertes oder ein- oder mehrfach durch $R^{10}$ und/oder $R^{11}$ substituiertes Thiazolyl) umsetzt oder

3.5.3 mit einer Verbindung der Formel $R^{19}$ -SM (worin $R^{19}$ 2-Imidazolinylthio oder 2-Benzimidazolylthio bedeutet) oder einem ihrer reaktionsfähigen Derivate zu einer Verbindung der Formel I ($R^5 = R^{19}-CHR^{10}-CO-$) umsetzt oder

3.5.4 mit einem Guanidin der Formel $H_2N-C(=NH)-NR^7R^8$ (worin $R^7$ und $R^8$ die in Anspruch 1 angegebenen Bedeutungen haben) oder einem seiner reaktionsfähigen Derivate zu einer Verbindung der Formel I ($R^5$ = 2-$NR^7R^8$-4-imidazolyl) umsetzt oder

3.5.5 mit einem 2-Aminopyridinderivat der Formel VIII

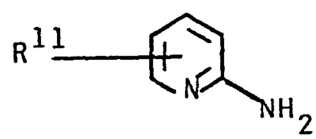

VIII

worin $R^{11}$ die in Anspruch 1 angegebene Bedeutung hat zu einem Imidazopyridinderivat der Formel I ($R^5$ = unsubstituiertes oder ein- oder mehrfach durch $R^{10}$ und/oder $R^{11}$ substituiertes Imidazo[1,2-a]pyridin-2-yl) umsetzt, oder daß man

3.6 eine Verbindung der Formel I ($R^5$ = HO-$CHR^{10}$-CO-) mit einer Verbindung der Formel $R^{20}$-H (worin $R^{20}$ A-COO-, $CF_3$-COO-, A-$SO_2$-O- oder Ar-$SO_2$-O- bedeutet) oder einem ihrer reaktionsfähigen Derivate zu einer Verbindung der Formel I ($R^5$ = $R^{20}$-$CHR^{10}$-CO-) umsetzt, oder daß man

3.7 eine 1,3-Dicarbonylverbindung der Formel I ($R^5 = R^{17}-CO-CHR^{10}-CO-$)

3.7.1 mit einem Hydrazinderivat der Formel $R^{11}-NH-NH_2$ zu einem Pyrazolderivat der Formel I ($R^5$ = unsubstituiertes oder ein- oder mehrfach durch $R^{10}$ und/oder $R^{11}$ substituiertes Pyrazolyl) oder

3.7.2 mit Hydroxylamin zu einem Isoxazolderivat der Formel I ($R^5$ = unsubstituiertes oder ein- oder mehrfach durch $R^{10}$ und/oder $R^{11}$ substituiertes Isoxazolyl) umsetzt, oder daß man

3.8 eine Carbonylverbindung der Formel I ($R^5 = R^{21}-CHR^{10}-CO-$, $R^{21}$ = Cl, Br, A-COO-, $CF_3COO-$, $A-SO_2-O-$oder $Ar-SO_2-O-$) mit einem Amidinderivat der Formel $R^{11}-C(=NH)-NH_2$ zu einem Imidazolderivat der Formel I ($R^5$ = unsubstituiertes oder ein- oder mehrfach durch $R^{10}$ und/oder $R^{11}$ substituiertes Imidazolyl)

umsetzt,

und/oder daß man in einer Verbindung der Formel I einen oder mehrere der Reste $R^1$,$R^3$,$R^4$,$R^5$ und/oder $R^6$ in andere Reste $R^1$,$R^3$,$R^4$,$R^5$ und/oder $R^6$ umwandelt, indem man

3.9 eine primäre oder sekundäre Aminogruppe alkyliert, benzyliert oder acyliert und/oder

3.10 eine CN-Gruppe zu einer $CONH_2$- oder einer COOH-Gruppe oder eine Carbamoyl- oder COOA-Gruppe zu einer COOH-Gruppe oder einen Lactimester zum freien Pyridon und/oder eine Dialkylphosphono-Gruppe zu einer Alkylphosphono- oder zu einer Phosphono-Gruppe oder eine Alkylphosphono-Gruppe zu einer Phosphono-Gruppe und/oder eine Thioethergruppe zu einer OH-Gruppe und/oder eine N-Acyl-Gruppe zu einer NH-Gruppe hydrolysiert und/oder

3.11 eine Thioethergruppe mit Ammoniak oder einem Amin der Formel $HNR^7R^8$ in eine $NR^7R^8$-Gruppe umwandelt und/oder

3.12 eine Thioethergruppe mit einem Alkohol oder einem seiner reaktionsfähigen Derivate in eine AO-Gruppe umwandelt und/oder

3.13 eine COOH-Gruppe decarboxyliert und/oder

3.14 eine -CH-CH-Gruppe zu einer -C=C-Gruppe dehydriert und/oder

3.15 eine Thioethergruppe zu einer Sulfoxid- oder zu einer Sulfongruppe oxydiert und/oder

3.16 eine COOH- oder Phosphonsäure-Gruppe verestert und/oder

3.17 eine N-Oxid-Gruppe zur entsprechenden tertiären Aminogruppe reduziert und/oder

3.18 zwei H-Atome durch Kondensation mit einem Amidacetal der Formel $A_2N-CH(OA)_2$ durch die Gruppe $A_2N-CH=$ ersetzt und/oder

3.19 eine Verbindung der Formel I ($R^5$ = 2-$R^{11}$-5-H-4-thiazolyl, $R^6$ = $CH_3$) mit einem Ameisensäurederivat zu einer Verbindung der Formel I ($R^5$ = 2-$R^{11}$-5-$R^{10}$-4-thiazolyl, $R^6$ und $R^{10}$ zusammen = -CH=CH-) umsetzt und/oder

3.20 zwei H-Atome durch Kondensation mit einem Aldehyd durch die Gruppe Ar-CH= ersetzt und/oder

3.21 eine Carbamoylgruppe durch Behandeln mit Halogen und einer starken Base in eine Aminogruppe umwandelt und/oder

daß man eine Base der Formel I durch Behandeln mit einer Säure in eines ihrer Säureadditionssalze überführt.

4. Verfahren zur Herstellung pharmazeutischer Zubereitungen, dadurch gekennzeichnet, daß man eine Verbindung der Formel I und/oder einen entsprechenden Lactimester und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einen entsprechenden Lactimester und/oder einem ihrer physiologisch unbedenklichen Salze.

6. Verbindungen der allgemeinen Formel I nach Patentanspruch 1 zur Bekämpfung von Krankheiten.

7. Verwendung von Verbindungen der allgemeinen Formel I bei der Bekämpfung von Krankheiten, insbesondere Herzinsuffizienz und/oder Bluthochdruck.

0154190

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 85101326.8

## EINSCHLÄGIGE DOKUMENTE

| Kategorie | Kennzeichnung des Dokuments mit Angabe, soweit erforderlich, der maßgeblichen Teile | Betrifft Anspruch |
|---|---|---|
| A | DE - A1 - 3 317 289 (SANDOZ)<br>* Ansprüche 1,4,8,10 *<br>-- | 1,5,6 |
| D,A | DE - A1 - 3 106 460 (SANDOZ)<br>* Ansprüche 1,9; Zusammenfassung *<br>-- | 1,5,6 |
| D,A | DE - A1 - 2 646 469 (STERLING)<br>* Ansprüche 1,40 *<br>-- | 1,5,6 |
| P,A | EP - A1 - 0 120 589 (FUJISAWA)<br>* Zusammenfassung *<br>-- | 1 |
| A | EP - A2/3 - 0 092 459 (SYNTHELABO)<br>* Zusammenfassung *<br>-- | 1 |

KLASSIFIKATION DER ANMELDUNG (Int. Cl.4)

C 07 D 417/04
C 07 D 513/04
C 07 D 213/85
C 07 D 417/14
C 07 D 471/04
C 07 D 215/54
C 07 D 401/04
C 07 D 401/06
C 07 D 401/12
C 07 D 401/14
C 07 D 413/04
C 07 D 213/73
C 07 D 498/04
C 07 F 9/65
A 61 K 31/44
A 61 K 31/47
A 61 K 31/535
A 61 K 31/54
A 61 K 31/675

RECHERCHIERTE SACHGEBIETE (Int. Cl.4)

C 07 D 417/00
C 07 D 513/00
C 07 D 213/00
C 07 D 471/00
C 07 D 215/00
C 07 D 401/00
C 07 D 413/00
C 07 D 498/00
C 07 F 9/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: -
Nicht recherchierte Patentansprüche: 7
Grund für die Beschränkung der Recherche:

(Verfahren zur therapeutischen Behandlung des menschlichen oder tierischen Körpers; Artikel 52(4) EPÜ)

| Recherchenort | Abschlußdatum der Recherche | Prüfer |
|---|---|---|
| WIEN | 30-05-1985 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82

0154190
Nummer der Anmeldung

Europäisches Patentamt

# EUROPÄISCHER TEILRECHERCHENBERICHT

EP 85101326.8

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | DE - A1 - 2 502 588 (TROPONWERKE)<br>* Ansprüche 1,3 *<br>-- | 1,5,6 | |
| D,A | US - A - 4 313 951 (LESHER)<br>-- | | |
| A | US - A - 4 104 273 (MC NULTY)<br>---- | | |
| | | | RECHERCHIERTE SACHGEBIETE (Int. Cl.4) |